# EUROPEAN PATENT SPECIFICATION

(11) **EP 0 490 667 B1**
(45) Date of publication and mention of the grant of the patent: **09.06.1999**
(21) Application number: 91311549.9
(22) Date of filing: 11.12.1991
(51) Int. Cl.: C07K 5/02, C07K 7/02, A61K 38/04

(54) **HIV protease inhibitors**
HIV-Proteaseinhibitoren
Inhibiteurs d'HIV protéase

(30) Priority: 11.12.1990 JP 40967390; 25.01.1991 JP 2575591; 28.03.1991 JP 8997691; 14.06.1991 JP 16917491; 23.10.1991 JP 30404391
(43) Date of publication of application: 17.06.1992
(73) Proprietor: JAPAN ENERGY CORPORATION, Minato-ku, Tokyo (JP)
(72) Inventor: Mimoto, Tsutomu, c/o Nippon Mining Co., Ltd., Toda-shi, Saitama (JP); Hattori, Naoko, c/o Nippon Mining Co., Ltd., Toda-shi, Saitama (JP); Nagano, Yuuichi, c/o Nippon Mining Co., Ltd., Toda-shi, Saitama (JP); Shintani, Makoto, c/o Nippon Mining Co., Ltd., Toda-shi, Saitama (JP); Kiso, Yoshiaki, Osaka (JP)
(74) Representative: Geering, Keith Edwin

(56) References cited:
- JOURNAL OF MEDICINAL CHEMISTRY, vol. 20, no. 4, 1977, Washington, DC (US); TAKITA et al., pp. 510-515
- CHEMICAL PHARMACEUTICAL BULLETIN, vol. 38, no. 9, 1990, Tokyo (JP); IIZUKA et al., pp. 2487-2493
- JOURNAL OF ORGANIC CHEMISTRY, vol. 45, no. 12, 1980, Easton, PA (US); RICH et al., pp. 2288-2290
- SCIENCE, vol. 248, April 1990, Lancaster, PA (US); ROBERTS et al., pp. 358-361
- JOURNAL OF MEDICINAL CHEMISTRY, 1990, Washington, DC (US); IIZUKA et al., pp. 2707-2714
- CHEMICAL PHARMACEUTICAL BULLETIN, vol. 39, no. 9, September 1991, Tokyo (JP); MIMOTO et al., pp. 2465-2467
- CHEMICAL PHARMACEUTICAL BULLETIN, Vol. 39, no. 11, November 1991, Tokyo (JP); MIMOTO ET AL., pp. 3000 - 3090
- CHEMICAL PHARMACEUTICAL BULLETIN, Vol. 39, no. 11, November 1991, Tokyo (JP); MIMOTO ET AL., pp. 3000 - 3090

## Description

### BACKGROUND OF THE INVENTION

### Field of the Invention:

This invention relates to peptide derivatives which inhibit human immunodeficiency virus (HIV) protease and pharmaceutically acceptable salts thereof.

### Description of the Related Art:

Heretofore, various efforts for the therapy of acquired immunodeficiency syndrome (AIDS) and prevention of infection of the human immunodeficiency virus (HIV) by inhibiting the HIV protease have been performed. Some peptide derivatives have been proposed as the inhibitor, for example, EP 357332, EP 337714, EP 372537, EP 373497 and EP 373549.

Some HIV protease inhibitors contain hydroxyamino acid isosteres, and in addition, a formation of hydrogen bond between the hydroxy group and Asp25 in the active site of HIV protease was proposed [cf. Proc. Natl. Acad. Sci., U.S.A., 87, 8805 (1990)].

Also, a β-amino-α-hydroxy-carboxylic residue shown by the following general formula (6) was reported in relation to an anti-cancer agent [e.g. J. Med. Chem., 20, 510 (1977)].

Some peptide derivatives containing an isosters, for example, -NH-CH(R)-CH(OH)-CH₂- [EP 372537 and EP 373497], NH-CH (R)-CH=N(O)R [EP 373549], -NH-CH(R)-CH(OH)-CH₂-CO- [EP 357332 and EP 337714], or -NH-CH(R)-CH(OH)-CH₂-CH(R)-CO- [EP 357332 and EP 337714] wherein Rs are variable substituents, have been reported to inhibit the HIV protease. It was also proposed, based on X-ray crystallographic study, that a hydroxyl group in an isostere improves enzyme-inhibitor binding through hydrogen bond formation with Asp 25/125 of HIV protease [Proc. Natl. Acad. Sci., U.S.A., 87, 8805 (1990)].

On the other hand, β-amino-α-hydroxy-carboxylic acids represented by formula (6) as amino acid isosteres and their preparation were reported in relation to a renin inhibitor [Chem. Pharm. Bull., 1990, 38, 2487; J.Med.Chem., 1990, 33, 2707; Japanese Unexamined Patent Publication No.101098 (1990)] and an anti-cancer agent [J. Med.Chem., 1977, 20, 510].

Peptide derivatives containing the isoster of formula (6) had not reported as HIV protease inhibitors at the time of the invention. A hydroxyl group in the isostere of formula (6), however, could be expected to form hydrogen bonds with Asp 25/125 of HIV protease from the relevant art mentioned above. On the other hand, effects of a carbonyl group in the isostere of formula (6) upon HIV protease inhibition had not been reported. The applicants found that the claimed peptide derivatives containing some class of the isostere of formula (6) highly inhibit HIV protease activity.

The carbonyl group in the isosteres of formula (6) possibly form "additional" hydrogen bond with the Asp 25/125 which would afford more tight enzyme-inhibitor binding. The hydrogen bond network between the isostere of formula (6) and the Asp 25/125 was "later" supported by X-ray crystallographic study for one of the claimed compounds [Structure, 1995, 3, 581].

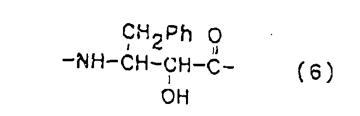

However, no β -amino- α-hydroxy-carbocyclic acid mentioned above has been used as amino acid isostere in an an HIV protease inhibitor.

Further, no compound having the following basic structure represented by the general formula (3) has been known. 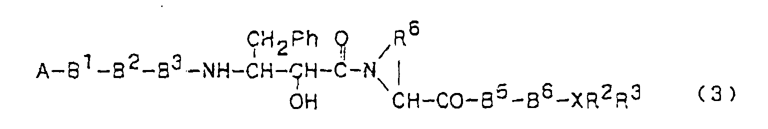 where A represents a hydrogen atom or a peptide N-terminal protective group;
B¹, B², B⁵ and B^{b} represent independently single bond or amino acid residue optionally the amino group of said amino acid be substituted with a hydrocarbon residue having 12 or less carbon atoms:
X is a nitrogen atom or oxygen atom:
R² and R³ each represents hydrogen atom or an optionally substituted hydrocarbon group having 12 or less carbon atoms which form cycles by forming bonds between said carbon atoms which may optionally be replaced with oxygen, nitrogen or sulfur with the proviso that no R³ is present when X is oxygen:
B³ is the residue of an amino acid selected from asparagine, glutamine, serine, O-methylserine, O-methyl-threonine, methylthioalanine, methanesulfonylalanine, valine, norvaline, leucine, isoleucine and tert-leucine, and R⁶ represents a bivalent hydrocarbon group forming a 5-7 membered ring optionally substituted or fused with the other 5-7 membered ring, and a part of carbon atoms in said rings optionally replaced with hetero atoms.

### SUMMARY OF THE INVENTION

The present invention provides novel HIV protease inhibitors.

It provides novel compounds with said inhibitory action having β-amino-α-hydroxycarboxylic acid residue as an amino acid isostere.

The present invention includes the following novel compounds and HIV protease inhibitors containing said compounds or pharmaceutically acceptable salts thereof: 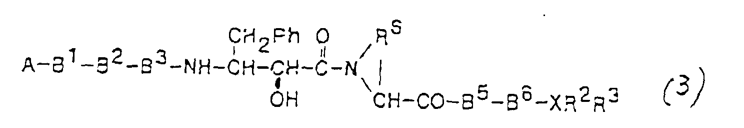 where A represents hydrogen atom or a peptide N-terminal protective group;
B¹ , B², B⁵ and B⁶ represent independently single bond or amino acid residue optionally the amino group of said amino acid be substituted with a hydrocarbon residue having 12 or less carbon atoms:
X is a nitrogen atom or oxygen atom:
R² and R³ each represents hydrogen atom or an optionally substituted hydrocarbon group having 12 or less carbon atoms which may form cycles by forming bonds between said carbon atoms which may optionally be replaced with oxygen, nitrogen or sulfur with the proviso that no R³ is present when X represents oxygen atom:
B³ is the residue of an amino acid selected from asparagine, glutamine, serine, O-methylserine, O-methylthreonine, methylthioalanine, methanesulfonylalanine, valine, norvaline, leucine, isoleucine and tert-leucine, and R⁶ represents a bivalent hydrocarbon group forming a 5-7 membered ring optionally substituted or fused with the other 5-7 membered ring, and a part of carbon atoms in said rings optionally replaced with hetero atoms.

Herein "lower" as applied to "alkyl", "alkoxy", "alkane" etc., indicates a preference for a said group of up to 6 carbon atoms.

### BRIEF DESCRIPTION OF THE DRAWINGS

Figures 1-6 show NMR spectra of the compounds of Examples 82, 86, 100, 106, 130 and 178, respectively.

### DETAILED DESCRIPTION OF THE INVENTION

The inventors have discovered that inhibition of HIV protease is shown by peptide derivatives having an amino acid isostere of *β* -amino-α -hydroxycarboxylic acid residue represented by the following general formula (6)

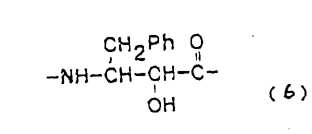

The inhibitory activity may be derived from a hydrogen bond formed between a hydroxy group of above mentioned *β* -amino- α -hydroxycarboxylic acid residue and Asp25 in the active site of HIV protease, and also from the other hydrogen bond formation by a carbonyl group in the active site. The inhibitory activity is presumed to be enforced by fixing the conformation of neighboring amino acid residue by said carbonyl group through the amide bond.

The present invention relates to a human immunodeficiency virus (HIV) protease inhibitor peptide derivatives represented by the following general formula (3) or pharmaceutically acceptable salts thereof.

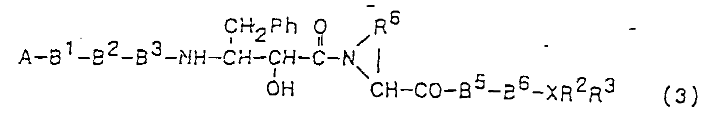

In the general formula (3), A represents hydrogen atom or a peptide N-terminal protective group. The N-terminal-protective group includes such as acetyl group (Ac-), propionyl group, butyryl group, isobutyryl group, valeryl group, isovaleryl group, pivaloyl group, hexanoyl group, heptanoyl group, octanoyl group, benzyl group (Ph-CH₂-), benzoyl group, phenylacetyl group (Ph-CH₂-CO-), 3-phenylpropionyl group [Ph-(CH₂)₂-CO-], phenylpropenoyl group, pyridinecarbonyl group (Pyridine-CO-), quinoline-2-carbonyl group (Quinoline-CO-), phenoxyacetyl group (Ph-O-CH₂-CO-), o-chlorophenoxyacetyl group (oCl-Ph-O-CH₂-CO-), m-chlorophenoxyacetyl group (mCl-Ph-O-CH₂-CO-), p-chlorophenoxyacetyl group (pCl-Ph-O-CH₂-CO-), o-phenylphenoxyacetyl group (oPh-Ph-O-CH₂-CO-), m-phenylphenoxyacetyl group (mPh-Ph-O-CH₂-CO-), p-phenylphenoxyacetyl group (pPh-Ph-O-CH₂-CO-), 1-naphthoxyacetyl group (1Nap-O-CH₂-CO-), 2-naphthoxyacetyl group (2Nap-O-CH₂-CO-), N-(1-naphthyl)-aminoacetyl group (1Nap-NH-CH₂-CO-), glutaryl group [-CO-(CH₂)₃-CO-], succinyl group [-CO-(CH₂)₂-CO-], 3-(p-methylbenzyl)thiopropionyl group, diphenylmethyloxyacetyl group [(C₆H₅)₂CH-O-CH₂-CO-], bis(p-chlorophenyl)methyloxyacetyl group [(p-ClPh)₂CH-O-CH₂-CO-], (5-isoquinolyloxy)acetyl group (5Isoquinoline-O-CH₂-CO-), naphthalenecarbonyl group, isoquinoline-1-carbonyl group (1-Isoquinoline-CO-), furancarbonyl group (furan-CO-), thiophenecarbonyl group (thiophene-CO-), methoxycarbonyl group, ethoxycarbonyl group, propoxycarbonyl group, tert-butoxycarbonyl group (Boc-), benzyloxycarbonyl group (Ph-CH₂-O-CO-), 1-naphthyl-methyloxycarbonyl group (1Nap-CH₂-O-CO-), 9-fluorenylmethoxycarbonyl group (Fmoc-), naphthalene-1-sulfonyl group (1Nap-SO₂-), benzofurancarbonyl group (Benzofuran-CO-), (E)-4-phenyl-3-butenyl group ([(E)Ph-CH=CH-CH₂-CO-], m-(iso-propyloxy)phenyloxyacetyl group [m-(iPrO)-Ph-O-CH₂-CO-], 5,6,7,8-tetrahydro-1-naphthyloxyacetyl group [1-Tna-O-CH₂-CO-], m-(N-phenylamino)phenyloxyacetyl group [m-(Ph-NH)-Ph-O-CH₂-CO-], m- (morpholinocarbonyl)-phenyloxyacetyl group [m-(Morph-CO)-Ph-O-CH₂-CO-], m-(piperidinocarbonyl)phenyloxyacetyl group [m-(Piper-CO)-Ph-O-CH₂-CO-], 2,3-dimethylphenyloxyacetyl group (2,3-diMe-Ph-O-CH₂-CO-) and 8-quinolyloxyacetyl group (8-Qoa-). Among them, aryloxyacetyl groups such as m-chlorophenoxyacetyl group, m-phenylphenoxyacetyl group, 1-naphthoxyacetyl group, (5-isoquinolyloxy)-acetyl group, m-(N-phenylamino)phenyloxy-acetyl group are particularly preferable for the marked elevation of HIV protease inhibitory activity. In addition abbreviations used in the above parentheses are used as abbreviations in the specification B¹, B², B⁵, and B⁶ represent amino acid residues and mean independently natural or un-natural amino acid residue and the corresponding amino acid include, for example, glycine (Gly), alanine (Ala), valine (Val), leucine (Leu), isoleucine (Ile), serine (Ser), threonine (Thr), cysteine (Cys), methionine (Met), asparagine (Asn), glutamine (Gln), phenylalanine (Phe), tyrosine (Tyr), tryptophan (Try), aspartic acid (Asp), glutamic acid (Glu), histidine (His), lysine (Lys), arginine (Arg), proline (Pro), *β*-acetylalanine (Aca), phenylglycine (Phg), α-allylglycine (Alg), α-propargylglycine (Prg), N-cyclohexylmethylglycine [(cHexm)Gly], N-benzylglycine [(Bzl)Gly], β-alanine (βAla), *β*-cyclohexylalanine, β-(naphthyl)alanine, β-cyanoalanine, β-(cyanomethyl)alanine, β-(sulfonylmethyl)alanine, *β*-(methanesulfonyl)alanine (Msa), *β*-(methanesulfonylmethyl)alanine [Met(O)₂], β-sulfanylalanine,β-(methanesulfinyl)alanine [Smc(O)], sulfanylmethylalanine, *β*-sulfamoylalanine (Sma), β-methylthioalanine (Mta), *β*-(dimethylsulfonio)alanine [Mta⁺(Me)], D-valine (D-Val), norvaline (Nva), β-(methanesulfonyl )valine (Msv), β-(methylthio)valine (Mtv), norleucine, tert-leucine (Tle), homoserine (Hse), O-methylserine [Ser(Me)], O-methylthreonine [Thr(Me)], D-phenylalanine (D-Phe), O-methylaspartic acid, β-hydrazinoaspartic acid [Asp(NHNH₂)], O-methylglutamic acid, hydroxyproline (Hyp), 4-benzyloxypyrrolidine-2-carboxylic acid [Hyp(Bzl)], 4-methoxypyrrolidine-2-carboxylic acid [Hyp(Me)], 4-ethoxypyrrolidine-2-carboxylic acid [Hyp(Et)], 4-allyloxypyrrolidine-2-carboxylic acid [Hyp(Allyl)], cis-4-cyclohexylpyrrolidine-2-carboxylic acid (Ccp), trans-4-cyclohexylpyrrolidine-2-carboxylic acid (Tcp), 4-benzyl-pyrrolidine-2-carboxylic acid, 3-phenylpyrrolidine-2-carboxylic acid (Php), cis-4-phenylpyrrolidine-2-carboxylic acid (Cpp), 4-hydroxy-4-phenylpyrrolidine-2-carboxylic acid (Hpp), 4-phenyl-2,5-dihydropyrrole-2-carboxylic acid (Pdp), 4-methylthiopyrrolidine-2-carboxylic acid, 4-phenylthiopyrrolidine-2-carboxylic acid, 4-fluoropyrrolidine-2-carboxylic acid, 4,4-di(methylthio)pyrrolidine-2-carboxylic acid [Pro(SMe)₂], 3,3-dimethylpyrrolidine-2-carboxylic acid (Dmp), 2-aminooctanoic acid, 2-aminoheptanoic acid, indoline-2-carboxylic acid (Inc), octahydroindole-2-carboxylic acid (Oic), octahydrocyclo-penta[b]pyrrole-2-carboxylic acid, L-pipecolic acid [(L)-Pip], D-pipecolic acid [(D)-Pip], L-1,2,3,4-tetrahydroisoquinoline-3-carboxylic acid [(L)-Tic], D-1,2,3,4-tetrahydroisoquinoline-3-carboxylic acid [(D)-Tic], decahydroisoquinoline-3-carboxylic acid (3Dic), decahydroisoquinoline-1-carboxylic acid (1Dic), 5,5-dimethyl-1,3-thiazolidine-4-carboxylic acid (Dtc), β-amino isobutyric acid (BAIB), &B-amino butyric acid (BANB), γ-aminobutyric acid (GABA) and 1,3-thiazolidine-4-carboxylic acid (Thz). In addition abbreviations used in the above parentheses are used as abbreviations in the specification.

The amino group in these amino acids may be substituted with a hydrocarbon group having 12 or less carbon atoms. Such hydrocarbon groups include such as methyl, ethyl, benzyl and cyclohexylmethyl groups.

B³ is the residue of an amino acid selected from asparagine, glutamine, serine, O-methylserine, O-methylthreonine, methylthioalanine, methanesulfonylalanine, valine, norvaline, leucine, isoleucine and tert-leucine; and are preferable for the improvement of HIV protease inhibitory activity.

In the general formula (3), amino acid residues represented by the general formula (11) are also preferable for the improvement of HIV protease inhibitory activity.

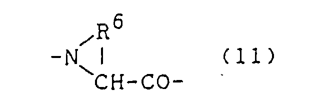

In the general formula (11), R⁶ represents a bivalent hydrocarbon group forming a 5-7 membered ring and optionally substituted or fused with the other 5-7 membered ring, some or one of which carbon atoms in said rings may be replaced with hetero atom(s).

The corresponding amino acid include, for example, proline, 4-hydroxypyrrolidine-2-carboxylic acid, 4-benzyloxypyrrolidine-2-carboxylic acid, 4-cyclohexylpyrrolidine-2-carboxylic acid, 4-phenylpyrrolidine-2-carboxylic acid, 3-phenylpyrrolidine-2-carboxylic acid, 4-benzylpyrrolidine-2-carboxylic acid, 4-methylthiopyrrolidine-2-carboxylic acid, 4-phenylthiopyrrolidine-2-carboxylic acid, 4-fluoro-pyrrolidine-2-carboxylic acid, 4,4-bis(methylthio)-pyrrolidine-2-carboxylic acid, 3,3-dimethylpyrrolidine-2-carboxylic acid, 1,3-thiazolidine-4-carboxylic acid, 5,5-dimethyl-1,3-thiazolidine-4-carboxylic acid, indoline-2-carboxylic acid, octahydroindole-2-carboxylic acid, octa-hydrocyclopenta[b]pyrrole-2-carboxylic acid, pipecolinic acid, 1,2,3,4-tetrahydroisoquinoline-3-carboxylic acid, 1,2,3,4-tetrahydroisoquinoline-1-carboxylic acid, decahydro-isoquinoline-3-carboxylic acid, and decahydroisoquinoline-1-carboxylic acid. Among them, proline, 3,3-dimethyl-pyrrolidine-2-carboxylic acid, 1,3-thiazolidine-4-carboxylic acid and 5,5-dimethyl-1,3-thiazolidine-4-carboxylic acid are particularly preferable. The number of peptide bonds and number of amino acid unit in the molecule is preferably decreased for the better in vivo stability and membrane permeability, thus the absence of B¹ and B² is preferable, particularly the absence of B¹, B², B⁵ and B⁶ is more preferable.

In the partial formula of -XR²R³, X represents nitrogen or oxygen atom, and R² and R³ represent independently hydrogen atom or a hydrocarbon group having 12 or less carbon atoms optionally replaced with oxygen, nitrogen or sulfur atom, and when X represents oxygen, no R³ exists. Said hydrocarbon group in R² and R³ represents, for example, methyl group (-Me), isopropyl group (-iPr), isobutyl group (-iBu), sec-butyl group (-sBu), 2-pentyl group, 1-ethylpropyl group, tert-butyl group (-tBu), neopentyl group, tert-amyl group (tAmyl), 3-methyl-2-butyl group, 2,3-dimethyl-2-butyl group, cyclohexyl group (-C₆H₁₁), cyclohexylmethyl group (-CH₂-C₆H₁₁), cyclopropyl group, cyclopentyl group, phenyl group (-Ph), benzyl group, naphthyl group and naphthylmethyl group. Said substituted hydrocarbon group includes, for example, 3-hydroxy-2-methyl-2-propyl group, 1,1-bis(hydroxy-methyl)ethyl group, 1-hydroxymethyl-2-methylpropyl group, 1-hydroxy-2-methylbutyl group, 2-hydroxy-1-phenylethyl group, 2-hydroxycyclohexyl group (-chex-ol), o-hydroxyphenyl group [-Ph(o-OH)], m-hydroxyphenyl group [-Ph(m-OH)] and p-hydroxyphenyl group [-Ph(p-OH)]. Furthermore, when X represents nitrogen atom, said R² and R³ may be bridged to form a ring, and a part of carbon atoms in said rings may be replaced with oxygen, nitrogen or sulfur atom. These groups include, for example, 1,2,3,4-tetrahydroisoquinolin-2-yl group, decahydroquinolin-1-yl group, decahydroisoquinolin-2-yl group (-Diq), 1-indolyl group, octahydroindol-1-yl group, 2-isoindolyl group, octahydroisoindol-2-yl group, 1-pyrrolidinyl group, 1-piperidinyl group (-piperidine), 1-morpholinyl group, 1,3-thiazolidin-3-yl group, 5,5-dimethyl-1,3-thiazolidin-3-yl group, tetrahydro-1,4-thiazin-3-yl group and hexahydro-azepin-1-yl group. In addition abbreviations used in the above parentheses are used as abbreviations in the specification.

The pharmaceutically acceptable salts of the peptide derivatives in the present invention and represented by the general formula 3 include, for example, inorganic acid addition salts such as hydrochloride, sulfate, and phosphate, organic acid addition salts such as acetate, oxalate, maleate, metal salts such as sodium, potassium and calcium salt, and organic amine salts such as triethylamine salt.

The β-amino-α-hydroxycarboxylic acid having the structure represented by the general formula (6) can be synthesized by conventional methods. For example, first the amino group of phenylalanine is protected by a known protecting group such as tert-butoxycarbonyl group carboxy group of the protected amino acid is esterified and hydroxymethyl group is introduced by reduction.

Said hydroxymethyl group is converted into formyl group by the reaction with an oxidant such as dimethylsulfoxide, which is caused to react with sodium cyanide to make cyanohydrin compound. The resultant compound is hydrolyzed, for example, with hydrochloric acid to give the said β-amino-α-hydroxy-carboxylic acid. The groups of general formula (6) have two asymmetric carbon atoms and therefore, compounds having the residue represented by the general formula (6) are often obtained as a mixture of (2S,3S) and (2R,3S) compounds from the starting material of optically active compound represented by the general formula (14), for example S-type compound. In the present invention those mixtures can be used but isomers obtained by a separation by conventional methods such as silica gel column chromatography are preferred. For the separation, suitable protecting group may be introduced to amino group or carboxy group. For example, a compound with general formula (6), such as (2RS,3S)-3-amino-2-hydroxy-4-phenyl-butanoic acid can be separated into (2R,3S) and (2S,3S) isomers by the protection of amino group with tert-butoxycarbonyl group and carboxy group with benzyl ester, followed by silica gel column chromatography.

The peptide derivatives represented by the general formula (3) of the present invention, including pharmaceutically acceptable salts thereof, inhibit cleavage of a peptide substrate, for example, Ac-Arg-Ala-Ser-Gln-Asn-Tyr-Pro-Val-Val-NH₂ [Biochem. Biophys. Res. Comm., 159, 420 (1989)] by chemically synthesized HIV protease, in which two cysteine residues in the reported sequence [Science, 230, 949 (1985)] were replaced with alanine residues, or recombinant HIV protease [Biochemistry, 29, 264 (1990)]. Therefore the peptide derivatives of the present invention can be used as an inhibitor of HIV protease and may be used for the therapy and prevention of AIDS.

The peptide derivatives represented by the general formula (3) can be prepared from amino acid derivatives having the residue represented by general formula (6) by conventional methods in peptide chemistry. L-type amino acid residues are preferred for B¹, B², B³, B⁵, B⁶ and the residue of the general formulae in the general formula (3). The preferred configuration in the amino acid residue of formula (6) means (2S, 3S).

Separation of isomers may be carried out after a peptide bond formation started from isomeric mixture of the amino acid derivatives having a residue represented by the general formula (6).

The preferred peptide derivatives represented by the general formula (3) are illustrated in the Table by the example Nos.

**Table 1**

| Example | Chemical Formula | Residual Activity (%) | |
|---|---|---|---|
| | | 1 mM | 5 µM |
| B | Boc-Asn-(2R,3S)-AHPBA-NH-CH₂-C₆H₁₁ | 32.8 | |
| C | Boc-Phe-Asn-(2R,3S)-AHPBA-NH-CH₂-C₆H₁₁ | 19.5 | |
| D | Boc-Asn-(2S,3S)-AHPBA-NH-CH₂-C₆H₁₁ | 52.9 | |
| E | Boc-Phe-Asn-(2S,3S)-AHPBA-NH-CH₂-C₆H₁₁ | 20.3 | |
| F | Boc-Ser-(2R,3S)-AHPBA-NH-CH₂-C₆H₁₁ | 82.9 | |
| G | Boc-Phe-Ser-(2R,3S)-AHPBA-NH-CH₂-C₆H₁₁ | 33.3 | |
| H | Boc-Ser-(2S,3S)-AHPBA-NH-CH₂-C₆H₁₁ Boc-Phe-Ser-(2S,3S)-AHPBA-NH-CH₂-C₆H₁₁ | 15.6 | |
| J | | 47.0 | |
| K | H-Asn-(2R,3S)-AHPBA-NH-CH₂-C₆H₁₁ | 92.1 | |
| L | H-Phe-Asn-(2R,3S)-AHPBA-NH-CH₂-C₆H₁₁ | 43.4 | |
| M | H-Phe-Asn-(2S,3S)-AHPBA-NH-CH₂-C₆H₁₁ | 16.4 | |
| N | H-Ser-(2R,3S)-AHPBA-NH-CH₂-C₆H₁₁ | 89.7 | |
| O | H-Phe-Ser-(2R,3S)-AHPBA-NH-CH₂-C₆H₁₁ | 58.9 | |
| P | H-Ser-(2S,3S)-AHPBA-NH-CH₂-C₆H₁₁ | 95.5 | |
| Q | H-Phe-Ser-(2S,3S)-AHPBA-NH-CH₂-C₆H₁₁ | 23.8 | |
| 16 | PhCH₂CH₂CO-Asn-(2S,3S)-AHPBA-Pro-Ile-Val-NH₂ | | 7.0 |
| 17 | PhCH₂CH₂CO-Asn-(2R,3S)-AHPBA-Pro-Ile-Val-NH₂ | 1.5 | 19.3 |
| R | H-Val-Val-(2R,3S)-AHPBA-Phe-Val-Val-NH₂ | 1.0 | 5.6 |
| S | H-Val-Val-(2S,3S)-AHPBA-Phe-Val-Val-NH₂ | 5.1 | 32.1 |
| 20 | PhCH₂CO-Ser-(2S,3S)-AHPBA-Pro-Ile-Val-NH₂ | 2.8 | 42.8 |
| 21 | PhCH₂CO-Ser-(2R,3S)-AHPBA-Pro-Ile-Val-NH₂ | 27.7 | 80.9 |
| T | H-Val-Val-(2S,3S)-AHPBA-(D)-Phe-(D)-Val-(D)-Val-NH₂ | 52.9 | |
| U | H-Val-Val-(2R,3S)-AHPBA-(D)-Phe-(D)-Val-(D)-Val-NH₂ | 28.3 | |
| V | H-Val-Val-(2S,3S)-AHPBA-(Bzl)Gly-Val-Val-NH₂ | 30.4 | |
| W | H-Val-Val-(2R,3S)-AHPBA-(Bzl)Gly-Val-Val-NH₂ | 2.3 | |
| 26 | PhCH₂CH₂CO-Ser-(2R,3S)-AHPBA-Pro-Ile-Val-NH₂ | 15.7 | 83.1 |
| 27 | PhCH₂CH₂CO-Asn-(2S,3S)-AHPBA-Pro-Melle-Val-NH₂ | | 92.9 |
| X | Boc-(2S,3S)-AHPBA-Pro-Ile-O-C₆H₁₁ | 63.9 | |
| Y | Boc-(2R,3S)-AHPBA-Pro-Ile-O-C₆H₁₁ | 88.3 | |
| Z | Boc-(2S,3S)-AHPBA-Pro-Ile-NH-CH₂-C₆H₁₁ | 23.4 | |
| AA | Boc-(2R,3S)-AHPBA-Pro-Ile-NH-CH₂-C₆H₁₁ | 47.4 | |
| 32 | PhCH₂-O-CO-Asn-(2S,3S)-AHPBA-Pro-Ile-NH-CH₂-C₆H₁₁ | | 4.0 |
| BB | Boc-(2S,3S)-AHPBA-(cHexm)Gly-Ile-NH-CH₂-C₆H₁₁ | 85.6 | |

**Table 2**

| Example | Chemical Formula | Residual Activity (%) | |
|---|---|---|---|
| | | 1 mM | 5 µM |
| CC | Boc-(2R,3S)-AHPBA- (cHexm)Gly-Ile-NH-CH₂-C₆H₁₁ | | |
| 35 | PhCH₂CH₂CO-Asn-(2S,3S)-AHPBA-Pro-β Ala-NH₂ | | 60.9 |
| 36 | PhCH₂CH₂CO-Gln-(2S,3S)-AHPBA-Pro-Ile-Val-NH₂ | | 20.6 |
| DD | PhCH₂CH₂CO-Asp(NMe₂)-(2S,3S)-AHPBA-Pro-Ile-Val-NH₂ | | 76.5 |
| 38 | PhCH₂CH₂CO-Asn-(2S,3S)-AHPBA-Pro-Val-Val-NH₂ | | 7.2 |
| 39 | PhCH₂CH₂CO-Asn-(2S,3S)-AHPBA-Pro-Leu-Val-NH₂ | | 48.2 |
| EE | PhCH₂CH₂CO-(2S,3S)-AHPBA-Pro-Ile-Val-NH₂ | | 74.2 |
| 41 | PhO-CH₂CO-Asn-(2S,3S)-AHPBA-Pro-Ile-Val-NH₂ | | 1.2 |
| 42 | Pyridine-CO-Asn-(2S,3S)-AHPBA-Pro-Ile-Val-NH₂ | | 6.4 |
| 43 | Quinoline-CO-Asn-(2S,3S)-AHPBA-Pro-Ile-Val-NH₂ | | 0.8 |
| 44 | H-Ser-Phe-Asn-(2S,3S)-AHPBA-Pro-Ile-Val-NH₂ | | 2.2 |
| 45 | H-Ser-Phe-Asn-(2R,3S)-AHPBA-Pro-Ile-Val-NH₂ | | 3.2 |
| 46 | Boc-Asn-(2S,3S)-AHPBA-Pro-Ile-NH-CH₂-C₆H₁₁ | | 51.9 |
| 47 | PhCH₂CH₂CO-Asn-(2S,3S)-AHPBA-Pro-Ile-NH-CH₂-C₆-H₁₁ | | 13.2 |
| 48 | Boc-(2S,3S)-AHPBA-Pro-NH-CH₂-C₆H₁₁ | 57.5 | |
| 49 | PhCH₂-O-CO-Asn-(2S,3S)-AHPBA-Pro-NH-CH₂-C₆H₁₁ | | 51.9 |
| FF | Boc-(2S,3S)-AHPBA-Pro-Gln-NH-CH₂-C₆H₁₁ | 62.9 | |
| 51 | Boc-Asn-(2S,3S)-AHPBA-Pro-Ile-NH-iBu | | 85.3 |
| GG | Boc-Val-(2R,3S)-AHPBA-Phe-Val-NH-iBu | | 94.6 |
| HH | PhCH₂-O-CO-Val-(2R,3S)-AHPBA-Phe-Val-NH-iBu | | 83.4 |
| JJ | PhCH₂-O-CO-Val-(2R,3S)-AHPBA-Phe-NH-iBu | | 84.9 |
| 55 | PhCH₂-O-CO-Asn-(2S,3S)-AHPBA-Pro-NH-tBu | | 3.5 |
| 56 | PhCH₂-O-CO-Asn-(2R,3S)-AHPBA-Pro-NH-tBu | | 95.1 |
| KK | PhCH₂CH₂CO-Asn-(2S,3S)-ACHBA-Pro-Ile-Val-NH₂ | | 25.7 |
| LL | PhCH₂CH₂CO-Asn-(2R,3S)-ACHBA-Pro-Ile-Val-NH₂ | | 81.3 |
| MM | PhCH₂CH₂CO-His-(2S,3S)-AHPBA-Pro-lle-Val-NH₂ | | 18.2 |
| 60 | PhCH₂CH₂CO-Ser(Me)-(2S,3S)-AHPBA-Pro-Ile-Val-NH₂ | | 10.6 |
| NN | PhCH₂CH₂CO-Smc(O)-(2S,3S)-AHPBA-Pro-Ile-Val-NH₂ | | 90.9 |
| 62 | PhCH₂CH₂CO-Msa-(2S,3S)-AHPBA-Pro-Ile-Val-NH₂ | | 3.2 |
| 63 | Fmoc-Asn-(2S,3S)-AHPBA-Pro-Ile-Val-NH₂ | | 1.0 |
| 64 | 1Nap-O-CH₂CO-Asn-(2S,3S)-AHPBA-Pro-Ile-Val-NH₂ | | 0.5 |
| 65 | Furan-CO-Asn-(2S,3S)-AHPBA-Pro-Ile-Val-NH₂ | 9.3 | |
| 66 | Pyrazine-CO-Asn-(2S,3S)-AHPBA-Pro-Ile-Val-NH₂ | 6.2 | |
| 67 | Thiophen-CO-Asn-(2S,3S)-AHPBA-Pro-Ile-Val-NH₂ | 4.9 | |
| 68 | H-Inc-Asn-(2S,3S)-AHPBA-Pro-Ile-Val-NH₂ | 5.5 | |
| 69 | H-(D)-Tic-Asn-(2S,3S)-AHPBA-Pro-Ile-Val-NH₂ | 29.0 | |

**Table 3**

| Example | Chemical Formula | Residual Activity (%) | |
|---|---|---|---|
| | | 1mM | 5mM |
| 70 | H-(L)-Tic-Asn-(2S,3S)-AHPBA-Pro-Ile-Val-NH₂ | 3.4 | |
| OO | PhCH₂CH₂CO-Asn-(2S,3S)-AHPBA(OMe)-Pro-Ile-Val-NH₂ | 95.5 | |
| PP | PhCH₂CH₂CO-Met(O)₂-(2S,3S)-AHPBA-Pro-Ile-Val-NH₂ | | 96.8 |
| 73 | PhCH₂CH₂CO-Ser-(2S,3S)-AHPBA-Pro-Ile-Val-NH₂ | 21.5 | |
| 74 | PhCH₂CH₂CO-Leu-(2S,3S)-AHPBA-Pro-Ile-Val-NH₂ | 31.3 | |
| 75 | PhCH₂CH₂CO-Asn-(2S,3S)-AHPBA-Pro-Gln-Ile-NH₂ | 62.0 | |
| 76 | PhCH₂CH₂CO-Asn-(2S,3S)-AHPBA-Pro-Val-NH₂ | 39.1 | |
| 77 | PhCH₂CH₂CO-Asn-(2S,3S)-AHPBA-Pro-Ile-NH₂ | 57.0 | |
| 78 | PhCH₂-O-CO-Asn-(2S,3S)-AHPBA-(L)-Pip-NH-tBu | 11.7 | |
| 79 | PhCH₂-O-CO-Asn-(2S,3S)-AHPBA-(D)-Pip-NH-tBu | 84.0 | |
| 80 | Boc-Asn-(2S,3S)-AHPBA-Pro-NH-tBu | 68.8 | |
| 81 | 1Nap-CH₂-O-CO-Asn-(2S,3S)-AHPBA-Pro-NH-tBu | 1.4 | |
| 82 | PhCH₂-O-CO-Asn-(2S,3S)-AHPBA-Thz-NH-tBu | 1.3 | |
| 83 | PhCH₂-O-CO-Asn-(2S,3S)-AHPBA-Pro-NH-CH₂-C(Me)₃ | 12.4 | |
| 84 | PhCH₂-O-CO-Asn-(2S,3S)-AHPBA-Pro-NH-C₆H₁₁ | 9.6 | |
| 85 | PhCH₂-O-CO-Asn-(2S,3S)-AHPBA-Pro-NH-iPr | 10.0 | |
| 86 | PhCH₂-O-CO-Asn-(2S,3S)-AHPBA-Pro-O-tBu | 12.6 | |
| 87 | PhCH₂-O-CO-Asn-(2S,3S)-AHPBA-Pro-NH-tAmyl | 5.8 | |
| 88 | PhCH₂-O-CO-Asn-(2S,3S)-AHPBA-Pro-NH-cyclopropyl | <10 | |
| 89 | PhCH₂-O-CO-Asn-(2S,3S)-AHPBA-Pro-NH-CH(C₂H₅)₂ | <10 | |
| 90 | 1Nap-CH₂-O-CO-Msa-(2S,3S)-AHPBA-Pro-NH-tBu | 1.5 | |
| 91 | 1Nap-O-CH₂CO-Asn-(2S,3S)-AHPBA-Pro-NH-tBu | | 20.5 |
| 92 | Fmoc-Asn-(2S,3S)-AHPBA-Pro-NH-tBu | | 53.5 |

**Table 4**

| Example | Chemical Formula | Residual Activity (%) | |
|---|---|---|---|
| | | 1mM | 5mM |
| 93 | PhCH₂-O-CO-Asn-(2S,3S)-AHPBA-Pro-Aib-NH₂ | <10 | |
| 94 | (p-ClPh)₂CH-O-CH₂CO-Asn-(2S,3S)-AHPBA-Pro-NH-tBu | | 37.0 |
| 95 | PhCH₂-O-CO-Asn-(2S,3S)-AHPBA-Hyp(Bzl)-NH-tBu | 34.6 | |
| 96 | PhCH₂-O-CO-Asn-(2S,3S)-AHPBA-Inc-NH-tBu | 95.2 | |
| QQ | Boc-Sma-(2S,3S)-AHPBA-Pro-NH-tBu | 92.9 | |
| RR | 1Nap-O-CH₂CO-Sma(2S,3S)-AHPBA-Pro-NH-tBu | | 70.5 |
| 99 | PhCH₂-O-CO-Asn-(2S,3S)-AHPBA-Pro-NH-C(Me)₂CH₂OH | | 62.2 |
| 100 | 1Nap-O-CH₂CO-Msa-(2S,3S)-AHPBA-Thz-NH-tBu | | 6.1 |
| 101 | 1NaP-O-CH₂CO-Asn-(2S,3S)-AHPBA-Thz-NH-tBu | | 8.7 |
| 102 | PhCH₂-O-CO-Asn-(2S,3S)-ASPBA-(L)-Tic-NH-tBu | | 89.2 |
| 103 | PhCH₂-O-CO-Asn-(2S,3S)-AHPBA-(D)-Tic-NH-tBu | | 91.4 |
| 104 | PhCH₂-O-CO-Asn-(2S,3S)-AHPBA-Dtc-NH-tBu | | 3.8 |
| 105 | 1Nap-O-CH₂CO-Msa-(2S,3S)-AHPBA-Dtc-NH-tBu | | 1.0 |
| 106 | 1Nap-O-CH₂-CO-Asn-(2S,3S)-AHPBA-Dtc-NH-tBu | | 1.1 |
| 107 | 1Nap-CH₃-O-CO-Asn-(2S,3S)-AHPBA-Dtc-NH-tBu | | 1.6 |
| 108 | (E)-Ph-CH=CH-CH₂CO-Asn-(2S,3S)-AHPBA-Dtc-NH-tBu | | 3.7 |
| 109 | oCl-Ph-O-CH₂CO-Asn-(2S-3S)-AHPBA-Dtc-NH-tBu | | 2.7 |
| 110 | oPh-Ph-O-CH₂CO-Asn-(2S,3S)-AHPBA-Dtc-NH-tBu | | 3.0 |
| 111 | mPh-Ph-O-CH₂CO-Asn-(2S,3S)-AHPBA-Dtc-NH-tBu | | 1.1 |
| 112 | pPh-Ph-O-CH₂CO-Asn-(2S,3S)-AHPBA-Dtc-NH-tBu | | 2.0 |
| 113 | mCl-Ph-O-CH₂CO-Asn-(2S-3S)-AHPBA-Dtc-NH-tBu | | 2.3 |
| 114 | 1Tna-O-CH₂CO-Asn-(2S,3S)-AHPBA-(Dtc-NH-tBu | | 2.1 |
| 115 | 5-isoquinoline-O-CH₂CO-Asn-(2S,3S)-AHPBA-Dtc-NH-tBu | | 2.4 |
| 116 | m-(Ph-NH)-Ph-O-CH₂CO-Asn-(2S,3S)-AHPBA-Dtc-NH-tBu | | 0.9 |
| 117 | 8Qoa-Asn-(2S,3S)-AHPBA-Dtc-NH-tBu | | 44.1 |
| 118 | Quinoline-CO-Asn-(2S,3S)-AHPBA-Dtc-NH-tBu | | 1.8 |
| 119 | 1Nap-O-CH₂CO-Mta-(2S,3S)-AHPBA-Dtc-NH-tBu | | 0.7 |
| 120 | 8Qoa-Mta-(2S,3S)-AHPBA-Dtc-NH-tBu | | 9.5 |
| SS | 1Nap-O-CH₂CO-Mta⁺ (Me)-(2S,3S)-AHPBA-Dtc-NH-tBu . AcO⁻ | | 32.0 |
| 122 | 1Nap-O-CH₂CO-Mta-(2S,3S)-AHPBA-Pro-NH-tBu | | 6.9 |
| 123 | 1Nap-NH-CH₂CO-Msa-(2S,3S)-AHPBA-Pro-NH-tBu · AcOH | | 44.6 |
| 124 | 1Nap-NH-CH₂CO-Msa-(2S,3S)-AHPBA-Thz-NH-tBu · AcOH | | 28.2 |
| 125 | 1Nap-O-CH₂CO-Msa-(2S,3S)-AHPBA-Thz-NH-C(Me)₂CH₂OH | | 8.2 |

**Table 5**

| Example | Chemical Formula | Residual Activity (%) | |
|---|---|---|---|
| | | 1mM | 5mM |
| 126 | 1Nap-O-CH₂CO-Msa-(2S,3S)-AHPBA-Pro-NH-C(CH₂OH)₂Me | | 67.9 |
| 127 | 1Nap-O-CH₂CO-Asn-(2S,3S)-AHPBA-Thz-piperidine | <10 | |
| 128 | 1Nap-O-CH₂CO-Asn-(2S,3S)-AHPBA-Thz-NH-cyclopropyl | | 10.5 |
| 129 | mPh-Ph-O-CH₂CO-Mta-(2S,3S)-AHPBA-Dtc-NH-tBu | | 0.8 |
| 130 | 5-Isoquinoline-O-CH₂CO-Mta-(2S,3S)-AHPBA-Dtc-NH-tBu · AcOH | | 0.9 |
| 131 | 2Nap-O-CH₂CO-Msa-(2S,3S)-AHPBA-Pro-NH-tBu | | 28.8 |
| TT | 1Nap-O-CH₂CO-Hse-(2S,3S)-AHPBA-Thz-NH-tBu | | |
| UU | 1Nap-O-CH₂CO-Thr-(2S,3S)-AHPBA-Thz-NH-tBu | | 54.7 |
| 134 | 1Nap-O-CH₂CO-Tle-(2S,3S)-AHPBA-Thz-NH-tBu | | 22.6 |
| 135 | 1Nap-O-CH₂CO-Msa-(2S,3S)-AHPBA-Thz-NH-CH(iPr)CH₂OH | | 19.3 |
| 136 | Benzofuran-CO-Msa-(2S,3S)-AHPBA-Thz-NH-tBu | | 19.5 |
| 137 | 1Nap-O-CH₂CO-Msa-(2S,3S)-AHPBA-Thz-NH-CH(sBu)CH₂OH | <10 | |
| 138 | Quinoline-CO-Asn-(2S,3S)-AHPBA-Pro-NH-tBu | | 27.3 |
| VV | 1Nap-O-CH₂-CO-Asp(NHNH₂)-(2S, 3S)-AHPBA-Pro-NH-tBu · AcOH | | 27.2 |
| 140 | 1-Isoquinoline-CO-Asn-(2S,3S)-AHPBA-Pro-NH-tBu · AcOH | | 72.8 |
| 141 | 1Nap-SO₂-Asn-(2S**,**3S)-AHPBA-Pro-NH-tBu | | 88.2 |
| 142 | 1Nap-O-CH₂CO-Msa-(2S,3S)-AHPBA-Thz-NH-tAmyl | | 11.7 |
| 143 | Biphenyl-CO-Msa-(2S,3S)-AHPBA-Thz-NH-tBu | <10 | |
| 144 | 1Nap-O-CH₂CO-Msa-(2S,3S)-AHPBA-3Dic-NH-tBu | | 95.9 |
| 145 | 1Nap-O-CH₂CO-Msa-(2S,3S)-AHPBA-1Dic-NH-tBu | | 96.6 |
| 146 | 1Nap-O-CH₂CO-Msa-(2S,3S)-AHPBA-Oic-NH-tBu | | 97.5 |
| 147 | 1Nap-O-CH₂CO-Msa-(2S,3S)-AHPBA-Pro-NH-Ph(o-OH) | | 96.7 |
| 148 | 1Nap-O-CH₂CO-Msa-(2S,3S)-AHPBA-Pro-NH-Ph-(m-OH) | | 88.9 |
| 149 | 1Nap-O-CH₂CO-Msa-(2S,3S)-AHPBA-Pro-NH-Ph(p-OH) | <10 | |
| 150 | 1Nap-O-CH₂CO-Msa-(2S,3S)-AHPBA-Hyp-NH-tBu | | 31.6 |
| 151 | 1Nap-O-CH₂CO-Msa-(2S,3S)-AHPBA-Hyp(Me)-NH-tBu | | 74.8 |
| 152 | 1Nap-O-CH₂CO-Msa-(2S,3S)-AHPBA-Hyp(Et)-NH-tBu | | 91.2 |
| 153 | 1Nap-O-CH₂CO-Msa-(2S,3S)-AHPBA-Hyp(Allyl)-NH-tBu | | 70.6 |
| WW | 1Nap-O-CH₂CO-Mtv-(2S,3S)-AHPBA-Thz-NH-tBu | | 35.4 |
| XX | 1Nap-O-CH₂CO-Msv-(2S,3S)-AHPBA-Thz-NH-tBu | | 7.3 |

**Table 6**

| Example | Chemical Formula | Residual Activity (%) | |
|---|---|---|---|
| | | 1mM | 5mM |
| 156 | 1Nap-O-CH₂CO-Msa-(2S,3S)-AHPBA-Thz-NH-CH(Ph)CH₂OH | | 94.5 |
| 157 | 1Nap-O-CH₂CO-Msa-(2S,3S)-AHPBA-Thz-Phg-NH₂ | | 93.9 |
| 158 | 1Nap-O-CH₂CO-Msa-(2S,3S)-AHPBA-Thz-NH-chex-ol | <10 | |
| 159 | 1Nap-O-CH₂CO-Msa-(2S,3S)-AHPBA-Thz-Pip-OMe | | 90.8 |
| YY | 1Nap-O-CH₂CO-Phg-(2S,3S)-AHPBA-Thz-NH-tBu | | 54.8 |
| 161 | 1Nap-O-CH₂CO-Ile-(2S,3S)-AHPBA-Thz-NH-tBu | | 7.0 |
| 162 | 1Nap-O-CH₂CO-Mta-(2S,3S)-AHPBA-Thz-NH-tBu | | 2.9 |
| 163 | 1Nap-O-CH₂CO-Thr(Me)-(2S,3S)-AHPBA-Thz-NH-tBu | | 5.7 |
| 164 | PhCH₂-O-CO-Asn-(2S,3S)-AHPBA-Pdp-NH-tBu | | 49.8 |
| 165 | 1Nap-O-CH₂CO-Nva-(2S,3S)-AHPBA-Thz-NH-tBu | | 10.7 |
| 166 | m-(iPr-O)-Ph-O-CH₂CO-Msa-(2S,3S)-AHPBA-Dtc-NH-tBu | | 2.0 |
| 167 | m-(Piper-CO)-Ph-O-CH₂CO-Msa-(2S,3S)-AHPBA-Thz-NH-tBu | | 25.4 |
| 168 | m-(Morph-CO)-Ph-O-CH₂CO-Msa-(2S,3S)-AHPBA-Thz-NH-tBu | | 44.5 |
| 169 | m-(iPr-O)-Ph-O-CH₂CO-Asn-(2S,3S)-AHPBA-Dtc-NH-tBu | | 1.4 |
| ZZ | 1Nap-O-CH₂CO-Alg-(2S,3S)-AHPBA-Thz-NH-tBu | | 7.3 |
| 171 | 2,3-diMe-Ph-O-CH₂CO-Asn-(2S,3S)-AHPBA-Dtc-NH-tBu | | 2.9 |
| 172 | 1Nap-O-CH₂CO-Msa-(2S,3S)-AHPBA-Thz-Gly-NH₂ | | 77.9 |
| 173 | 1Nap-O-CH₂CO-Msa-(2S,3S)-AHPBA-Thz-GABA-NH₂ | | 65.0 |
| 174 | 1Nap-O-CH₂CO-Msa-(2S,3S)-AHPBA-Thz-BAIB-NH₂ | | 46.6 |
| 175 | 1Nap-O-CH₂CO-Msa-(2S,3S)-AHPBA-Thz-BANB-NH₂ | | 33.0 |
| 176 | PhCH₂-O-CO-Asn-(2S,3S)-AHPBA-Pro-NH-sBu | | 82.6 |
| 177 | PhCH₂-O-CO-Asn-(2S,3S)-AHPBA-Dtc-NH₂ | | 74.2 |
| A' | PhCH₂-O-CO-Asn-(2S,3S)-AHPBA-Diq | | 8.6 |
| 179 | 1Nap-O-CH₂CO-Val-(2S,3S)-AHPBA-Thz-NH-tBu | | 5.5 |
| B' | 1Nap-O-CH₂CO-Prg-(2S,3S)-AHPBA-Thz-NH-tBu | | 26.0 |
| C' | 1Nap-O-CH₂CO-Aca-(2S,3S)-AHPBA-Thz-NH-tBu | | 73.0 |
| 182 | PhCH₂-O-CO-Asn-(2S,3S)-AHPBA-Dmp-NH-tBu | | 26.8 |
| 183 | 1Nap-O-CH₂CO-Msa-(2S,3S)-AHPBA-Dmp-NH-tBu | | 4.3 |
| 184 | PhCH₂-O-CO-Asn-(2S,3S)-AHPBA-Php-NH-tBu | | 16.8 |
| 185 | PhCH₂-O-CO-Asn-(2S,3S)-AHPBA-Cpp-NH-tBu | | 96.0 |
| 186 | PhCH₂-O-CO-Asn-(2S,3S)-AHPBA-Tcp-NH-tBu | | 99.5 |
| 187 | PhCH₂-O-CO-Asn-(2S,3S)-AHPBA-Ccp-NH-tBu | | 99.0 |
| 188 | PhCH₂-O-CO-Asn-(2S,3S)-AHPBA-Dmp-NH₂ | | 98.1 |

The compounds represented by the general formula (9) and pharmaceutically acceptable salts thereof can be prepared as HIV protease inhibitors by conventional methods with conventional carriers and fillers. For example, tablets, capsules and granules are orally administered and injection preparations are administered intravenously or intramuscularly. Furthermore, adhesive plasters, suppositories, sprays are used topically.

More specifically, the compounds of the invention can be administered by topical, intravenous, intraperitoneal, oral, and subcutaneous administration. The compounds of the invention may be administered to a domestic animal or to an animal such as a mammal (e.g. mouse, rat or human).

The compounds of the present invention can be made into pharmaceutical compositions by combination with appropriate medical carriers or diluents. For example, the compounds of the present invention can be dissolved in oils, propyleneglycol or other solvents commonly used to prepare injectable solutions. Suitable carriers include physiological saline, polyethylene glycol, ethanol, sesame oil, cremophor and isopropyl myristate. For topical application, the compounds of the invention can be formulated as an ointment or cream.

In terms of composition, compounds should be present between 0.1 to 100%, preferably 1 to 90% based on the total weight of the composition.

The compounds of the present invention in pharmaceutical dosage forms may be used in the form of their pharmaceutically acceptable salts, and also may be used alone or in appropriate association, as well as in combination with other pharmaceutically active compounds.

The compounds of the present invention may be formulated into preparations for injections by dissolving, suspending, or emulsifying them in aqueous solvents such as normal saline, Dextrose 5%, or non-aqueous solvent, such as vegetable oil, synthetic aliphatic acid glycerides, esters of higher aliphatic acids or propylene glycol; and if desired, with conventional additives such as solubilizers, isotonic agents, suspending agents, emulsifying agents, stabilizers and preservatives.

The compounds of the invention may be combined with other compounds having the desired effect.

The dosage may be suitably determined according to the symptoms, ages and sexes of the patients, and doses of 0.001-5 g per person in a day are generally used for adults in 1-5 divided portions. One of preferable administration is a method of absorbing through the nasal membrane by a nasal spray. In this case, a compound represented by the general formula (9) is dissolved in fluorocarbon or saline solution together with preservatives such as benzyl alcohol and absorption accelerating agent for the improved bioavailability and the resultant formulation can be administered by nasal aerosol or inhalation.

The peptide derivatives of the present invention represented by the general formula (9) and pharmaceutically acceptable salts thereof are presumed to have no acute toxicity.

The HIV protease inhibitors of the present invention markedly inhibit the HIV protease activity. In addition, they are stable against proteolytic enzymes due to their amino acid isostere. Therefore, the HIV protease inhibitors of the present invention are expected to be useful for the therapy of acquired immunodeficiency syndrome (AIDS) and prevention of HIV infection.

The invention will be explained in detail by the following examples:

### EXAMPLES

### Ref. Example A: (2RS,3S)-3-N-(t-butoxycarbonyl)amino-2-hydroxy-4-phenylbutanoic acid

In 15 ml of purified water, 1.49 g of hydrochloride of (2RS,3S)-3-amino-2-hydroxy-4-phenylbutanoic acid (hereinafter, the amino acid residue thereof is abbreviated as -AHPBA- ) was suspended, 1.75 ml of triethylamine was added under ice cooling, then 2.20 g of di-tert-butyl dicarbonate (Boc₂O) in 15 ml of tetrahydrofuran (THF) was added, and the resultant mixture was stirred for 14 hr. The reaction mixture was washed with ether and the aqueous layer was condensed to the half volume. The condensate was adjusted to pH 2-3 with citric acid, extracted with ethyl acetate, washed with saturated sodium chloride aqueous solution and dried over anhydrous sodium sulfate. The dried solution was condensed under reduced pressure and hexane was added to the residue to give 1.86 g of crystals of Boc-(2RS,3S)-AHPBA-OH,

### Comparative Example B,C,D,E,F,C,H,J

### [Process 1] H-AHPBA-NH-CH₂-C₆H₁₁.HCl

To a solution of 300 mg (1.15 mmol) of Boc-AHPBA-OH in 2.0 ml of N,N-dimethylformamide (DMF), and 162 µl (1.15 mmol) of cyclohexylmethylamine, 204 mg (1.15 mmol) of N-hydroxy-norbornene-2,3-dicarboximide (HONB) and 336 mg (1.73 mmol) of 1-ethyl-3-(3-N,N-dimethylaminopropyl)carbodiimide (EDC) hydrochloride were added, and the mixture was stirred for 14 hr. The reaction mixture was condensed and the residue was dissolved in ethyl acetate and washed successively with 1N-HCl, 5% aqueous solution of sodium hydrogencarbonate and saturated aqueous sodium chloride solution. The washed solution was dried over anhydrous sodium sulfate. The dried solution was evaporated to dryness under reduced pressure, mixed with 8.65 ml (34.59 mmol) of 4N-HCl dioxane solution under ice cooling and stirred for 60 min. The reaction mixture was condensed and ether was added to the residue to give precipitates. The precipitates was collected and purified using a silica gel column chromatography with CHCl₃:MeOH and treated with 4N-HCl dioxane solution to give the title compound (2R,3S: 181 mg, 2S,3S: 132 mg).

### [Process 2] Boc-Phe-Asn-OH

In a solution of 10 ml of DMF containing 500 mg (1.38 mmol) of Boc-Phe-succinimide ester (Boc-Phe-OSu), 5 ml of aqueous solution of H-Asn-OH.Et₃N [prepared from 365 mg (2.76 mmol) of H-Asn-OH and 384 µl (2.76 mmol) of Et₃N] was added under ice cooling and the mixture was stirred for 14 hr. The reaction mixture was evaporated under reduced pressure and the residue was dissolved in ethyl acetate, washed with 1N-HCl and saturated sodium chloride aqueous solution, dried over anhydrous sodium sulfate. The dried solution was condensed under reduced pressure and the oily residue was triturated with ether to give 364 mg of crystals of the title compound with a yield of 70%.

### [Process 3] Boc-Phe-Ser-OH

In a 10 ml solution of DMF containing 500 mg (1.38 mmol) of Boc-Phe-OSu was added 5 ml of an aqueous solution of H-Ser-OH.Et₃N [prepared from 290 mg (2.76 mmol) of H-Ser-OH and 384 µl (2.76 mmol) of Et₃N] and the mixture was stirred for 14 hr. The reaction mixture was evaporated and the residue was dissolved in ethyl acetate and washed with 1N-HCl and saturated sodium chloride aqueous solution, successively, together with salting out. The resultant solution was dried over anhydrous sodium sulfate, evaporated under reduced pressure, and the resulted residue was purified with a silica gel column chromatography (CHCl₃:MeOH). The eluate was mixed with ether and n-hexane to give crystals of 150 mg of the title compound with a yield of 31%.

### [Process 41 Compound of Comparative Example B-J

In a 2.0 ml solution of DMF containing 20 mg (0.065 mmol) of H-AHPBA-NH-CH₂-C₆H₁₁ hydrochloride obtained by the process 1 were added 9.0 µl (0.065 mmol) of Et₃N, 0.065 mmol of an amino acid or a peptide derivative (Boc-Asn-OH: 15.0 mg, Boc-Ser-OH: 13.3 mg, Boc-Phe-Asn-OH: 24.6 mg or Boc-Phe-Ser-OH: 22.8 mg), 11.6 mg (0.065 mmol) of HONB and 18.6 mg (0.097 mmol) of EDC.HCl, successively, and the resultant mixture was stirred for 14 hr. The reaction mixture was evaporated and purified by the following method (a) or (b). The used natural amino acids were L-form except otherwise stated and so forth.
(a) The residue was dissolved in ethyl acetate, washed with 1N-HCl and saturated aqueous sodium chloride solution solution, and dried over anhydrous sodium sulfate. The dried solution was evaporated under reduced pressure, purified with a silica gel column chromatography (CHCl₃:MeOH) and crystallized from ether or n-hexane.
(b) The residue was mixed with water, the formed precipitates were collected by filtration, dried, purified with a silica gel column chromatography (CHCl₃:MeOH), and crystallized from ether.

### Example K-Q

The compound obtained in Example B-J (Process 4) was stirred in 2-3 ml of 4N-HCl in dioxane at room temperature for 60 min, respectively. The reaction mixture was evaporated under reduced pressure, ether was added to the residue and the formed precipitates were collected by centrifugation. The resultant precipitates were dissolved in 1N-acetic acid, purified with a reversed-phase column chromatography and lyophilized to give powders of compounds of Example K-Q , respectively.

### Example 16: 3-Phenylpropionyl-Asn-(2S,3S)-AHPBA-Pro-Ile-Val-NH₂

### [Process 1] Diastereomeric separation of Boc-(2RS,3S)-AHPBA-O-benzyl

In 20 ml of DMF, 2.10 g of Boc-(2RS,3S)-AHPBA-OH obtained by the reference Example was dissolved. To the resultant solution were added under ice cooling 1.42 ml of dicyclohexylamine (DCHA) and 1.02 ml of benzyl bromide, successively, and the obtained mixture was stirred for 14 hr. The reaction mixture was filtered and the filtrate was evaporated under reduced pressure. The residue was dissolved in ethyl acetate, washed with 5% aqueous citric acid solution, 5% aqueous sodium hydrogen carbonate solution and saturated aqueous sodium chloride solution, successively, and dried over anhydrous sodium sulfate. The dried solution was evaporated under reduced pressure and the residue was subjected to a flash chromatography using 100 g of silica gel column and eluted with CHCl₃ to give 0.87 g of (2R,3S)-Boc-AHPBA-O-benzyl and 1.20 g of the (2S,3S)-isomer TLC: Rf 0.63 for (2R,3S) (chloroform : methanol = 60:1) TLC: Rf 0.41 for (2S,3S) (chloroform : methanol = 60:1)

### [Process 2] Debenzylation

In 10 ml of ethanol, 1.03 g of Boc-(2S,3S)-AHPBA-O-benzyl obtained by the process 1 was dissolved, hydrogen gas was introduced in the presence of 0.10 g of 10% palladium on charcoal and stirred for 60 min. The reaction mixture was filtered, the resultant filtrate was evaporated and crystallized by the addition of hexane to give 0.78 g of Boc-(2S,3S)-AHPBA-OH. Boc-(2R,3S)-AHPBA-OH was obtained from Boc-(2R,3S)-AHPBA-O-benzyl by a similar method.

### [Process 3] 3-Phenylpropionyl-Asn-(2S,3S)-AHPBA-Pro-Ile-Val-NH₂

Protected amino acids, Boc-Val-OH, Boc-Ile-OH, Boc-Pro-OH, Boc-(2S,3S)-AHPBA-OH and Boc-Asn-OH, and 3-phenylpropionic acid were successively condensed by a solid phase peptide synthetic method [see Peptide Chemistry, 1988, 123 (1989)] using p-methylbenzhydrylamine resin. The resultant protected peptide resin was treated with anhydrous hydrogen fluoride under ice cooling for 60 min in the presence of m-cresol. The hydrogen fluoride was removed, ether was added, the formed precipitates were extracted with 50% aqueous acetic acid solution and the resultant extract was lyophilized. The lyophilized dried powder was dissolved in a mixture of 50% aqueous acetic acid and methanol and the obtained solution was subjected to a reversed-phase HPLC (water-acetonitrile system except otherwise stated and so forth). The fractionated eluate was evaporated and lyophilized to give the title compound.
Analytical HPLC: 23.9 min (The condition was as follows) Column:YMC AM-302 (4.6 x 150 mm)
Solvent A: 0.1% trifluoroacetic acid aqueous solution
Solvent B: acetonitrile
Gradient: 10% B for 2 min, then B was increased in 1.67%/min
Flow rate: 0.7 ml/min
FAB-MS: 750 (M+1)

### Example 17: 3-Phenylpropionyl-Asn-(2R,3S)-AHPBA-Pro-Ile-Val-NH₂

The title compound was obtained by a solid phase method similar to Example 16 (Process 3).
Analytical HPLC: 25.3 min (For the condition, see Example 16.)
FAB-MS: 750 (M+1)

### Comparative Example R: H-Val-Val-(2R,3S)-AHPBA-Phe-Val-Val-NH₂

### Comparative Example S: H-Val-Vat-(2S,3S)-AHPBA-Phe-Val-Val-NH₂

The title compounds were obtained by a solid phase method similar to Example 16 (Process 3) using the protected amino acid, (2RS,3S)-AHPBA-OH. Compounds (2R,3S) and (2S,3S) were divided during reversed-phase HPLC fractionation .
Analytical HPLC (2R,3S): 20.5 min (For the condition, see: Example 16.)
Analytical HPLC (2S,3S): 21.5 min (For the condition, see: Example 16.)
FAB-MS: 738 (M+1)

### Example 20: Phenylacetyl-Ser-(2S,3S)-AHPBA-Pro-Ile-Val-NH₂

### Example 21: Phenylacetyl-Ser-(2R,3S)-AHPBA-Pro-Ile-Val-NH₂

The title compounds were obtained by a solid phase method similar to Example 16 (Process 3). Compounds (2R,3S) and (2S,3S) were divided during reversed-phase HPLC fractionation.
Analytical HPLC (2R,3S): 21.97 min (For the condition, see: Example 16.)
Analytical HPLC (2S,3S): 20.49 min (For the condition, see: Example 16.)
FAB-MS: 709 (M+1)

### Comparative Example T: H-Val-Val-(2S,3S)-AHPBA-(D)-Phe-(D)-Val-(D)-Val-NH₂

### Comparative Example U: H-Val-Val-(2R,3S)-AHPBA-(D)-Phe-(D)-Val-(D)-Val-NH₂

The title compounds were obtained by a solid phase method similar to Example 16 (Process 3). Compounds (2R,3S) and (2S,3S) were divided during reversed-phase HPLC fractionation.
Analytical HPLC (2R,3S): 20.48 min (For the condition, see: Example 16.)
Analytical HPLC (2S,3S): 20.98 min (For the condition, see: Example 16.)
FAB-MS: 738 (M+1)

### Comparative Example V: H-Val-Val-(2S,3S)-AHPBA-(Bzl)Gly-Val-Val-NH₂

### Comparative Example W: H-Val-Val-(2R,3S)-AHPBA-(Bzl)Gly-Val-Val-NH₂

The title compounds were obtained by a solid phase method similar to Example 16 (Process 3). Compounds (2R,3S) and (2S,3S) were divided during reversed-phase HPLC fractionation.
Analytical HPLC (2R,3S): 23.09 min (For the condition, see: Example 16.)
Analytical HPLC (2S,3S): 23.45 min (For the condition; see: Example 16.)
FAB-MS: 738 (M+1)

### Example 26: 3-Phenylpropionyl-Ser-(2R,3S)-AHPBA-Pro-Ile-Val-NH₂

The title compound was obtained by a solid phase method similar to Example 16 (Process 3).
Analytical HPLC: 25.96 min (For the condition, see: Example 16.)
FAB-MS: 723 (M+1)

### Example 27: 3-Phenylpropionyl-Asn-(2S,3S)-AHPBA-Pro-Melle-Val-NH₂

The title compound was obtained by a solid phase method similar to Example 16 (Process 3).
Analytical HPLC: 16.85 min (For the condition, see: Example 16.)
FAB-MS: 764 (M+1)

### Comparative Example X: Boc-(2S,3S)-AHPBA-Pro-Ile-O-C₆H₁₁

### Comparative Example Y: Boc-(2R,3S)-AHPBA-Pro-Ile-O-C₆H₁₁

### [Process 1] pMZ-Ile-O-C₆H₁₁

In a methylene chloride solution of 1.00 g of N-(p-methoxybenzyloxycarbonyl)isoleucine (pMZ-Ile-OH), 0.35 ml of cyclohexanol and 0.84 g of N,N-dicyclohexylcarbodiimide (DCC) were added in the presence of 4 mg of dimethylaminopyridine and the resultant mixture was stirred for 2 hr under ice cooling. The reaction mixture was filtered and the filtrate was washed with 5% citric acid aqueous solution, 5% sodium hydrogencarbonate aqueous solution and saturated sodium chloride aqueous solution, successively, and dried over anhydrous sodium sulfate. The dried solution was evaporated under reduced pressure and the residue was subjected to a silica gel column chromatography (chloroform) to give 1.01 g of pMZ-Ile-O-C₆H₁₁.
TLC: Rf 0.56 (chloroform)

### [Process 2] Boc-Pro-Ile-O-C₆H₁₁

To 207 mg of the protected amino acid obtained by the [process 1], 4 ml of 4N-HCl in dioxane was added in the presence of 100 µl of anisole and the resultant mixture was stirred for 60 min. The reaction mixture was evaporated under reduced pressure and the resultant residue was redissolved in 4 ml of DMF and neutralized with 76 µl of triethylamine under ice cooling. To the neutralized solution, 118 mg of Boc-Pro-OH, 84 mg of N-hydroxybenzotriazol (HOBt) and 126 mg of EDC hydrochloride were added and the obtained mixture was stirred for 14 hr. The reaction mixture was evaporated under reduced pressure and the resultant residue was redissolved in ethyl acetate. The ethyl acetate solution was washed with 5% citric acid aqueous solution, 5% sodium hydrogencarbonate aqueous solution and saturated sodium chloride aqueous solution, successively, and dried over anhydrous sodium sulfate. The dried solution was evaporated under reduced pressure and the residue was subjected to a silica gel column chromatography (chloroform) to give 130 mg of the title compound.
TLC: Rf 0.61 (chloroform : methanol = 60:1)

### [Process 3] Boc-(2S,3S)-AHPBA-Pro-Ile-O-C₆H₁₁ and Boc-(2R,3S)-AHPBA-Pro-Ile-O-C₆H₁₁

To 130 mg of the protected peptide obtained by the process 2, 2 ml of 4N-HCl in dioxane was added and the resultant mixture was stirred for 60 min at room temperature. The reaction mixture was evaporated under reduced pressure and the resultant residue was redissolved in 2 ml of DMF and neutralized with 44 µl of triethylamine under ice cooling. To the neutralized solution, 94 mg of Boc-(2RS,3S)-AHPBA-OH, 140 mg of benzotriazol-1-yloxy tris(N,N,-dimethylaminophosphonium) hexafluorophosphate [Bop reagent] and 88 µl of triethylamine were added and the resultant mixture was stirred for 2 hr. The reaction mixture was treated similarly to that in the process 2, except for the chromatography solvent (chloroform : methanol = 50:1), to give 54 mg of Boc-(2R,3S)-AHPBA-Pro-Ile-O-C₆H₁₁ and 58 mg of Boc-(2S,3S)-AHPBA-Pro-Ile-O-C₆H₁₁.
TLC: Rf 0.78, 0.46 (chloroform : methanol = 60:1)
FAB-MS: 588 (M+1)

### Comparative Example Z: Boc-(2S,3S)-AHPBA-Pro-Ile-NH-CH₂-C₆H₁₁

### [Process 1] pMZ-Ile-NH-CH₂-C₆H₁₁

In a 10 ml of DMF solution containing 1.00 g of HONB ester of pMZ-isoleucine, 0.28 ml of cyclohexylmethylamine and 0.27 ml of N-methylmorpholine were added under ice cooling and the resultant mixture was stirred for 2 hr. The reaction mixture was evaporated under reduced pressure, purified water was added to the residue, the formed precipitates were collected and reprecipitated from DMF and ether to give 0.64 g of the title compound.
TLC: Rf 0.63 (chloroform : methanol = 40:1)

### [Process 2] H-Ile-NH-CH₂-C₆H₁₁

In 2 ml of trifluoroacetic acid, 0.50 g of the product obtained by the process 1 was added in the presence of 0.25 ml of anisole under ice cooling and stirred for 60 min. The reaction mixture was evaporated under reduced pressure, redissolved in 5 ml of DMF and neutralized by the addition of triethylamine under ice cooling to prepare a solution of the title compound.

### [Process 3] Boc-Pro-Ile-NH-CH₂-C₆H₁₁

In a solution prepared of 0.33 g of Boc-Pro-OH and 2 ml of DMF, 0.23 ml of triethylamine and 0.22 ml of isobutyl chloroformate were added at -15 °C and stirred for 10 min. The reaction solution was added to the entire solution prepared by the process 2 and the resultant mixture was stirred for 60 min. The reaction mixture was treated similarly to that in Comparative Example X (Process 1) and recrystallized from hexane to give the title compound.
TLC : Rf 0.38 (chloroform : methanol = 20:1)

### [Process 4] Boc-(2S,3S)-AHPBA-Pro-Ile-NH-CH₂-C₆H₁₁

Deprotection of 50 mg of the compound obtained by the process 3 was performed similarly to that in Comparative Example X (Process 3), and the obtained product was dissolved in 3 ml of DMF and neutralized with 17 µl of triethylamine under ice cooling. To the neutralized solution, 35 mg of Boc-(2S,3S)-AHPBA-OH, 52 mg of Bop reagent and 34 µl of triethylamine were added and the resultant mixture was stirred for 2 hr. The reaction mixture was treated similarly to that in Comparative Example X (Process 2),except for the chromatography solvent (chloroform : methanol = 30:1), to give 68 mg of the title compound.
TLC: Rf 0.41 (chloroform : methanol = 20:1)

### Comparative Example AA: Boc-(2R,3S)-AHPBA-Pro-Ile-NH-CH₂-C₆H₁₁

The title compound was synthesized by a similar method of Comparative Example Z
TLC: Rf 0.57 (chloroform :methanol = 20:1)
FAB-MS: 601 (M+1)

### Example 32: Benzyloxycarbonyl-Asn-(2S,3S)-AHPBA-Pro-Ile-NH-CH₂-C₆H₁₁

Deprotection of 68 mg of the compound obtained by Comparative Example Z (Process 4) was performed similarly to that in Comparative Example X (Process 3), and the obtained product was dissolved in 4 ml of DMF and neutralized with 16 µl of triethylamine under ice cooling. To the neutralized solution, 30 mg of N-(benzyloxycarbonyl)asparagine, 17 mg of HOBt, 50 mg of Bop reagent and 39 µl of triethylamine were added and the resultant mixture was stirred for 2 hr. The reaction mixture was treated similarly to that in Comparative Example Z (Process 4) to give 30 mg of the title compound.
TLC : Rf 0.40 (chloroform : methanol = 9:1)
FAB-MS : 749 (M+1)

### Comparative Example BB: Boc-(2S,3S)-AHPBA-(cHexm)Gly-Ile-NH-CH₂-C₆H₁₁

### Comparative Example CC: Boc-(2R,3S)-AHPBA-(cHexm)Gly-Ile-NH-CH₂-C₆H₁₁

### [Process 1] Boc-(cHexm)Gly-OH cyclohexylamine salt

In a methanol solution containing 2.0 g of H-Gly-OMe.HCl, 2.12 ml of cyclohexanecarboxaldehyde was added and the resultant mixture was stirred overnight in H₂ atomosphere in the presence of 200 mg of 10% palladium-charcoal. The reaction mixture was filtered and the filtrate was evaporated under reduced pressure to give N-(cyclohexylmethyl)glycine methyl ester [H-(cHexm)Gly-OMe]. The CHCl₃ solution of the amino ester obtained above was mixed with 3.32 ml of triethylamine and 4.18 g of Boc₂O under ice cooling and the mixture was stirred for 3 hr. The stirred mixture was washed with 5% aqueous citric acid solution, 5% aqueous sodium hydrogencarbonate solution, and saturated aqueous sodium chloride solution, successively, and dried over anhydrous sodium sulfate. The dried solution was evaporated under reduced pressure. The residue was subjected to a silica gel column chromatography (chloroform) to give oily Boc-(cHexm)Gly-OMe. The oily product was dissolved in methanol and 11.4 ml of 1N-NaOH aqueous solution was added and the resultant solution was stirred for 2 hr at room temperature. The obtained solution was neutralized with citric acid, evaporated and dissolved in ethyl acetate. The ethyl acetate solution was washed with 5% aqueous citric acid solution and saturated aqueous sodium chloride solution, successively, and dried over anhydrous sodium sulfate. The dried solution was evaporated under reduced pressure. The residue was dissolved in methanol and cyclohexylamine was added to the solution, then the resultant solution was evaporated and crystallized from ether to give 1.75 g of the title compound.
TLC: Rf 0.71 (chloroform : methanol:acetic acid = 9:1:0.5)

### [Process 2] Boc-(cHexm)GlY-Ile-NH-CH₂C₆H₁₁

Deprotection of 100 mg of the compound obtained by Comparative Example Z (Process 1) was performed similarly to that in Comparative Example X (Process 3) in the presence of 50 µl of anisol, and the obtained product was dissolved in 5 ml of DMF and neutralized with 36 µl of triethylamine under ice cooling. To the neutralized solution, Boc-(cHexm)Gly-OH obtained from 114 mg of the compound obtained by the process 1, 39 mg of HOBt and 59 mg of EDC hydrochloride were added and the obtained mixture was stirred for 14 hr. The reaction mixture was treated similarly to that in Comparative Example X (Process 2) to give 115 mg of the title compound.

### [Process 3] Boc-(2S,3S)-AHPBA-(cHexm)Gly-Ile-NH-CH₂-C₆H₁₁ and Boc-(2R,3S)-AHPBA-(cHexm)Gly-Ile-NH-CH₂-C₆H₁₁

Deprotection of 50 mg of the compound obtained by the process 2 was performed similarly to that in Comparative Example X (Process 3) , and the obtained product was dissolved in 5 ml of DMF and neutralized with 14 µl of triethylamine under ice cooling. To the neutralized solution, 31 mg of Boc-(2RS,3S)-AHPBA-OH, 46 mg of Bop reagent and 28 µl of triethylamine were added and the resultant mixture was stirred for 2 hr. The reaction mixture was treated similarly to that in Comparative Example X (Process 2), except for the chromatography solvent (chloroform : methanol = 100:1), to give 20.3 mg of Boc-(2S,3S)-AHPBA-(cHexm)Gly-Ile-NH-CH₂-C₆H₁₁ and 9.8 mg of Boc-(2R,3S)-AHPBA-(cHexm)Gly-Ile-NH-CH₂-C₆H₁₁.
TLC: Rf 0.61, 0.46 (chloroform : methanol = 20:1)
FAB-MS: 657 (M+1)

### Example 35: 3-Phenylpropionyl-Asn-(2S,3S)-AHPBA-Pro-β-Ala-NH₂

The title compound was obtained by a solid phase method similar to Example 16 (Process 3).

Analytical HPLC: 12.33 min (The condition was as follows) Column:YMC AM-302 (4.6 x 150 mm)
Solvent A: 0.1% trifluoroacetic acid aqueous solution
Solvent B: acetonitrile
Gradient: 20% B for 2 min, then B was increased in 2% /min
Flow rate: 0.7 ml/min
FAB-MS: 609 (M+1)

### Example 36: 3-Phenylpropionyl-Gln-(2S,3S)-AHPBA-Pro-Ile-Val-NH₂

The title compound was obtained by a solid phase method similar to Example 16 (Process 3).
Analytical HPLC: 16.68 min (For the condition, see: Example 35)
FAB-MS: 764 (M+1)

### Comparative Example DD : 3-Phenylpropionyl-Asp(NMe₂)-(2S,3S)-AHPBA-Pro-Ile-Val-NH₂

The title compound was obtained by a solid phase method similar to Example 16 (Process 3).
Analytical HPLC: 18.88 min (For the condition, see: Example 35)
FAB-MS: 778 (M+1)

### Example 38: 3-Phenylpropionyl-Asn-(2S,3S)-AHPBA-Pro-Val-Val-NH₂

The title compound was obtained by a solid phase method similar to Example 16 (Process 3).
Analytical HPLC: 15.97 min (For the condition, see: Example 35)
FAB-MS: 736 (M+1)

### Example 39: 3-Phenylpropionyl-Asn-(2S,3S)-AHPBA-Pro-Leu-Val-NH₂

The title compound was obtained by a solid phase method similar to Example 16 (Process 3).
Analytical HPLC: : 17.68 min (For the condition, see: Example 35)
FAB-MS: 750 (M+1)

### Comparative Example EE: 3-Phenylpropionyl-(2S,3S)-AHPBA-Pro-Ile-Val-NH₂

The title compound was obtained by a solid phase method similar to Example 16 (Process 3).
Analytical HPLC: 21.84 min (For the condition, see: Example 35)
FAB-MS: 636 (M+1)

### Example 41: Phenoxyacetyl-Asn-(2S,3S)-AHPBA-Pro-Ile-Val-NH₂

The title compound was obtained by a solid phase method similar to Example 16 (Process 3).
Analytical HPLC: 16.57 min (For the condition, see: Example 35)
FAB-MS: 752 (M+1)

### Example 42: 2-Pyridinecarbonyl-Asn-(2S,3S)-AHPBA-Pro-Ile-Val-NH₂

The title compound was obtained by a solid phase method similar to Example 16 (Process 3).
Analytical HPLC: 13.64 min (For the condition, see: Example 35)
FAB-MS: 723 (M+1)

### Example 43: 2-Quinolinecarbonyl-Asn-(2S,3S)-AHPBA-Pro-Ile-Val-NH₂

The title compound was obtained by a solid phase method similar to Example 16 (Process 3).
Analytical HPLC: 17.81 min (For the condition, see: Example 35)
FAB-MS: 773 (M+1)

### Example 44: H-Ser-Phe-Asn-(2S,3S)-AHPBA-Pro-Ile-Val-NH₂

The title compound was obtained by a solid phase method similar to Example 16 (Process 3).
Analytical HPLC: 19.13 min (For the condition, see: Example 16)
FAB-MS: 852 (M+1)

### Example 45: H-Ser-Phe-Asn-(2R,3S)-AHPBA-Pro-Ile-Val-NH₂

The title compound was obtained by a solid phase method similar to Example 16 (Process 3).
Analytical HPLC: 21.54 min (For the condition, see: Example 16)
FAB-MS: 852 (M+1)

### Example 46: Boc-Asn-(2S,3S)-AHPBA-Pro-Ile-NH-CH₂-C₆H₁₁

Deprotection of 81 mg of the compound obtained by Example 30 (Process 4) was performed similarly to that in Example 28 (Process 3), and the obtained product was dissolved in 5 ml of DMF and neutralized with 17 µl of triethylamine under ice cooling. To the neutralized solution, 33 mg of Boc-Asn-OH, 22 mg of HOBt and 41 mg of EDC hydrochloride were added and the resultant mixture was stirred for 14 hr. The reaction mixture was treated similarly to that in Example 28(Process 2),except for the chromatography solvent (chloroform : methanol = 20:1) to give 41 mg of the title compound.
TLC: Rf 0.36 (chloroform : methanol = 9:1)

### Example 47: 3-Phenylpropionyl-Asn-(2S,3S)-AHPBA-Pro-Ile-NH-CH₂-C₆H₁₁

Deprotection of 33 mg of the compound obtained by Example 46 was performed similarly to that in Comparative Example X (Process 3), and the obtained product was dissolved in 4 ml of DMF and neutralized with 6 µl of triethylamine under ice cooling. To the neutralized solution, 7 mg of phenylpropionic acid, 20 mg of Bop reagent and 13 µl of triethylamine were added and the resultant mixture was stirred for 2 hr. The reaction mixture was treated similarly to that in Example 46 to give 6 mg of the title compound.
TLC: Rf 0.82 (chloroform : methanol = 9:1)
Analytical HPLC: 24.50 min (For the condition, see Example 16)
FAB-MS: 747 (M+1)

### Example 48: Boc-(2S,3S)-AHPBA-Pro-NH-CH₂-C₆-H₁₁

### [Process 1] Boc-Pro-NH-CH₂C₆H₁₁

In a DMF solution containing 1.0 g of Boc-Pro-OH, 0.6 ml of cyclohexylmethylamine, 0.83 g of HOBt and 1.07 g of EDC hydrochloride were added under ice cooling, and the resultant mixture was stirred for 14 hr. The reaction mixture was treated similarly to that in Example 46 to give the title compound.
TLC: Rf = 0.77 (chloroform : methanol = 20:1)

### [Process 2] Boc-(2S,3S)-AHPBA-Pro-NH-CH₂-C₆H₁₁

Deprotection of 50 mg of the compound obtained by the process 1 was performed similarly to that in Example 28 (Process 3), and the obtained product was dissolved in 3 ml of DMF and neutralized with 22 µl of triethylamine under ice cooling. To the neutralized solution, 48 mg of Boc-(2S,3S)-AHPBA-OH, 71 mg of Bop reagent and 45 µl of triethylamine were added and the resultant mixture was stirred for 2 hr. The reaction mixture was treated similarly to that in Example 46 to give 62 mg of the title compound.
TLC: Rf 0.57 (chloroform : methanol = 9:1)

### Example 49: 3-Phenylpropionyl-Asn-(2S,3S)-AHPBA-Pro-NH-CH₂--C₆-H₁₁

Deprotection of 62 mg of the compound obtained by Example 48 was performed similarly to that in Example 28 (Process 3), and the obtained product was dissolved in 5 ml of DMF and neutralized with 18 µl of triethylamine under ice cooling. To the neutralized solution, 98 mg of p-nitrophenyl ester of benzyloxycarbonyl-Asn-OH [benzyloxycarbonyl-Asn-ONp], 39 mg of HOBt and 28 µl of N-methylmorpholine were added and the resultant mixture was stirred for 14 hr. The reaction mixture was treated similarly to that in Example 46, except for the chromatography solvent (chloroform : methanol = 10:1), and crystallized from ether to give 49 mg of the title compound.
TLC: Rf 0.41 (chloroform : methanol = 9:1)
FAB-MS: 636 (M+1)

### Comparative Example FF: Boc-(2S,3S)-AHPBA-Pro-Gln-NH-CH₂C₆H₁₁

### [Process 1] pMZ-Gln-NH-CH₂-C₆H₁₁

In a DMF solution containing 1.0 g of pMZ-Gln-ONp, 0.3 ml of cyclohexylmethylamine and 0.35 ml of triethylamine were added under ice cooling and the resultant mixture was stirred for 14 hr. The reaction mixture was evaporated under reduced pressure and water was added to the resultant residue to give a solid mass. The solid mass was reprecipitated from DMF/ethyl acetate to give 0.71 g of the title compound.
TLC: Rf 0.52 (chloroform : methanol:H₂O = 8:3:1, lower layer)

### [Process 2] Boc-Pro-Gln-NH-CH₂-C₆H₁₁

Deprotection of 710 mg of the compound obtained by the process 1 was performed similarly to that in Comparative Example Z (Process 2), and the obtained product was dissolved in 3 ml of DMF and neutralized with triethylamine under ice cooling. To the neutralized solution, a mixed anhydride prepared from 452 mg of Boc-Pro-OH, 321 µl of triethylamine and 300 µl of isobutyl chloroformate was added and the resultant mixture was stirred for 1 hr. The reaction mixture was evaporated under reduced pressure and the resultant residue was redissolved in ethyl acetate. The ethyl acetate solution was washed with 5% citric acid aqueous solution, 5% sodium hydrogencarbonate aqueous solution and saturated sodium chloride aqueous solution, successively, and dried over anhydrous sodium sulfate. The dried solution was evaporated under reduced pressure and the residue was crystallized from ether to give 550 mg of the title compound.
TLC: Rf 0.57 (chloroform : methanol:H₂O = 8:3:1, lower layer)

### [Process 3] Boc-(2S,3S)-AHPBA-Pro-Gln-NH-CH₂C₆H₁₁

Deprotection of 100 mg of the compound obtained by the process 2 was performed similarly to that in Comparative Example X (Process 3), and the obtained product was dissolved in 2 ml of DMF and neutralized with 32 µl of triethylamine under ice cooling. To the neutralized solution, 67 mg of Boc-(2S,3S)-AHPBA-OH, 101 mg of Bop reagent, and 64 µl of triethylamine were added and the resultant mixture was stirred for 2 hr. The reaction mixture was treated similarly to that in Example 46 to give 73 mg of the title compound.
FAB-MS: 616 (M+1)

### Example 51: Boc-Asn-(2S,3S)-AHPBA-Pro-Ile-NH-CH₂-CH(CH₃)₂

### [Process 1] Boc-Ile-NH-CH₂-CH(CH₃)₂

In a DMF solution containing 2.0 g of Boc-Ile-OH, 0.82 ml of isobutylamine, 1.27 g of HOBt and 2.06 g of DCC were added under ice cooling and the resultant mixture was stirred for 14 hr. The reaction mixture, after filtration, was treated similarly to that in Example 50 (Process 2),except for the crystallization solvent (hexane) to give 1.44 g of the title compound.

### [Process 2] Boc-Pro-Ile-NH-CH₂-CH(CH₃)₂

Deprotection of 1.44 g of the compound obtained by the process 1 was performed similarly to that in Example 28 (Process 3), and the obtained product was dissolved in 3 ml of DMF and neutralized with triethylamine under ice cooling. To the neutralized solution, 1.10 g of Boc-Pro-OH, 0.77 g of HOBt and 1.25 g of DCC were added and the resultant mixture was stirred for 14 hr. The reaction mixture was filtered and the filtrate was evaporated under reduced pressure and redissolved in ethyl acetate. The ethyl acetate solution was washed with 5% citric acid aqueous solution, 5% sodium hydrogencarbonate aqueous solution and saturated sodium chloride aqueous solution, successively, and dried over anhydrous sodium sulfate. The dried solution was evaporated under reduced pressure to give 1.00 g of the title compound.

### [Process 3] Boc-(2S,3S)-AHPBA-Pro-Ile-NH-CH₂-CH(CH₃)₂

Deprotection of 50 mg of the compound obtained by the process 2 was performed similarly to that in Example 28 (Process 3), and the obtained product was dissolved in 2 ml of DMF and neutralized with 18 µl of triethylamine under ice cooling. To the neutralized solution, 38 mg of Boc-(2S,3S)-AHPBA-OH, 57 mg of Bop reagent, and 36 µl of triethylamine were added and the resultant mixture was stirred for 2 hr. The reaction mixture was treated similarly to that in Example 46 to give 57 mg of the title compound.
TLC: Rf 0.77 (chloroform : methanol = 9:1)

### [Process 4] Boc-Asn-(2S,3S)-AHPBA-Pro-Ile-NH-CH₂-CH(CH₃)₂

Deprotection of 51 mg of the compound obtained by the process 3 was performed similarly to that in Comparative Example X (Process 3), and the obtained product was dissolved in 5 ml of DMF and neutralized with 13 µl of triethylamine under ice cooling. To the neutralized solution, 64 mg of p-nitrophenyl ester of Boc-Asn-OH, 14 mg of HOBt and 20 µl of N-methylmorpholine were added and the resultant mixture was stirred for 14 hr. The reaction mixture was treated similarly to that in Comparative Example FF (Process 2) to give 45 mg of the title compound.
TLC: Rf 0.39 (chloroform : methanol = 9:1)
FAB-MS: 675 (M+1)

### Comparative Example GG: Boc-Val-(2R,3S)-AHPBA-Phe-Val-NH-CH₂-CH(CH₃)₂

### [Process 1] Boc-Val-NH-CH₂CH(CH₃)₂

In a DMF solution containing 2.0 g of Boc-Val-OH, 0.92 ml of isobutylamine, 1.40 g of HOBt and 2.28 g or DCC were added under ice cooling and the resultant mixture was stirred for 14 hr. The reaction mixture was treated similarly to that in Example 51 (Process 1) to give 1.89 g of the title compound.

### [Process 2] Boc-Phe-Val-NH-CH₂-CH(CH₃)₂

Deprotection of 1.89 g of the compound obtained by the process 1 was performed similarly to that in Comparative Example X (Process 3), and the obtained product was dissolved in 3 ml of DMF and neutralized with triethylamine under ice cooling. To the neutralized solution, 1.85 g of Boc-Phe-OH, 1.06 g of HOBt and 1.72 g of DCC were added and the resultant mixture was stirred for 14 hr. The reaction mixture was filtered and the filtrate was evaporated under reduced pressure and water was added to the residue to give a solid mass. The mass was washed with water and reprecipitated from THF-ether to give 2.20 g of the title compound.

### [Process 3] Boc-(2R,3S)-AHPBA-Phe-Val-NH-CH₂-CH(CH₃)₂

Deprotection of 300 mg of the compound obtained by the process 2 was performed similarly to that in Comparative Example X (Process 3), and the obtained product was dissolved in 10 ml of DMF and neutralized with 99 µl of triethylamine under ice cooling. To the neutralized solution, 211 mg of Boc-(2R,3S)-AHPBA-OH, 316 mg of Bop reagent, and 198 µl of triethylamine were added and the resultant mixture was stirred for 2 hr. The reaction mixture was treated similarly to that in Comparative Example Z (Process 4) to give 94 mg the title compound.
TLC: Rf 0.41 (chloroform : methanol = 9:1)

### [Process 4] Boc-Val-(2R,3S)-AHPBA-Phe-Val-NH-CH₂-CH(CH₃)₂

Deprotection of 30 mg of the compound obtained by the process 3 was performed similarly to that in Comparative Example X (Process 3), and the obtained product was dissolved in 3 ml of DMF and neutralized with 7 µl of triethylamine under ice cooling. To the neutralized solution, 11 mg of Boc-Val-OH, 22 mg of Bop reagent and 14 µl of triethylamine were added and the resultant mixture was stirred for 2 hr. The reaction mixture was treated similarly to that in Example FF (Process 2) and the obtained precipitates were reprecipitated from DMF-ether to give 17 mg of the title compound.
TLC: Rf 0.88 (chloroform : methanol = 9:1)
FAB-MS: 696 (M+1)

### Comparative Example HH: Benzyloxycarbonyl-Val-(2R,3S)-AHPBA-Phe-Val-NH-CH₂-CH(CH₃)₂

Deprotection of 30 mg of the compound obtained by Example 52 (Process 3) was performed similarly to that in Comparative Example X (Process 3), and the obtained product was dissolved in 3 ml of DMF and neutralized with 7 µl of triethylamine under ice cooling. To the neutralized solution, 21 mg of benzyloxycarbonyl-Val-OH.DCHA, 22 mg of Bop reagent and 7 µl of triethylamine were added and the resultant mixture was stirred for 2 hr. The reaction mixture was evaporated under reduced pressure and water was added to the resultant residue to give precipitates. The precipitates were washed with water and reprecipitated from THF-ether to give 6 mg of the title compound.
TLC: Rf 0.88 (chloroform : methanol = 20:1)
FAB-MS: 730 (M+1)

### Comparative Example JJ: Benzyloxycarbonyl-Val-(2R,3S)-AHPBA-Phe-NH-CH₂-CH(CH₃)₂

### [Process 1] Boc-Phe-NH-CH₂CH(CH₃)₂

In a DMF solution containing a mixed anhydride prepared from 2.0 g of Boc-Phe-OH, 1.15 ml of triethylamine and 1.08 ml of isobutyl chloroformate, 1.50 ml of isobutylamine was added under ice cooling and the resultant mixture was stirred for 14 hr. The reaction mixture was treated similarly to that in Example 51 (Process 1) to give 1.91 g of the title compound.
TLC: Rf 0.82 (chloroform : methanol = 20:1)

### [Process 2] Boc-(2R,3S)-AHPBA-Phe-NH-CH₂-CH(CH₃)₂

Deprotection of 100 mg of the compound obtained by the process 1 was performed similarly to that in Comparative Example X (Process 3), and the obtained product was dissolved in 5 ml of DMF and neutralized with 43 µl of triethylamine under ice cooling. To the neutralized solution, 92 mg of Boc-(2R,3S)-AHPBA-OH, 138 mg of Bop reagent and 87 µl of triethylamine were added and the resultant mixture was stirred for 2 hr. The reaction mixture was evaporated under reduced pressure and water was added to the resultant residue to give precipitates. The precipitates were washed with water and reprecipitated from DMF-ether to give 98 mg of the title compound.
TLC: Rf 0.86 (chloroform : methanol = 9:1)

### [Process 3] Benzyloxycarbonyl-Val-(2R,3S)-AHPBA-Phe-NH-CH₂-CH(CH₃)₂

Deprotection of 30 mg of the compound obtained by the process 2 was performed similarly to that in Comparative Example X (Process 3), and the obtained product was dissolved in 3 ml of DMF and neutralized with 8 µl of triethylamine under ice cooling. To the neutralized solution, 26 mg of benzyloxycarbonyl-Val-OH.DCHA, 27 mg of Bop reagent and 8 µl of triethylamine were added and the resultant mixture was stirred for 2 hr. The reaction mixture was evaporated under reduced pressure and water was added to the resultant residue to give precipitates. The precipitates were washed with water and reprecipitated from THF-ether to give 10 mg of the title compound.
TLC: Rf 0.36 (chloroform : methanol = 20:1)
FAB-:MS: 631 (M+1)

### Example 55: Benzyloxycarbonyl-Asn-(2S,3S)-AHPBA-Pro-NH-tBu

### [Process 1] Boc-Pro-NH-tBu

In a DMF solution of 0.50 g of Boc-Pro-OH, 0.24 ml of tert-butylamine, 0.36 g of HOBt and 0.53 g of EDC hydrochloride were added under ice cooling and the mixture was stirred for 14 hr. The reaction mixture was treated similarly to that in Comparative Example X (Process 2) to give 373 mg of the title compound.
TLC: Rf 0.41 (chloroform : methanol = 20:1)

### [Process 2] Boc-(2S,3S)-AHPBA-Pro-NH-tBu

Deprotection of 100 mg of the compound obtained by the process 1 was performed similarly to that in Comparative Example X (Process 3), and the obtained product was dissolved in 5 ml of DMF and neutralized with 52 µl of triethylamine under ice cooling. To the neutralized solution, 100 mg of Boc-(2S,3S)-AHPBA-OH, 164 mg of Bop reagent, 104 µl of triethylamine were added and the resultant mixture was stirred for 2 hr. The reaction mixture was treated similarly to that in Example 46 and crystallized from hexane to give 69 mg of the title compound.
TLC : Rf 0.38 (chloroform : methanol = 9:1)

### [Process 3] Benzyloxycarbonyl-Asn-(2S,3S)-AHPBA-Pro-NH-tBu

Deprotection of 30 mg of the compound obtained by the process 2 was performed similarly to that in Comparative Example X (Process 3), and the obtained product was dissolved in 3 ml of DMF and neutralized with 9.3 µl of triethylamine under ice cooling. To the neutralized solution, 52 mg of benzyloxycarbonyl-Asn-ONp, 21 mg of HOBt and 15 µl of N-methylmorpholine were added and the resultant solution was stirred for 14 hr. The reaction mixture was treated similarly to that in Example 49, except for the crystallization solvent (ether-hexane), to give 22 mg of the title compound.
TLC : Rf 0.29 (chloroform : methanol = 9:1)
FAB-MS: 596 (M+1)

### Example 56: Benzyloxycarbonyl-Asn-(2R,3S)-AHPBA-Pro-NH-tBu

The title compound was synthesizerd by a similar method with that in Example 55.
Analytical HPLC: 18.36 min (For the condition, see: Example 35).
FAB-MS: 596 (M+1)

### Comparative Example KK : 3-Phenylpropionyl-Asn-(2S,3S)-ACHBA-Pro-Ile-Val-NH₂

### [Process 1] (2S,3S)-3-N-t-butoxycarbonylamino-4-cyclohexyl-2-hydroxybutanoic acid

In 2.5 ml of ethanol, 148 mg of Boc-(2S,3S)-AHPBA-OH was dissolved and 15 mg of 5% Rh/Al₂O₃ was added to the solution. The resultant mixture was stirred for 5 days at room temperature in the hydrogen atmosphere at 4.4 x 10⁵Pa (4.5 kg/cm²), then filtered to separate the catalyst using celite and the filtrate was evaporated under reduced pressure to give the title compound (hereinafter abbreviated as Boc-(2S,3S)-ACHBA-OH).

### [Process 2] 3-Phenylpropionyl-Asn-(2S,3S)-ACHBA-Pro-Ile-Val-NH₂

The title compound was obtained by a solid phase method similar to Example 16 (Process 3) from Boc-(2S,3S)-ACHBA-OH prepared above .
Analytical HPLC: 19.46 min (For the condition, see: Example 35)
FAB-MS: 756 (M+1)

### Comparative Example LL: 3-Phenylpropionyl-Asn-(2R,3S)-ACHBA-Pro-Ile-Val-NH₂

### [Process 1] Boc-(2R,3S)-ACHBA-OH

In 2.5 ml of ethanol, 148 mg of Boc-(2R,3S)-AHPBA-OH was dissolved and 15 mg of 5% Rh/Al₂O₃ was added to the solution. The resultant mixture was stirred for 5 days at room temperature in the hydrogen atmosphere at 4.4 x 10⁵Pa (4.5 kg/cm²), then filtered using celite to separate the catalyst and the filtrate was evaporated under reduced pressure to give the title compound.

### [Process 2] 3-Phenylpropionyl-Asn-(2R,3S)-ACHBA-Pro-Ile-Val-NH₂

The title compound was obtained by a solid phase method similar to Example 16 (Process 3) from Boc-(2R,3S)-ACHBA-OH prepared above.
Analytical HPLC: 21.41 min (For the condition, see: Example 35)
FAB-MS: 756 (M+1)

### Comparative Example MM: 3-Phenylpropionyl-His-(2S,3S)-AHPBA-Pro-Ile-Val-NH₂

The title compound was obtained by a solid phase method similar to Example 16 (Process 3).
Analytical HPLC: 15.22 min (For the condition, see: Example 35)
FAB-MS: 773 (M+1)

### Example 60: 3-Phenylpropionyl-Ser(Me)-(2S,3S)-AHPBA-Pro-Ile-Val-NH₂

### [Process 1] Boc-Ser(Me)-OH.DCHA

In a DMF solution containing 2.00 g of Boc-Ser-OH, 0.86 g of sodium hydride (60% oily suspension) was added under ice cooling and the resultant mixture was stirred for 30 min. To the resultant solution, 0.72 ml of methyl iodide was added and the resultant solution was stirred for 3 hr. The reaction mixture was neutralized with citric acid and evaporated under reduced pressure. The obtained residue was redissolved in ethyl acetate, and the solution was washed with 5% aqueous citric acid solution and saturated aqueous sodium chloride solution, successively, and dried over anhydrous sodium sulfate. The dried solution was evaporated under reduced pressure. The residue was subjected to a silica gel column chromatography (chloroform : methanol = 10:1) and crystallized as its DCHA salt from n-hexane to give 0.79 g of the title compound.
TLC: Rf 0.45(chloroform:,methanol:acetic acid = 9:1:0.5)

### [Process 2] 3-Phenylpropionyl-Ser(Me)-(2S,3S)-AHPBA-Pro-Ile-Val-NH₂

The title compound was obtained by a solid phase method similar to Example 16 (Process 3).
Analytical HPLC: 20.54 min (For the condition, see: Example 35)
FAB-MS: 737 (M+1)

### Comparative Example NN: 3-Phenylpropionyl-Smc(O)-(2S,3S)-AHPBA-Pro-Ile-Val-NH₂

### [Process 1] N-(tert-Butoxycarbonyl)methanesulfinylalanine

In 10 ml of purified water, 1.0 g of S-methyl-L-cysteine was suspended and 1.54 ml of triethylamine was added under ice cooling. To this was added a solution of 1.94 g of Boc₂O in 10 ml of THF and the resultant reaction mixture was stirred for 14 hr. The reaction mixture was washed with ether and the aqueous layer was evaporated up to the half volume. The condensed solution was adjusted to pH 2-3 with citric acid and extracted with ethyl acetate, washed with saturated aqueous sodium chloride solution. To the organic solution, was added an aqueous solution of 1.36 g of sodium perborate tetrahydrate, and the reaction mixture was stirred overnight. The organic layer was washed with saturated sodium chloride aqueous solution and dried over anhydrous sodium sulfate. The dried solution was evaporated under reduced pressure and ether was added to the residue to crystallize 1.33 g of the title compound [Boc-Smc(O)-OH].
TLC: Rf 0.51 (n-BuOH:acetic acid:pyridine:H₂O 4:1:1:2)

### [Process 2] 3-Phenylpropionyl-Smc(O)-(2S,3S)-AHPBA-Pro-Ile-Val-NH₂

The title compound was obtained by a solid phase method similar to Example 16 (Process 3).
Analytical HPLC: 17.86 and 18.61 min (For the condition, see: Example 35)
FAB-MS: 769 (M+1)

### Example 62: 3-Phenylpropionyl-Msa-(2S,3S)-AHPBA-Pro-Ile-Val-NH₂

### [Process 1] N-(tert-Butoxycarbonyl)methanesulfonylalanine

In 2 ml of chloroform, 300 mg of Boc-Smc(O)-OH was dissolved, 206 mg of m-chloroperbenzoic acid was added and the resultant mixture was stirred for 14 hr. The reaction mixture was filtered, and the filtrate Was evaporated and crystallized by the addition of a mixture of ether and n-hexane to give 267 mg of the title compound.
TLC: Rf 0.60 (n-BuOH:acetic acid:pyridine:H₂O = 4:1:1:2)

### [Process 2] 3-Phenylpropionyl-Msa-(2S,3S)-AHPBA-Pro-Ile-Val-NH₂

The title compound was obtained by a solid phase method similar to Example 16 (Process 3).
Analytical HPLC: 19.21 min (For the condition, see: Example 35)
FAB-MS: 785 (M+1)

### Example 63: Fmoc-Asn-(2S,3S)-AHPBA-Pro-Ile-Val-NH₂

The title compound was obtained by a solid phase method similar to Example 16 (Process 3).
Analytical HPLC: 22.57 min (For the condition, see: Example 35)
FAB-MS: 840 (M+1)

### Example 64: 1-Naphthoxyacetyl-Asn-(2S,3S)-AHPBA-Pro-Ile-Val-NH₂

The title compound was obtained by a solid phase method similar to Example 16 (Process 3).
Analytical HPLC: 20.06 min (For the condition, see: Example 35)
FAB-MS: 802 (M+1)

### Example 65: Furancarbonyl-Asn-(2S,3S)-AHPBA-Pro-Ile-Val-NH₂

The title compound was obtained by a solid phase method similar to Example 16 (Process 3).
Analytical HPLC: 12.92 min (For the condition, see: Example 35)
FAB-MS: 712 (M+1)

### Example 66: Pyrazinecarbonyl-Asn-(2S,3S)-AHPBA-Pro-Ile-Val-NH₂

The title compound was obtained by a solid phase method similar to Example 16 (Process 3).
Analytical HPLC: 11.54 min (For the condition, see: Example 35)
FAB-MS: 724 (M+1)

### Example 67: Thiophenecarbonyl-Asn-(2S,3S)-AHPBA-Pro-Ile-Val-NH₂

The title compound was obtained by a solid phase method similar to Example 16 (Process 3).
Analytical HPLC: 14.18 min (For the condition, see: Example 35)
FAB-MS: 728 (M+1)

### Example 68: L-Indoline-2-carbonyl-Asn-(2S,3S)-AHPBA-Pro-Ile-Val-NH₂

The title compound was obtained by a solid phase method similar to Example 16 (Process 3).
Analytical HPLC: 14.41 min (For the condition, see: Example 35)
FAB-MS: 763 (M+1)

### Example 69: H-(D)-Tic-Asn-(2S,3S)-AHPBA-Pro-Ile-Val-NH₂

### Example 70: H-(L)-Tic-Asn-(2S,3S)-AHPBA-Pro-Ile-Val-NH₂

D- and L-form of 1,2,3,4-Tetrahydroisoquinoline-3-carbonyl-Asn-(2S,3S)-AHPBA-Pro-Ile-Val-NH₂ were obtained by a solid phase method similar to Example 16 (Process 3).
Analytical HPLC (D): 11.17 min (For the condition, see: Example 35)
Analytical HPLC (L): 12.52 min (For the condition, see: Example 35)
FAB-MS: 777 (M+1)

### Comparative Example OO: 3-Phenylpropionyl-Asn-(2S,3S)-AHPBA(OMe)-Pro-Ile-Val-NH₂

The title compound was obtained by a solid phase method similar to Example 16 (Process 3), AHPBA(OMe) means 3-amino-2-hydroxy-4-(p-methoxyphenyl)butanoic acid resudure.
Analytical HPLC: 18.01 min (For the condition, see: Example 35)
FAB-MS: 780 (M+1)

### Comparative Example PP: 3-Phenylpropionyl-Met(O)₂-(2S,3S)-AHPBA-Pro-Ile-Val-NH₂

The title compound was obtained by a solid phase method similar to Example 16 (Process 3).
Analytical HPLC: 18.68 min (For the condition, see: Example 35)
FAB-MS: 799 (M+1)

### Example 73: 3-Phenylpropionyl-Ser-(2S,3S)-AHPBA-Pro-Ile-Val-NH₂

The title compound was obtained by a solid phase method similar to Example 16 (Process 3).
Analytical HPLC: 18.17 min (For the condition, see: Example 35)
FAB-MS: 723 (M+1)

### Example 74: 3-Phenylpropionyl-Leu-(2S,3S)-AHPBA-Pro-Ile-Val-NH₂

The title compound was obtained by a solid phase method similar to Example 16 (Process 3).
Analytical HPLC: 25.03 min (For the condition, see: Example 35)
FAB-MS: 749 (M+1)

### Example 75: 3-Phenylpropionyl-Asn-(2S,3S)-AHPBA-Pro-Gln-Ile-NH₂

The title compound was obtained by a solid phase method similar to Example 16 (Process 3).
Analytical HPLC: 21.78 min (For the condition, see: Example 35)
FAB-MS: 779 (M+1)

### Example 76: 3-Phenylpropionyl-Asn-(2S,3S)-AHPBA-Pro-Val-NH₂

The title compound was obtained by a solid phase method similar to Example 16 (Process 3).
Analytical HPLC: 14.78 min (For the condition, see: Example 35)
FAB-MS: 637 (M+1)

### Example 77: 3-Phenylpropionyl-Asn-(2S,3S)-AHPBA-Pro-Ile-NH₂

The title compound was obtained by a solid phase method similar to Example 16 (Process 3).
Analytical HPLC: 16.13 min (For the condition, see: Example 35)
FAB-MS: 651 (M+1)

### Example 78: Benzyloxycarbonyl-Asn-(2S,3S)-AHPBA-(L)-Pip-NH-tBu

### Example 79: Benzyloxycarbonyl-Asn-(2S,3S)-AHPBA-(D)-Pip-NH-tBu

### [Process 1] Boc-(DL)-Pip-NH-tBu

In a DMF solution containing 0.20 g of N-(tert-butoxy-carbonyl)-(DL)-pipecolic acid, 92 µl of tert-butylamine, 134 mg of HOBt and 200 mg of EDC hydrochloride were added under ice cooling and the resultant mixture was stirred for 14 hr. The reaction mixture was treated similarly to that in

### Comparative Example Z (Process 3) to give 108 mg of the title compound TLC: Rf 0.39 (chloroform : methanol = 20:1)

### [Process 2] Boc-(2S,3S)-AHPBA-(DL)-Pip-NH-tBu

Deprotection of 100 mg of the compound obtained by the process 1 was performed similarly to that in Comparative Example X (Process 3), and the obtained product was dissolved in 5 ml of DMF and neutralized with 49 µl of triethylamine under ice cooling. To the neutralized solution, 104 mg of Boc-(2S,3S)-AHPBA-OH, 156 mg of Bop reagent, 98 µl of triethylamine were added and the resultant mixture was stirred for 2 hr. The reaction mixture was treated similarly to that in Example 46 to give 109 mg of the title compound.
TLC : Rf 0.65 (chloroform : methanol = 9:1)

### [Process 3] Benzyloxycarbonyl-Asn-(2S,3S)-AHPBA-(L)-Pip-NH-tBu and Benzyloxycarbonyl-Asn-(2S,3S)-AHPBA-(D)-Pip-NH-tBu

Deprotection of 100 mg of the compound obtained by the process 2 was performed similarly to that in Comparative Example X (Process 3), and the obtained product was dissolved in 5 ml of DMF and neutralized with 30 µl of triethylamine under ice cooling. To the neutralized solution, 169 mg of benzyloxycarbonyl-Asn-ONp, 66 mg of HOBt and 48 µl of N-methylmorpholine were added and the resultant solution was stirred for 14 hr. The reaction mixture was treated similarly to that in Example 55 ( Process 3) to give 58 mg of benzyloxycarbonyl-Asn-(2S,3S)-AHPBA-(DL)-Pip-NH-tBu.
TLC: Rf 0.55 (chloroform : methanol = 9:1)
The obtained mixture was dissolved in methanol, fractionated by a reversed-phase HPLC and lyophilized to give the title compounds.
FAB-MS: 610 (M+1)

### Example 80: Boc-Asn-(2S,3S)-AHPBA-Pro-NH-tBu

Deprotection of 85 mg of the compound obtained by Example 55 (Process 2) was performed similarly to that in Comparative Example X (Process 3), and the obtained product was dissolved in 3 ml of DMF and neutralized with 27 µl of triethylamine under ice cooling. To the neutralized solution, 134 mg of Boc-Asn-ONp, 58 mg of HOBt, 42 µl of N-methylmorpholine were added and the resultant solution was stirred for 14 hr. The reaction mixture was treated similarly to that in Example 55 (Process 3) to give 51 mg of the title compound.
TLC: Rf 0.33 (chloroform : methanol = 9:1)

### Example 81: 1-Naphthylmethyloxycarbonyl-Asn-(2S,3S)-AHPBA-Pro-NH-tBu

### [Process 1] 1-Naphthylmethyl 4-nitrophenyl carbonate

In 5 ml of pyridine containing 1.0 g of 1-naphthyl-methanol, 1.27 g of 4-nitrophenyl chloroformate was added under ice cooling and the resultant mixture was stirred for 3 hr. Purified water and ethyl acetate, each 20 ml, were added to the reaction mixture and the ethyl acetate layer was separated and washed with purified water. The ethyl acetate layer was dried over anhydrous sodium sulfate, evaporated under reduced pressure and crystallized by the addition of ethanol to give 1.08 g of the title compound.
TLC: Rf 0.81 (chloroform)

### [Process 2] 1-Naphthylmethyloxycarbonyl-Asn-(2S,3S)-AHPBA-Pro-NH-t Bu

Deprotection of 30 mg of the compound obtained by Example 55 (Process 2) was performed similarly tb that in Comparative Example X (Process 3), and the obtained product was dissolved in 3 ml of DMF and neutralized with 8 µl of triethylamine under ice cooling. To the neutralized solution, 35 mg of 1-naphthylmethyl 4-nitrophenyl carbonate 12 µl of N-methylmorpholine were added and the resultant solution was stirred for 14 hr. The reaction mixture was treated similarly to that in Example 49 to give 5 mg of the title compound.
TLC: Rf 0.40 (chloroform : methanol = 9:1)
FAB-MS: 646 (M+1)

### Example 82: Benzyloxycarbonyl-Asn-(2S,3S)-AHPBA-Thz-NH-tBu

### [Process 1] Boc-Thz-NH-tBu

In a DMF solution containing 0.10 g of N-(t-butoxy-carbonyl)-1,3-thiazolidine-4-carboxylic acid, 45 µl of tert-butylamine, 66 mg of HOBt and 98 mg of EDC hydrochloride were added under ice cooling and the resultant mixture was stirred for 14 hr. The reaction mixture was treated similarly to that in Comparative Example BB (Process 3) to give 90 mg of the title compound.
TLC: Rf 0.43 (chloroform : methanol = 20:1)

### [Process 2] Boc-(2S,3S)-AHPBA-Thz-NH-tBu

Deprotection of 50 mg of the compound obtained by the process 1 in the presence of 25 µl of anisole and 14 µl of 1,2-ethanedithiol was performed similarly to that in Example 28 (Process 3), and the obtained product was dissolved in 5 ml of DMF and neutralized with 24 µl of triethylamine under ice cooling. To the neutralized solution, 51 mg of Boc-(2S,3S)-AHPBA-OH, 77 mg of Bop reagent, 48 µl of triethylamine were added and the resultant mixture was stirred for 2 hr. The reaction mixture was treated similarly to that in Comparative Example X (Process 3) and crystallized from hexane to give 51 mg of the title compound.
TLC : Rf 0.64 (chloroform : methanol = 9:1)

### [Process 3] Benzyloxycarbonyl-Asn-(2S,3S)-AHPBA-Thz-NH-tBu

Deprotection of 51 mg of the compound obtained by the process 2 in the presence of 25 µl of anisole and 10 µl of ethanedithiol was performed similarly to that in Comparative Example X (Process 3), and the obtained product was dissolved in 5 ml of DMF and neutralized with 15 µl of triethylamine under ice cooling. To the neutralized solution, 85 mg of benzyloxycarbonyl-Asn-ONp, 34 mg of HOBt and 24 µl of N-methylmorpholine were added and the resultant solution was stirred for 14 hr. The reaction mixture was treated similarly to that in Example 49 to give 20 mg of benzyloxycarbonyl-Asn-(2S,3S)-AHPBA-Thz-NH-tBu. In methanol, seven mg of the obtained crystals were dissolved, fractionated by a reversed-phase HPLC and lyophilized to give four mg of the title compound.
Analytical HPLC: 21.37 min (For the condition, see: Example 35).
FAB-MS: 614 (M+1)
¹H NMR (CDCl₃, 500 MHz): Fig. 1

### Example 83: Benzyloxycarbonyl-Asn-(2S,3S)-AHPBA-Pro-NH-CH₂-C(CH₃)₃

### [Process 1] Boc-Pro-NH-CH₂-C(CH₃)₃

In a DMF solution containing 1.00 g of Boc-Pro-OH, 0.58 ml of neopentylamine, 0.71 g of HOBt and 1.06 g of EDC hydrochloride were added and the resultant mixture was stirred for 14 hr. The reaction mixture was treated similarly to that in Comparative Example Z (Process 3) to give 605 mg of the title compound.
TLC: Rf 0.56 (chloroform : methanol = 20:1)

### [Process 2] Benzyloxycarbonyl-Asn-(2S,3S)-AHPBA-Pro-NH-CH₂-C(CH₃)₃

The title compound was obtained according to the method of Example 55 from the protected amino acid obtained in the above process 1.
Analytical HPLC: 21.24 min (For the condition, see: Example 35)
FAB-MS: 610 (M+1)

### Example 84: Benzyloxycarbonyl-Asn-(2S,3S)-AHPBA-Pro-NH-C₆H₁₁

### [Process 1] Boc-Pro-NH-C₆H₁₁

In a DMF solution containing 1.00 g of Boc-Pro-OH, 0.53 ml of cyclohexylamine, 0.63 g of HOBt and 1.07 g of EDC hydrochloride were added under ice cooling and the resultant mixture was stirred for 14 hr. The reaction mixture was treated similarly to that in Comparative Example Z (Process 3) to give 853 mg of the title compound.
TLC: Rf 0.77 (chloroform : methanol = 20:1)

### [Process 2] Benzyloxycarbonyl-Asn-(2S,3S)-AHPBA-Pro-NH-C₆H₁₁

The title compound was obtained according to the method of Example 55 from the protected amino acid obtained in the above process 1.
TLC: Rf 0.44 (chloroform : methanol = 9:1)
FAB-MS: 622 (M+1)

### Example 85: Benzyloxycarbonyl-Asn-(2S,3S)-AHPBA-Pro-NHCH(CH₃)₂

### [Process 1] Boc-Pro-NH-CH(CH₃)₂

In a DMF solution containing 1.00 g of Boc-Pro-OH, 0.40 ml of isopropylamine, 0.71 g of HOBt and 1.06 g of EDC hydrochloride were added under ice cooling and the resultant mixture was stirred for 14 hr. The reaction mixture was treated similarly to that in Comparative Example X (Process 2) to give 654 mg of the title compound.
TLC: Rf 0.41 (chloroform : methanol = 20:1)

### [Process 2] Benzyloxycarbonyl-Asn-(2S,3S)-AHPBA-Pro-NH-CH(CH₃)₂

The title compound was obtained according to the method of Example 55 from the protected amino acid obtained in the above process 1.
Analytical HPLC: 17.57 min (For the condition, see: Example 35)
FAB-MS: 582 (M+1)

### Example 86: Benzyloxycarbonyl-Asn-(2S,3S)-AHPBA-Pro-O-tBu

### [Process 1] Benzyloxycarbonyl-Asn-(2S,3S)-AHPBA-O-benzyl

Deprotection of 190 mg of Boc-(2S,3S)-AHPBA-O-benzyl obtained by Example 16 (Process 1) was performed similarly to that in Comparative Example X (Process 3), and the obtained product was dissolved in 3 ml of DMF and neutralized with 69 µl of triethylamine under ice cooling. To the neutralized solution, 286 mg of benzyloxycarbonyl-Asn-ONp, 113 mg of HOBt and 81 µl of N-methylmorpholine were added and the resultant mixture was stirred for 14 hr. The reaction mixture was evaporated under reduced pressure and the resultant residue was mixed with purified water and the formed precipitates were collected and washed thoroughly with purified water. The precipitates were recovered and reprecipitated from DMF-ether to give 240 mg of the title compound.
TLC: Rf 0.60 (chloroform : methanol:water = 8:3:1, lower layer)

### [Process 2] Benzyloxycarbonyl-Asn-(2S,3S)-AHPBA-OH

In 3 ml of DMF, 230 mg of the peptide obtained by the process 1 was dissolved and stirred with 0.52 ml of 1N-NaOH under ice cooling for 2 hr. The reaction mixture was neutralized with citric acid and evaporated under reduced pressure. To the resultant residue, 5% citric acid aqueous solution was added to cause precipitation and the precipitates were reprecipitated from DMF and ether to give 140 mg of the title compound.
TLC: Rf 0.59 (n-BuOH:acetic acid:pyridine:water = 4:1:1:2)

### [Process 3] Benzyloxycarbonyl-Asn-(2S,3S)-AHPBA-Pro-O-tBu

In 2 ml of DMF, 19 mg of H-Pro-O-tBu hydrochloride was dissolved and neutralized with 13 µl of triethylamine under ice cooling. To the neutralized solution, 20 mg of benzyloxycarbonyl-Asn-(25,35)-AHPBA-OH, 20 mg of Bop reagent and 26 µl of triethylamine were added and the resultant mixture was stirred for 2 hr. The reaction mixture was treated similarly to that in Example 49, except for the crystallization solvent (hexane), to give 22 mg of the title compound.
TLC: Rf 0.48 (chloroform : methanol = 9:1)
FAB-MS: 597 (M+1)
¹H NMR (CDCl₃, 500 MHz): Fig. 2

### Example 87: Benzyloxycarbonyl-Asn-(2S,3S)-AHPBA-Pro-NH-tAmyl

### [Process 1] Boc-Pro-NH-tAmyl

In a DMF solution containing 0.50 g of Boc-Pro-OH, 0.27 ml of tert-amylamine, 0.36 g of HOBt and 0.53 g of EDC hydrochloride were added under ice cooling and the resultant mixture was stirred for 14 hr. The reaction mixture treated similarly to that in Comparative Example X (Process 2) to give 448 mg of the title compound.
TLC: Rf 0.56 (chloroform : methanol = 20:1)

### [Process 2] Benzyloxycarbonyl-Asn-(2S,3S)-AHPBA-Pro-NH-tAmyl

Deprotection of 20 mg of the compound obtained by the process 1 was performed similarly to that in Comparative Example X (Process 3), and the obtained product was dissolved in 3 ml of DMF and neutralized with 10 µl of triethylamine under ice cooling. To the neutralized solution, 31 mg of benzyloxycarbonyl-Asn-(2S,3S)-AHPBA-OH, 31 mg of Bop reagent, 20 µl of triethylamine were added and the resultant mixture was stirred for 2 hr. The reaction mixture was treated similarly to that in Example 55 (Process 3) to give 33 mg of the title compound.
Analytical HPLC: 21.98 min (For the condition, see: Example 35.)
FAB-MS: 610 (M+1)

### Example 88: Benzyloxycarbonyl-Asn-(2S,3S)-AHPBA-Pro-NH-cyclopropyl

### [Process 1] Boc-Pro-NH-cyclopropyl

In a DMF solution containing 0.50 g of Boc-Pro-OH, 0.16 ml of cyclopropylamine, 0.36 g of HOBt and 0.53 g of EDC hydrochloride were added under ice cooling and the resultant mixture was stirred for 14 hr. The reaction mixture was treated similarly to that in Comparative Example Z (Process 3) to give 245 mg of the title compound.
TLC: Rf 0.47 (chloroform : methanol = 20:1)

### [Process 2] Benzyloxycarbonyl-Asn-(2S,3S)-AHPBA-Pro-NH-cyclopropyl

The title compound was obtained according to the method of Example 87 from the protected amino acid obtained in the above process 1.
TLC: Rf 0.60 (chloroform : methanol:water = 8:3:1, lower layer)
FAB-MS: 580 (M+1)

### Example 89: Benzyloxycarbonyl-Asn-(2S,3S)-AHPBA-Pro-NH-CH(C₂H₅)₂

### [Process 1] Boc-Pro-NH-CH(C₂H₅)₂

In a DMF solution containing 0.50 g of Boc-Pro-OH, 0.27 ml of 1-ethylpropylamine, 0.36 g of HOBt and 0.53 g of EDC hydrochloride were added under ice cooling and the resultant mixture was stirred for 14 hr. The reaction mixture was treated similarly to that in Comparative Example Z (Process 3) to give 324 mg of the title compound.
TLC: Rf 0.57 (chloroform : methanol = 20:1)

### [Process 2] Benzyloxycarbonyl-Asn-(2S,3S)-AHPBA-Pro-NH-CH(C₂H₅)₂

The title compound was obtained according to the method of Example 87 from the protected amino acid obtained in the above process 1.
TLC: Rf 0.48 (chloroform : methanol = 9:1)
FAB-MS: 610 (M+1)

### Example 90: 1-Naphthylmethyloxycarbonyl-Msa-(2S,3S)-AHPBA-Pro-NH-tBu

### [Process 1] Boc-Msa-(2S,3S)-AHPBA-Pro-NH-tBu

Deprotection of 30 mg of the compound obtained by Example 55 (Process 2) was performed similarly to that in Comparative Example X (Process 3), and the obtained product was dissolved in 3 ml of DMF and neutralized with 10 µl of triethylamine under ice cooling. To the neutralized solution, 18 mg of Boc-Msa-OH, 30 mg of Bop reagent and 19 µl of triethylamine were added and the resultant mixture was stirred for 2 hr. The reaction mixture was treated similarly to that in Example 86 (Process 3) to give 27 mg of the title compound.
TLC: Rf 0.52 (chloroform : methanol = 9:1)

### [Process 2] 1-Naphthylmethyloxycarbonyl-Msa-(2S,3S)-AHPBA-Pro-NH-tBu

The title compound was obtained according to the method of Example 81 from the protected peptide obtained the above process 1.
TLC: Rf 0.50 (chloroform : methanol = 9:1)
FAB-MS: 681 (M+1)

### Example 91: 1-Naphthyloxyacetyl-Asn-(2S,3S)-AHPBA-Pro-NH-tBu

Deprotection of 22 mg of the compound obtained by Example 80 was performed similarly to that in Comparative Example X (Process 3), and the obtained product was dissolved in 3 ml of DMF and neutralized with 5.4 µl of triethylamine under ice cooling. To the neutralized solution, 8 mg of 1-naphthoxyacetic acid, 17 mg of Bop reagent and 11 µl of triethylamine were added and the resultant mixture was stirred for 2 hr. The reaction mixture was treated similarly to that in Comparative Example FF (Process 2) to give 23 mg of the crude title compound. In methanol, 8 mg of the obtained crystals were dissolved, fractionated by a reversed-phase HPLC and lyophilized to give 4 mg of the title compound.
Analytical HPLC: 23.14 min (For the condition, see: Example 35.)
FAB-MS: 646 (M+1)

### Example 92: Fmoc-Asn-(2S,3S)-AHPBA-Pro-NH-tBu

Deprotection of 30 mg of the compound obtained by Example 55 (Process 2) was performed similarly to that in Comparative Example X (Process 3), and the obtained product was dissolved in 3 ml of DMF and neutralized with 9.3 µl of triethylamine under ice cooling. To the neutralized solution, 70 mg of Fmoc-Asn-O-pentafluorophenyl, 21 mg of HOBt and 15 µl of N-methylmorpholine were added and the resultant mixture was stirred for 14 hr. The reaction mixture was treated similarly to that in Comparative Example FF (Process 2) to give 85 mg of the crude product. In methanol, 12 mg of the crude product was dissolved, fractionated by a reversed-phase HPLC and lyophilizated to give 4.8 mg of the title compound.
TLC: Rf 0.36 (chloroform : methanol = 9:1)
FAB-MS: 684 (M+1)

### Example 93: Benzyloxycarbonyl-Asn-(2S,3S)-AHPBA-Pro-Aib-NH₂

### [Process 1] Boc-Aib-NH₂

In a DMF solution containing 1.00 g of 2-N-(tert-butoxy-carbonyl)aminoisobutyric acid, 0.75 ml of triethylamine and 0.70 ml of isobutyl chloroformate were added at -10 to -20 °C and the resultant mixture was stirred for 10 min. To the solution, 1.03 ml of concentrated ammonia water (28%) was added and the resultant mixture was stirred for 2 hr. The reaction mixture was evaporated under reduced pressure and water was added to the residue. The formed precipitates were thoroughly washed with purified water and re-precipitated from THF - ether to give 200 mg of the title compound.
TLC: Rf 0.63 (chloroform : methanol:water = 8:3:1, lower layer)

### [Process 2] Boc-Pro-Aib-NH₂

Deprotection of 100 mg of the compound obtained by the process 1 was performed similarly to that in Comparative Example X (Process 3), and the obtained product was dissolved in 3 ml of DMF and neutralized with 68 µl of triethylamine under ice cooling. To the neutralized solution, 107 mg of Boc-Pro-OH, 237 mg of Bop reagent and 137 µl of triethylamine were added and the resultant mixture was stirred for 2 hr. The reaction mixture was treated similarly to that in Example 86 (Process3) to give 35 mg of the title compound.
TLC: Rf 0.48 (chloroform : methanol = 9:1)

### [Process 3] Boc-(2S,3S)-AHPBA-Pro-Aib-NH₂

Deprotection of 35 mg of the compound obtained by the process 2 was performed similarly to that in Comparative Example X (Process 3), and the obtained product was dissolved in 5 ml of DMF and neutralized with 17 µl of triethylamine under ice cooling. To the neutralized solution, 35 mg of Boc-(2S,3S)-AHPBA-OH, 53 mg of Bop reagent, and 33 µl of triethylamine were added and the resultant mixture was stirred for 2 hr. The reaction mixture was treated similarly to that in Example 46, except for the chromatography solvent (chloroform : methanol = 10:1), to give 36 mg of the title compound.
TLC: Rf 0.28 (chloroform : methanol = 9:1)

### [Process 4] Benzyloxycarbonyl-Asn-(2S,3S)-AHPBA-Pro-Aib-NH₂

Deprotection of 35 mg of the compound obtained by the process 3 was performed similarly to that in Comparative Example X (Process 3), and the obtained product was dissolved in 3 ml of DMF and neutralized with 10 µl of triethylamine under ice cooling. To the neutralized solution, 57 mg of benzyloxycarbonyl-Asn-ONp , 23 mg of HOBt and 16 µl of N-methylmorpholine were added and the resultant mixture was stirred for 14 hr. The reaction mixture was treated similarly to that in Exampl 55 (Process3) to give the crude product. The crude product dissolved in methanol, fractionted by a reversed-phase HPLC and lyophilized to give 5.3 mg of the title compound.
TLC: Rf 0.63 (chloroform : methanol:water = 8:3:1, lower layer)
FAB-MS: 625 (M+1)

### Example 94: Bis(4-chlorophenyl)methyloxyacetyl-Asn-(2S,3S)-AHPBA-Pro-NH-tBu

Deprotection of 25 mg of the compound obtained by Example 80 (Process 2) was performed similarly to that in Comparative Example X (Process 3), and the obtained product was dissolved in 3 ml of DMF and neutralized with 6.0 µl of triethylamine under ice cooling. To the neutralized solution, 26 mg of bis(4-chlorophenyl)methyloxyacetic acid, 24 mg of Bop reagent, and 6 µl of N-methylmorpholine were added and the resultant mixture was stirred for 2 hr. The reaction mixture was treated similarly to that in Comp Example FF (Process2) and the formed precipitates were dissolved in methanol. The methanol solution was fractionated by a reversed-phase HPLC and lyophilized to give the title compound.
Analytical HPLC: 29.97 min (For the condition, see: Example 35).
FAB-MS: 755 (M+1)

### Example 95: Benzyloxycarbonyl-Asn-(2S,3S)-AHPBA-Hyp(Bzl)-NH-tBu

### [Process 1] Boc-Hyp(Bzl)-NH-tBu

In a DMF solution containing 100 mg of Boc-Hyp(Bzl)-OH, 33 µl of t-butylamine, 48 mg of HOBt and 71 mg of EDC hydrochloride were added and the resultant mixture was stirred for 14 hr. The reaction mixture was treated similarly to that in Comparative Example BB (Process3) to give 88 mg of the title compound.

### [Process 2] Boc-(2S,3S)-AHPBA-Hyp(Bzl)-NH-tBu

Deprotection of 76 mg of the compound obtained by the process 1 was performed similarly to that in Comp Example (Process 3), and the obtained product was dissolved in 5 ml of DMF and neutralized with 28 µl of triethylamine under ice cooling. To the neutralized solution, 60 mg of Boc-(2S,3S)-AHPBA-OH, 89 mg of Bop reagent and 56 µl of triethylamine were added and the resultant mixture was stirred for 2 hr. The reaction mixture was treated similarly to that in Comparative Example X (Process 3) to give 92 mg of the title compound.

### [Process 3] Benzyloxycarbonyl-Asn-(2S,3S)-AHPBA-Hyp(Bzl)-NH-tBu

Deprotection of 30 mg of the compound obtained by the process 2 was performed similarly to that in Comparative Example X (Process 3), and the obtained product was dissolved in 4 ml of DMF and neutralized with 6.4 µl of triethylamine under ice cooling. To the neutralized solution, 36 mg of benzyloxycarbonyl-Asn-ONp, 14 mg of HOBt and 10 µl of N-methylmorpholine were added and the resultant mixture was stirred for 14 hr. The reaction mixture was treated similarly to that in Example 49 to give 23 mg of the title compound.
TLC: Rf 0.41 (chloroform : methanol = 9:1)
Analytical HPLC: 26.65 min (For the condition, see: Example 35.)
FAB-MS: 702 (M+1)

### Example 96: Benzyloxycarbonyl-Asn-(2S,3S)-AHPBA-Inc-NH-tBu

### [Process 1] Boc-Inc-NH-tBu

In a DMF solution containing 500 mg of Boc-Inc-OH, 200 µl of t-butylamine, 291 mg of HOBt and 435 mg of EDC hydrochloride were added and the resultant mixture was stirred for 14 hr under ice cooling. The reaction mixture was treated similarly to that in Comparative Example BB (Process 3) to give the title compound.

### [Process 2] Benzyloxycarbonyl-Asn-(2S,3S)-AHPBA-Inc-NH-tBu

Deprotection of 20 mg of the compound obtained by the process 1 was performed similarly to that in comparative Example X (Process 3)in the presence of 10 µl of anisole and 26 µl of 1,2-ethanedithiol, and the obtained product was dissolved in 3 ml of DMF and neutralized with 7 µl of triethylamine under ice cooling. To the neutralized solution, 22 mg of Benzyloxycarbonyl-Asn-(2S,3S)-AHPBA-OH, 22 mg of Bop reagent and 11 µl of triethylamine were added and the resultant mixture was stirred for 2 hr. The reaction mixture was treated similarly to that in Comparative Example FF (Process 2) subjected to a reversed-phase HPLC to give 355 mg of the title compound.
Analytical HPLC: 22.88 min (For the condition, see: Example 35.)
FAB-MS: 644 (M+1)

### Comparative Example QQ Boc-Sma-(2S,3S)-AHPBA-Pro-NH-tBu

Deprotection of 30 mg of the compound obtained by Example 55 (Process 2) was performed similarly to that in Comparative Example X (Process 3), and the obtained product was dissolved in 3 ml of dichloromethane. To the dichloromethane solution, 30 mg of Boc-Sma-OH.DCHA and 435 mg of EDC hydrochloride were added under ice cooling and the mixture was stirred overnight. The reaction mixture was treated similarly to that in Comparative Example FF (Process 2) to give 7 mg of the title compound.
TLC: Rf 0.61 (chloroform : methanol:H₂O = 8:3:1)

### Comparative Example RR: 1-Napthoxyacetyl-Sma-(2S,3S)-AHPBA-Pro-NH-tBu

Deprotection of 7 mg of the compound obtained by Example 97 was performed similarly to that in Comparative Example X (Process 3), and the obtained product was dissolved in 2 ml of DMF and neutralized with 1.6 µl of triethylamine under ice cooling. To the neutralized solution, 2.4 mg of 1-naphthoxyacetic acid, 5.2 mg of Bop reagent and 3.2 µl of triethylamine were added and the resultant mixture was stirred for 2 hr. The reaction mixture was treated similarly to that in Example 51 (Process2), and the crude product was dissolved in methanol and subjected to a reversed-phase HPLC (water-acetonitrile system) and fractionated, purified and lyophilized to give 0.83 mg of the title compound.
Analytical HPLC: 25.08 min (For the condition, see: Example 35.)
FAB-MS: 682 (M+1)

### Example 99: Benzyloxycarbonyl-Asn-(2S,3S)-AHPBA-Pro-NH-C(CH₃)₂-CH₂-OH

### [Process 1] Boc-Pro-NH-C(CH₃)₂CH₂OH

In a DMF solution containing 0.20 g of Boc-Pro-OH, 0.09 ml of 2-amino-2-methyl-1-propanol, 0.14 g of HOBt and 0.21 g of EDC hydrochloride were added and the resultant mixture was stirred for 14 hrs. The reaction mixture was treated similarly to that in Example 46 to give 80 mg of the title compound.
TLC: Rf 0.32 (chloroform : methanol = 20:1)

### [Process 2] Boc-(2S,3S)-AHPBA-Pro-NH-C(CH₃)₂-CH₂OH

Deprotection of 50 mg of the compound obtained by the process 1 was performed similarly to that in Comparative Example X (Process 3), and the obtained product was dissolved in 5 ml of DMF and neutralized with 24 µl of triethylamine under ice cooling. To the neutralized solution, 52 mg of Boc-(2S,3S)-AHPBA-OH, 77 mg of Bop reagent and 48 µl of triethylamine were added and the resultant mixture was stirred for 2 hr. The reaction mixture was treated similarly to that in Example 55 (Process 2) to give 54 mg of the title compound.
TLC: Rf 0.53 (chloroform : methanol = 9:1)

### [Process 3] Benzyloxycarbonyl-Asn-(2S,3S)-AHPBA-Pro-NH-C(CH₃)₂-CH₂OH

Deprotection of 30 mg of the compound obtained by the process 2 was performed similarly to that in Comparative Example X (Process 3), and the obtained product was dissolved in 3 ml of DMF and neutralized with 9 µl of triethylamine under ice cooling. To the neutralized solution, 50 mg of benzyloxycarbonyl-Asn-ONp, 20 mg of HOBt and 14 µl of N-methylmorpholine were added and the resultant mixture was stirred for 14 hr. The reaction mixture was treated similarly to that in Example 49 to give 6.6 mg of the title compound.
Analytical HPLC: 16.37 min (For the condition, see: Example 35.)
FAB-MS: 612 (M+1)

### Example 100: 1-Naphthoxyacetyl-Msa-(2S,3S)-AHPBA-Thz-NH-tBu

### [Process 1] Boc-Msa-(2S,3S)-AHPBA-Thz-NH-tBu

Deprotection of 65 mg of the compound obtained by Example 82 (Process 2) was performed similarly to that in Comparative Example X (Process 3), and the obtained product was dissolved in 3 ml of DMF and neutralized with 20 µl of triethylamine under ice cooling. To the neutralized solution, 38 mg of Boc-methanesulfonylalanine, 62 mg of Bop reagent and 40 µl of triethylamine were added and the resultant mixture was stirred for 2 hr. The reaction mixture was treated similarly to that in Example 55 (Process2) to give 34 mg of the title compound.
TLC: Rf 0.53 (chloroform : methanol = 9:1) 2.1

### [Process 2] 1-Naphthoxyacetyl-Msa-(2S,3S)-AHPBA-Thz-NH-tBu

Deprotection of 34 mg of the compound obtained by the process 1 was performed similarly to that in Example (Process 3), and the obtained product was dissolved in 5 ml of DMF and neutralized with 8.0 µl of triethylamine under ice cooling. To the neutralized solution, 12 mg of 1-naphthoxyacetic acid, 25 mg of Bop reagent and 16 µl of triethylamine were added and the resultant mixture was stirred for 2 hr. The reaction mixture was treated similarly to that in Example 46 and ether was added to give 26 mg of the crude above mentioned compound. In methanol, 5 mg of the crude solid was dissolved, fractionated by a reversed-phase HPLC and lyophilized to give 1.4 mg of the title compound.
Analytical HPLC: 27.08 min (For the condition, see: Example 35.)
FAB-MS: 699 (M+1)
¹H NMR (DMSO-d₆, 500 MHz): Fig. 3

### Example 101: 1-Naphthoxyacetyl-Asn-(2S,3S)-AHPBA-Thz-NH-tBu

### [Process 1] Boc-Asn-(2S,3S)-AHPA-Thz-NH-tBu

Deprotection of 28 mg of the compound obtained by Example 82 (Process 2) was performed similarly to that in Example (Process 3), and the obtained product was dissolved in 3 ml of DMF and neutralized with 8 µl of triethylamine under ice cooling. To the neutralized solution, 40 mg of Boc-Asn-ONp, 8 mg of HOBt and 12 µl of N-methylmorpholine were added and the resultant mixture was stirred for 14 hr. The reaction mixture was treated similarly to that in Example 93 (Process 3) to give 28 mg of the title compound.

### [Process 2] 1-Naphthoxyacetyl-Asn-(2S,3S)-AHPBA-Thz-NH-tBu

Deprotection of 28 mg of the compound obtained by the process 1 was performed similarly to that in Comparative Example X (Process 3), and the obtained product was dissolved in 5 ml of DMF and neutralized with 6.5 µl of triethylamine under ice cooling. To the neutralized solution, 9.5 mg of 1-naphthoxyacetic acid, 21 mg of Bop reagent and 13 µl of triethylamine were added and the resultant mixture was stirred for 2 hr. The reaction mixture was treated similarly to that in Example 46 and ether was added to give the crude above mentioned compound. The crude solid was dissolved in methanol, subjected to a reversed-phase HPLC (water-acetonitrile system) and fractionated, purified and lyophilized to give 5.8 mg of the title compound.
Analytical HPLC: 24.38 min (For the condition, see: Example 35.)
FAB-MS: 664 (M+1)

### Example 102-103: Benzyloxycarbonyl-Asn-(2S,3S)-AHPBA-(DL)Tic-NH-tBu

### [Process 1] Boc-(DL)-Tic-NH-tBu

In a DMF solution containing 200 mg g of N-(tert-butoxycarbonyl)-(DL)-1,2,3,4-tetrahydroisoquinoline-3-carboxylic acid, 76 µl of tert-butylamine, 110 mg of HOBt and 165 mg of EDC hydrochloride were added and the resultant mixture was stirred for 14 hr. The reaction mixture was treated similarly to that in Comparative Example X (Process 2) to give 150 mg of the title compound.
TLC: Rf 0.80 (chloroform : methanol = 20:1)

### [Process 2] Boc-(2S,3S)-AHPBA-(DL)-Tic-NH-tBu

Deprotection of 60 mg of the compound obtained by the process 1 was performed similarly to that in Comparative Example X (Process 3), and the obtained product was dissolved in 5 ml of DMF and neutralized with 25 µl of triethylamine under ice cooling. To the neutralized solution, 53 mg of Boc-(2S,3S)-AHPBA-OH, 80 mg of Bop reagent and 50 µl of triethylamine were added and the resultant mixture was stirred for 2 hr. The reaction mixture was treated similarly to that in Comparative Example X (Process 3) to give 81 mg of the title compound.
TLC: Rf 0.59 (chloroform : methanol = 9:1)

### [Process 3] Benzyloxycarbonyl-Asn-(2S,3S)-AHPBA-(DL)Tic-NH-tBu

Deprotection of 81 mg of the compound obtained by the process 2 was performed similarly to that in Comparative Example X (Process 3), and the obtained product was dissolved in 8 ml of DMF and neutralized with 22 µl of triethylamine under ice cooling. To the neutralized solution, 124 mg of benzyloxycarbonyl-Asn-ONp, 24 mg of HOBt and 35 µl of N-methylmorpholine were added and the resultant mixture was stirred for 14 hr. The reaction mixture was treated similarly to that in Example 46 and crystallized by the addition of ether to give 44 mg of the crude title compound. In methanol, 10 mg of the crude solid was dissolved, fractionated by a reversed-phase HPLC and lyophilized to give the title compound.
Analytical HPLC: 24.44 and 25.16 min (For the condition, see: Example 35.)
FAB-MS: 658 (M+1)

### Example 104: Benzyloxycarbonyl-Asn-(2S,3S)-AHPBA-Dtc-NH-tBu

### [Process 1] Boc-Dtc-NH-tBu

In a methylene chloride solution containing 3.0 g of Boc-Dtc-OH, 1.45 ml of triethylamine, 2.89 g of 2-chloro-1,3-dimethylimidazolinium hexafluorophosphate and 3.28 ml of tert-butylamine were added, and the resultant mixture was stirred for 14 hr. The resultant solution was treated similarly to that in Comparative Example BB (Process 3) to give 2.49 g of the title compound as a mixture of cisoide and transoid.
TLC: Rf 0.54, 0.24 (chloroform : methanol = 40:1)

### [Process 2] Boc-(2S,3S)-AHPBA-Dtc-NH-tBu

Deprotection of 2.49 g of the compound obtained by the process 1 was performed similary to that in Comparative Example X (Process 3), and the obtained product was dissolved in 60 ml of methylene chloride and neutralized with 1.10 ml of triethylamine under ice cooling. To the neutralized solution, 3.75 g of Boc-(2S,3S)-AHPBA-OH.DCHA salt, 3.48 g of Bop reagent and 1.10 ml of triethylamine were added and the resultant mixture was stirred overnight. Further, 1.74 g of Bop reagent and 1.10 ml of triethylamine were added and the resultant mixture was stirred overnight. The reaction mixture was treated similarly to that in Comparative Example BB (Process 3) to give 2.81 g of the title compound.
TLC: Rf 0.66, 0.73 (chloroform : methanol = 9:1)

### [Process 3] Benzyloxycarbonyl-Asn-(2S,3S)-AHPBA-Dtc-NH-tBu

Deprotection of 53 mg of the compound obtained by the process 2 was performed similarly to that in Comparative Example X (Process 3), and the obtained product was dissolved in 5 ml of DMF and neutralized with 15 µl of triethylamine under ice cooling. To the neutralized solution, 83 mg of benzyloxycarbonyl-Asn-ONp, -Asn-ONp, 17 mg of HOBt and 24 µl of N-methylmorpholine were added and the resultant mixture was stirred for 14 hr. The reaction mixture was treated similarly to that in Example 46 and crystallized by the addition of ether to give 27 mg of the crude title compound. The crude solid was dissolved in methanol, fractionated by a reversed-phase HPLC and lyophilized to give 10.6 mg of the title compound.
Analytical HPLC: 23.80 min (For the condition, see: Example 35.)
FAB-MS: 642 (M+1)

### Example 105: 1-Naphthoxyacetyl-Msa-(2S,3S)-AHPBA-Dtc-NH-tBu

### [Process 1] Boc-Msa-(2S,3S)-AHPBA-Dtc-NH-tBu

Deprotection of 69 mg of the compound obtained by Example 104 (Process 2) was performed similarly to that in Comparative Example X (Process 3), and the obtained product was dissolved in 3 ml of DMF and neutralized with 20 µl of triethylamine under ice cooling. To the neutralized solution, 38 mg of Boc-methanesulfonylalanine, 62 mg of Bop reagent and 40 µl of triethylamine were added and the resultant mixture was stirred for 2 hr. The reaction mixture was treated similarly to that in Example 55 (Process 2) to give 55 mg of the title compound.
TLC: Rf 0.62 (chloroform : methanol = 9:1)

### [Process 2] 1-Naphthoxyacetyl-Msa-(2S,3S)-AHPBA-Dtc-NH-tBu

Deprotection of 55 mg of the compound obtained by the process 1 was performed similarly to that in Comparative Example X (Process 3), and the obtained product was dissolved in 5 ml of DMF and neutralized with 12 µl of triethylamine under ice cooling. To the neutralized solution, 18 mg of 1-naphthoxyacetic acid, 38 mg of Bop reagent, and 24 µl of triethylamine were added and the resultant mixture was stirred for 2 hr. The reaction mixture was treated similarly to that in Example 46, except for the chromatography solvent (chloroform : methanol = 15:1), to give 37 mg of the crude title compound. In methanol, 14 mg of the crude solid was dissolved, fractionated by a reversed-phase HPLC and lyophilized to give 6.5 mg of the title compound.
Analytical HPLC: 29.40 min (For the condition, see: Example 35.)
FAB-MS: 727 (M+1)

### Example 106: 1-Naphthoxyacetyl-Asn-(2S,3S)-AHPBA-Dtc-NH-tBu

### [Process 1] Boc-Asn-(2S, 3S)-AHPBA-Dtc-NH-tBu

Deprotection of 2.81 g of the compound obtained by Example 104 (Process 2) was performed similarly to that in Comparative Example X (Process 3), and the obtained product was dissolved in 50 ml of DMF and neutralized with 0.79 ml of triethylamine under ice cooling. To the neutralized solution, 3.02 g of Boc-Asn-ONp, 1.31 g of HOBt and 0.94 ml of N-methylmorpholine were added and the resultant mixture was stirred for 14 hr. The reaction mixture was evaporated under reduced pressure and the residue was mixed with 5% sodium hydrogencarbonate aqueous solution to give precipitates. The precipitates were filtered, washed and dried. The precipitates were subjected to a silica gel column chromatography (chloroform : methanol = 10:1) to give 1.50 g of the title compound.
TLC: Rf 0.30 (chloroform : methanol = 9:1)

### [Process 2] 1-Naphthoxyacetyl-Asn-(2S,3S)-AHPBA-Dtc-NH-tBu

Deprotection of 44 mg of the compound obtained by the process 1 was performed similarly to that in Comparative Example X (Process 3), and the obtained product was dissolved in 5 ml of DMF and neutralized with 10 µl of triethylamine under ice cooling. To the neutralized solution, 15 mg of 1-naphthoxyacetic acid, 32 mg of Bop reagent and 20 µl of triethylamine were added and the resultant mixture was stirred for 2 hr. The reaction mixture was treated similarly to that in Example 46, except for the chromatography solvent (chloroform : methanol = 15:1), to give the above mentioned compound. The solid was dissolved in methanol, fractionated by a reversed-phase HPLC and lyophilized to give 10.5 mg of the title compound.
Analytical HPLC: 26.68 min (For the condition, see: Example 35.)
FAB-MS: 692 (M+1)
¹H NMR (DMSO-d₆, 500 MHz): Fig. 4

### Example 107: 1-Naphthylmethyloxycarbonyl-Asn-(2S,3S)-AHPBADtc-NH-tBu

Deprotection of 40 mg of the compound obtained by Example 106 (Process 1) was performed similarly to that in Comparative Example X (Process 3), and the obtained product was dissolved in 5 ml of DMF and neutralized with 9 µl of triethylamine under ice cooling. To the neutralized solution, 32 mg of 1-naphthylmethyl 4-nitrophenyl carbonate, 15 mg of HOBt and 14 µl of N-methylmorpholine were added and the resultant mixture was stirred for 14 hr. The reaction mixture was evaporated under reduced pressure and the resultant residue was mixed with 5% aqueous sodium hydrogencarbonate solution to give precipitates. The precipitates were filtered, washed with water and dried. The dried precipitates were dissolved in methanol, fractionated by a reversed-phase HPLC and lyophilized to give 6.4 mg of the title compound.
Analytical HPLC: 26.93 min (For the condition, see: Example 35.)
FAB-MS: 692 (M+1)

### Example 108: (E)-Phenyl-CH=CH-CH₂CO-Asn-(2S,3S)-AHPBA-Dtc-NH-tBu

Deprotection of 40 mg of the compound obtained by Example 106 (Process 1) was performed similarly to that in Comparative Example X (Process 3), and the obtained product was dissolved in 5 ml of DMF and neutralized with 9 µl of triethylamine under ice cooling. To the neutralized solution, 11 mg of transstyrylacetic acid, 29 mg of Bop reagent and 18 µl of triethylamine were added and the resultant mixture was stirred for 2 hr. The reaction mixture was treated similarly to that in Example 107 to give 5.6 mg of the title compound.
Analytical HPLC: 24.49 min (For the condition, see: Example 35.)
FAB-MS: 652 (M+1)

### Example 109:

### o-Chlorophenoxyacetyl-Asn-(2S,3S)-AHPBA-Dtc-NH-tBu

Deprotection of 40 mg of the compound obtained by Example 106 (Process 1) was performed similarly to that in Comparative Example X (Process 3), and the obtained product was dissolved in 5 ml of DMF and neutralized with 9 µl of triethylamine under ice cooling. To the neutralized solution, 11 mg of o-chlorophenoxyacetic acid, 29 mg of Bop reagent and 18 µl of triethylamine were added and the resultant mixture was stirred for 2 hr. The reaction mixture was treated similarly to that in Example 107 to give 10.5 mg of the title compound.
Analytical HPLC: 24.49 min (For the condition, see: Example 35.)
FAB-MS: 676 (M+1)

### Example 110: o-Phenylphenoxyacetyl-Asn-(2S,3S)-AHPBA-Dtc NH-tBu

### [Process 1] o-Phenylphenoxyacetic acid DCHA salt

In 10 ml of acetonitrile, 1.0 g of o-phenylphenol and 1.29 ml of ethyl bromoacetate were added in the presence of 1.76 ml of 1,8-diazabicyclo[5.4.0]-7-undecene (DBU) and the resultant mixture was refluxed for 8 hr. The reaction mixture was treated similarly to that in Comparative Example X (Process2) to give 1.46 g of ethyl o-phenylphenoxyacetate. The ester was dissolved in 30 ml of ethanol, mixed with 10.8 ml of 1N-NaOH aqueous solution and stirred for 12 hr. The reaction mixture was evaporated under reduced pressure, acidified by the addition of 1N-HCl and extracted with ethyl acetate. The extract was washed with saturated sodium chloride aqueous solution and dried over anhydrous sodium sulfate. The dried solution was evaporated under reduced pressure and o-phenylphenoxyacetic acid was crystallized as DCHA salt from ether with the yield of 1.59 g.
TLC: Rf 0.40 (chloroform : methanol:acetic acid = 9:1:0.5)

### [Process 2] o-Phenylphenoxyacetyl-Asn-(2S,3S)-AHPBA-Dtc-NH-tBu

Deprotection of 40 mg of the compound obtained by Example 106 (Process 1) was performed similarly to that in Comparative Example X (Process 3), and the obtained product was dissolved in 5 ml of DMF and neutralized with 9 µl of triethylamine under ice cooling. To the neutralized solution, 34 mg of o-phenylphenoxyacetic acid DCHA salt, 29 mg of Bop reagent and 18 µl of triethylamine were added and the resultant mixture was stirred for 2 hr. The reaction mixture was treated similarly to that in Example 107 to give 9.4 mg of the title compound.
Analytical HPLC: 28.38 min (For the condition, see: Example 35.)
FAB-MS: 718 (M+1)

### Example 111: m-Phenylphenoxyacetyl-Asn-(2S,3S)-AHPBA-Dtc-NH-tBu

The compound shown above was obtained by the similar method to that of Example 110.
Analytical HPLC: 28.40 min (For the condition, see: Example 35.)
FAB-MS: 718 (M+1)

### Example 112: p-Phenylphenoxyacetyl-Asn-(2S,3S)-AHPBA-Dtc-NH-tBu

The compound shown above was obtained by the similar method to that of Example 110.
Analytical HPLC: 28.16 min (For the condition, see: Example 35.)
FAB-MS: 718 (M+1)

### Example 113: m-Chlorophenoxyacetyl-Asn-(2S,3S)-AHPBA-Dtc-NH-tBu

The compound shown above was obtained by the similar method to that of Example 110.
Analytical HPLC: 25.24 min (For the condition, see: Example 35.)
FAB-MS: 676 (M+1)

### Example 114: 5,6,7,8-Tetrahydro-1-naphthoxyacetyl-Asn-(2S,3S)-AHPBA-Dtc-NH-tBu

The compound shown above was obtained by the similar method to that of Example 110.
Analytical HPLC: 28.48 min (For the condition, see: Example 35.)
FAB-MS: 696 (M+1)

### Example 115: 5-Isoquinolyloxyacetyl-Asn-(2S,3S)-AHPBA-Dtc-NH-tBu

### [Process 1] 5-Isoquinolyloxyacetic acid

In 10 ml of acetonitrile, 1.0 g of 5-hydroxyisoquinoline and 1.52 ml of ethyl bromoacetate were added in the presence of 2.07 ml of DBU and the resultant mixture was refluxed for 8 hr. The reaction mixture was evaporated under reduced pressure and the resultant residue was redissolved in 1N-HCl, washed with ethyl acetate. The aqueous layer was made alkaline with sodium hydrogencarbonate and extracted with ethyl acetate. The extract was washed with saturated sodium chloride aqueous solution and dried over anhydrous sodium sulfate. The dried solution was evaporated under reduced pressure and subjected to a silica gel column chromatography (chloroform) to give 1.46 g of ethyl 5-isoquinolyloxyacetate. The ester was dissolved in 30 ml of ethanol, mixed with 6.5 ml of 1N-NaOH aqueous solution and stirred for 12 hr. The reaction mixture was evaporated under reduced pressure, neutralized by the addition of 1N-HCl and the precipitated crystals were filtered, washed with water and dried to give 0.68 g of the title compound.

### [Process 2] 5-Isoquinolyloxyacetyl-Asn-(2S,3S)-AHPBA-Dtc-NH-tBu

Deprotection of 40 mg of the compound obtained by Example 106 (Process 1) was performed similarly to that in Example 28 (Process 3), and the obtained product was dissolved in 5 ml of DMF and neutralized with 9 µl of triethylamine under ice cooling. To the neutralized solution, 14 mg of 5-isoquinolyloxyacetic acid, 29 mg of Bop reagent and 18 µl of triethylamine were added and the resultant mixture was stirred for 2 hr. The reaction mixture was treated similarly to that in Example 107 to give 3.3 mg of the title compound.
Analytical HPLC: 14.84 min (For the condition, see: Example 35.)
FAB-MS: 693 (M+1)

### Example 116: m-Phenylaminophenoxyacetyl-Asn-(2S,3S)-AHPBA-Dtc-NH-tBu

The compound shown above was obtained by a similar method to that of Example 115.
Analytical HPLC: 26.97 min (For the condition, see: Example 35.)
FAB-MS: 733 (M+1)

### Example 117: 8-Quinolyloxyacetyl-Asn-(2S,3S)-AHPBA-Dtc-NH-tBu

The compound shown above was obtained by a similar method to that of Example 115.
Analytical HPLC: 15.49 min (For the condition, see: Example 35.)
FAB-MS: 693 (M+1)

### Example 118: 2-Quinolinecarbonyl-Asn-(2S,3S)-AHPBA-Dtc-NH-tBu

Deprotection of 40 mg of the compound obtained by Example 106 (Process 1) was performed similarly to that in Comparative Example X (Process 3), and the obtained product was dissolved in 5 ml of DMF and neutralized with 9 µl of triethylamine under ice cooling. To the neutralized solution, 12 mg of 2-quinolinecarboxylic acid, 29 mg of Bop reagent and 18 µl of triethylamine were added and the resultant mixture was stirred for 2 hr. The reaction mixture was evaporated under reduced pressure and the resultant residue was re-dissolved in ethyl acetate. The extract was washed with 5% sodium hydrogencarbonate aqueous solution, then with saturated sodium chloride aqueous solution and dried over anhydrous sodium sulfate. The dried solution was evaporated under reduced pressure, dissolved in methanol, fractionated by reversed-phase HPLC and lyophilized to give 7.2 mg of the title compound.
Analytical HPLC: 24.25 min (For the condition, see: Example 35.)
FAB-MS: 648 (M+1)

### Example 119: 1-Naphthoxyacetyl-Mta-(2S,3S)-AHPBA-Dtc-NH-tBu

### [Process 1] Boc-Mta-(2S,3S)-AHPBA-Dtc-NH-tBu

Deprotection of 390 mg of the compound obtained by Example 104 (Process 2) was performed similarly to that in Comparative Example X (Process 3), and the obtained product was dissolved in 10 ml of DMF and neutralized with 110 µl of triethylamine under ice cooling. To the neutralized solution, 186 mg of Boc-methylthioalanine, 349 mg of Bop reagent and 220 µl of triethylamine were added and the resultant mixture was stirred for 2 hr. The reaction mixture was treated similarly to that in Comparative Example X (Process 3) to give 166 mg of the title compound.
TLC: Rf 0.63 (chloroform : methanol = 9:1)

### [Process 2] 1-Naphthoxyacetyl-Mta-(2S,3S)-AHPBA-Dtc-NH-tBu

Deprotection of 50 mg of the compound obtained by the process 1 was performed similarly to that in Comparative Example X (Process 3)in the presence of 25 µl of anisole, and the obtained product was dissolved in 5 ml of DMF and neutralized with 12 µl of triethylamine under ice cooling. To the neutralized solution, 17 mg of 1-naphthoxyacetic acid, 36 mg of Bop reagent and 23 µl of triethylamine were added and the resultant mixture was stirred for 2 hr. The reaction mixture was treated similarly to that in Example 93 (Process 3) to give 61 mg of the crude above mentioned compound. In methanol, 40 mg of the crude solid was dissolved, fractionated by a reversed-phase HPLC and lyophilized to give 17.7 mg of the title compound.
Analytical HPLC: 27.88 in (The condition was as follows) Column:YMC AM-302 (4.6 x 150 mm)
Solvent A: 0.1% trifluoroacetic acid aqueous solution
Solvent B: acetonitrile
Gradient: 30% B for 2 min, then B was increased in 2% /min
Flow rate: 0.7 ml/min
FAB-MS: 695 (M+1)

### Example 120: 8-Quinolyloxyacetyl-Mta-(2S,3S)-AHPBA-Dtc-NH-tBu

The compound shown above was obtained by a similar method to that of Example 115.
Analytical HPLC: 18.89 min (For the condition, see: Example 35.)
FAB-MS: 696 (M+1)

### Comparative Example SS: 1-Naphthoxyacetyl-Mta⁺(Me)-(2S,3S)-AHPBA-Dtc-NH-tBu.AcO⁻

In methanol solution containing 10 mg of compound obtained by Example 119, 200 µl of methyl iodide was added and the resultant mixture was stirred for 7 days at 4 °C. The reaction mixture was purified by a reversed-phase HPLC (0.1% AcOH-acetonitrile) to give 4.2 mg of the title compound.
Analytical HPLC: 18.90 min (For the condition, see: Example 119.)

### Example 122: 1-Naphthoxyacetyl-Mta-(2S,3S)-AHPBA-Pro-NH-tBu

### [Process 1] Boc-Mta-(2S,3S)-AHPBA-Pro-NH-tBu

Deprotection of 20 mg of the compound obtained by Example 55 (Process 2) was performed similarly to that in Comparative Example X (Process 3), and the obtained product was dissolved in 3 ml of DMF and neutralized with 6.2 µl of triethylamine under ice cooling. To the neutralized solution, 11 mg of Boc-methylthioalanine, 20 mg of Bop reagent and 12.4 µl of triethylamine were added and the resultant mixture was stirred for 2 hr. The reaction mixture treated similarly to that in Example 46 to give 24 mg of the title compound.
TLC: Rf 0.37 (chloroform : methanol = 9:1)

### [Process 2] 1-Naphthoxyacetyl-Mta-(2S,3S)-AHPBA-Pro-NH-tBu

Deprotection of 24 mg of the compound obtained by the process 1 was performed similarly to that in Comparative Example X (Process 3) in the presence of 12 µl of anisole, and the obtained product was dissolved in 3 ml of DMF and neutralized with 5.9 µl of triethylamine under ice cooling. To the neutralized solution, 8.6 mg of 1-naphthoxyacetic acid, 18.8 mg of Bop reagent and 11.8 µl of triethylamine were added and the resultant mixture was stirred for 2 hr. The reaction mixture was treated similarly to that in Example 93 (Process 3) to give the crude compound mentioned above. In methanol, the crude product was dissolved, fractionated by a reversed-phase HPLC and lyophilized to give 6.6 mg of the title compound.
Analytical HPLC: 29.52 min (For the condition, see: Example 35.)
FAB-MS: 649 (M+1)

### Example 123:

### 1-Naphthylaminoacetyl-Msa-(2S,3S)-AHPBA-Pro-NH-tBu.AcOH

### [Process 1] 1-Naphthylaminoacetic acid

In 5 ml of THF, 500 mg of 1-naphthylamine was added, then 168 mg of sodium hydride (60% in oil) was added and the resultant mixture was stirred for 30 min under ice cooling. To the reaction mixture, 0.46 ml of ethyl bromoacetate was added and the mixture was refluxed for 5 hr. The reaction mixture was evaporated under reduced pressure and the resultant residue was re-dissolved in ethyl acetate. The obtained solution was washed with 5% sodium hydrogencarbonate aqueous solution and saturated sodium chloride aqueous solution, successively, and dried over anhydrous sodium sulfate. The dried solution was evaporated under reduced pressure and subjected to a silica gel column chromatography (n-hexane:ethyl acetate = 10:1) to give 720 mg of ethyl 1-naphthylaminoacetate. In 10 ml of ethanol, 460 mg of the ester was dissolved, 3.78 ml of 1N-NaOH aqueous solution was added and the resultant mixture was stirred for 1 hr. The reaction mixture was evaporated under reduced pressure, neutralized by the addition of 1N-HCI and extracted with ethyl acetate. The extract was washed with saturated sodium chloride aqueous solution and dried over anhydrous sodium sulfate. The dried solution was evaporated under reduced pressure and crystallized by the treatment with ether to give 188 mg of the title compound.
TLC: Rf 0.46 (chloroform : methanol:acetic acid = 9:1:0.5)

### [Process 2]

### 1-Naphthylaminoacetyl-Msa-(2S,3S)-AHPBA-Pro-NH-tBu.AcOH

Deprotection of 20 mg of the compound obtained by Example 90 (Process 1) was performed similarly to that in Comparative Example X (Process 3), and the obtained product was dissolved in 3 ml of DMF and neutralized with 4.7 µl of triethylamine under ice cooling. To the neutralized solution, 8.4 mg of 1-naphthylaminoacetic acid, 15 mg of Bop reagent and 9.5 µl of triethylamine were added and the resultant mixture was stirred for 2 hr. The reaction mixture was evaporated under reduced pressure and the resultant residue was re-dissolved in ethyl acetate. The obtained solution was washed with 5% sodium hydrogencarbonate aqueous solution and saturated sodium chloride aqueous solution, successively, and dried over anhydrous sodium sulfate. The dried solution was evaporated under reduced pressure and subjected to a silica gel column chromatography (chloroform : methanol = 20:1) and treated with ether to give 20 mg of the crude compound mentioned above. In methanol, 6 mg of the crude crystals were dissolved, fractionated by a reversed-phase HPLC (0.1% acetic acid-acetonitrile system), lyophilized to give 3.3 mg of the title compound.
Analytical HPLC: 24.81 min (For the condition, see: that in example 35.)
FAB-MS: 680 (M+1)

### Example 124: 1-Naphthylaminoacetyl-Msa-(2S,3S)-AHPBA-Thz-NH-tBu.AcOH

The above mentioned compound was obtained by a similar method of Example 123 [process 2].
Analytical HPLC: 27.08 min (For the condition, see: that in example 35.)
FAB-MS: 698 (M+1)

### Example 125: 1-Naphthoxyacetyl-Msa-(2S,3S)-AHPBA-Thz-NH-C(CH₃)₂-CH₂OH

### [Process 1] Boc-Thz-NH-C(CH₃)₂-CH₂OH

In a methylene chloride solution containing 0.30 g of Boc-Thz-OH, 0.12 ml of 2-amino-2-methyl-1-propanol and 0.29 g of EDC hydrochloride were added and the resultant mixture was stirred for 14 hr. The reaction mixture was treated similarly to that in Comparative Example BB (Process 3) to give 190 mg of the title compound.
TLC: Rf 0.87 (chloroform : methanol:water = 8:3:1, lower layer)

### [Process 2] Boc-(2S,3S)-AHPBA-Thz-NH-C(CH₃)₂-CH₂OH

To 40 mg of the protected peptide obtained by the [process 1], 5 ml of trifluoroacetic acid was added and the resultant mixture was stirred for 60 min at room temperature. The reaction mixture was evaporated under reduced pressure, and the resultant residue was washed with n-hexane and redissolved in 5 ml of DMF and neutralized with 19 µl of triethylamine under ice cooling. To the neutralized solution, 63 mg of Boc-(2S,3S)-AHPBA-OH.DCHA, 58 mg of Bop reagent and 36 µl of triethylamine were added and the resultant mixture was stirred for 2 hr. The reaction mixture was treated similarly to that in Example 93 (Process 3) to give 51 mg of the title compound.
TLC: Rf 0.75 (chloroform : methanol:water = 8:3:1, lower layer)

### [Process 3] Boc-Msa-(2S,3S)-AHPBA-Thz-NH-C(CH₃)₂-CH₂OH

Deprotection of 51 mg of the compound obtained by the process 2 was performed similarly to that in Example 125 (Process 2), and the obtained product was dissolved in 5 ml of DMF and neutralized with 14 µl of triethylamine under ice cooling. To the neutralized solution, 26 mg of Boc-methanesulfonylalanine, 45 mg of Bop reagent and 28 µl of triethylamine were added and the resultant mixture was stirred for 2 hr. The reaction mixture was treated similarly to that in Example 46 to give 31 mg of the title compound.
TLC: Rf 0.81 (chloroform : methanol:water = 8:3:1, lower layer)

### [Process 4] 1-Naphthoxyacetyl-Msa-(2S,3S)-AHPBA-Thz-NH-C(CH₃)₂-CH₂OH

Deprotection of 31 mg of the compound obtained by the process 3 was performed similarly to that in Example 125 (Process 2), and the obtained product was dissolved in 3 ml of DMF and neutralized with 7 µl of triethylamine under ice cooling. To the neutralized solution, 9.7 mg of 1-naphthoxyacetic acid, 21.1 mg of Bop reagent and 13.3 µl of triethylamine were added and the resultant mixture was stirred for 2 hr. The reaction mixture was treated similarly to that in Example 122 to give 10.6 mg of the title compound.
Analytical HPLC: 22.88 min (For the condition, see: that in example 35.)
FAB-MS: 715 (M+1)

### Example 126: 1-Naphthoxyacetyl-Msa-(2S,3S)-AHPBA-Pro-NH-C(CH₃)(CH₂OH)₂

### [Process 1] Boc-Pro-NH-C(CH₃)(CH₂OH)₂

In a methylene chloride solution containing 0.20 g of Boc-Pro-OH, 0.49 g of 2-amino-2-methyl-1,3-propanediol and 0.89 g of EDC hydrochloride were added and the resultant mixture was stirred for 14 hr. The reaction mixture was treated similarly to that in Example 51 (Process 1) without filtration to give 0.36 g of the title compound.
TLC: Rf 0.48 (chloroform : methanol:acetic acid = 9:1:0.5)

### [Process 2] Boc-(2S,3S)-AHPBA-Pro-NH-C(CH₃)(CH₂OH)₂

Deprotection of 50 mg of the compound obtained by the process 1 was performed similarly to that in Example 125 (Process 2), and the obtained product was dissolved in 5 ml of DMF and neutralized with 22 µl of triethylamine under ice cooling. To the neutralized solution, 75 mg of Boc-(2S,3S)-AHPBA-OH.DCHA, 70 mg of Bop reagent and 22 µl of triethylamine were added and the resultant mixture was stirred for 2 hr. The reaction mixture was treated similarly to that in Example 93 (Process 3) to give 42 mg of the title compound.
TLC: Rf 0.74 (chloroform : methanol:water = 8:3:1, lower layer)

### [Process 3] Boc-Msa-(2S,3S)-AHPBA-Pro-NH-C(CH₃)(CH₂OH)₂

Deprotection of 42 mg of the compound obtained by the process 2 was performed similarly to that in Example 125 (Process 2), and the obtained product was dissolved in 5 ml of DMF and neutralized with 12 µl of triethylamine under ice cooling. To the neutralized solution, 23 mg of Boc-methanesulfonylalanine, 38 mg of Bop reagent and 12 µl of triethylamine were added and the resultant mixture was stirred for 2 hr. The reaction mixture was treated similarly to that in Example 93 (Process 3) to give 25 mg of the title compound.
TLC: Rf 0.63 (chloroform : methanol:water = 8:3:1, lower layer)

### [Process 4] 1-Naphthoxyacetyl-Msa-(2S,3S)-AHPBA-Pro-NH-C(CH₃)(CH₂OH)₂

Deprotection of 25 mg of the compound obtained by the process 3 was performed similarly to that in Example 125 (Process 2), and the obtained product was dissolved in 3 ml of DMF and neutralized with 5.4 µl of triethylamine under ice cooling. To the neutralized solution, 7.8 mg of 1-naphthoxyacetic acid, 17.1 mg of Bop reagent and 10.8 µl of triethylamine were added and the resultant mixture was stirred for 2 hr. The reaction mixture was treated similarly to that in Example 122 to give 4.5 mg of the title compound.
Analytical HPLC: 19.37 min (For the condition, see: that in example 35.)
FAB-MS: 713 (M+1)

### Example 127: 1-Naphthoxyacetyl-Asn-(2S,3S)-AHPBA-Thz-piperidine

### [Process 1] Boc-Thz-piperidine

In a methylene chloride solution containing 300 mg of Boc-Thz-OH, 358 µl of triethylamine, 358 mg of 2-chloro-1, 3-dimethylimidazolidinium hexafluorophosphate and 153 µl of piperidine were added under ice cooling and the resultant mixture was stirred for 14 hr. The reaction mixture was treated similarly to that in Comparative Example BB (Process 3) to give 300 mg of the title compound.
TLC: Rf 0.41 (chloroform : methanol = 40:1)

### [Process 2] Boc-(2S,3S)-AHPBA-Thz-piperidine

Deprotection of 100 mg of the compound obtained by the process 1 was performed similarly to that in Comparative Example X (Process 3), and the obtained product was dissolved in 10 ml of methylene chloride and neutralized with 46 µl of triethylamine under ice cooling. To the neutralized solution, 159 mg of Boc-(2S,3S)-AHPBA-OH.DCHA, 147 mg of Bop reagent and 46 µl of triethylamine were added and the resultant mixture was stirred for 2 hr. The reaction mixture was treated similarly to that in Example 46 to give 134 mg of the title compound.
TLC: Rf 0.59 (chloroform : methanol = 9:1)

### [Process 3] Boc-Asn-(2S,3S)-AHPBA-Thz-piperidine

Deprotection of 103 mg of the compound obtained by the process 2 was performed similarly to that in Comparative Example X (Process 3), and the obtained product was dissolved in 10 ml of DMF and neutralized with 30 µl of triethylamine under ice cooling. To the neutralized solution, 114 mg of Boc-Asn-ONp, 50 mg of HOBt, 36 µl of N-methylmorpholine were added and the resultant mixture was stirred for 14 hr. The reaction mixture was evaporated under reduced pressure and the resultant residue was mixed with 5% sodium hydrogencarbonate aqueous solution. The obtained solid was subjected to a silica gel column chromatography (chloroform : methanol = 10:1) to give 65 mg of the title compound.
TLC: Rf 0.69 (chloroform : methanol:water = 8:3:1, lower layer)

### [Process 4] 1-Naphthoxyacetyl-Asn-(2S,3S)-AHPBA-Thz-piperizine

Deprotection of 65 mg of the compound obtained by the process 3 was performed similarly to that in Comparative Example X (Process 3), and the obtained product was dissolved in 8 ml of DMF and neutralized with 15 µl of triethylamine under ice cooling. To the neutralized solution, 23 mg of 1-naphthoxyacetic acid, 49 mg of Bop reagent and 30 µl of triethylamine were added and the resultant mixture was stirred for 2 hr. The reaction mixture was evaporated under reduced pressure and the resultant residue was mixed with 5% sodium hydrogencarbonate aqueous solution to give precipitates. The formed precipitates were collected, washed with water and dried in vacuo to give 63 mg of the crude product. In methanol, 25 mg of the crude product was dissolved, fractionated by a reversed-phase HPLC and lyophilized to give 6.8 mg of the title compound.
Analytical HPLC: 20.42 min (For the condition, see: that in example 35.)
FAB-MS: 676 (M+1)

### Example 128: 1-Naphthoxyacetyl-Asn-(2S,3S)-AHPBA-Thz-NH-cyclopentyl

### [Process 1] Boc-Thz-NH-cyclopentyl

In a methylene chloride solution containing 200 mg of Boc-Thz-OH, 238 µl of triethylamine, 379 mg of Bop reagent, and 102 µl of cyclopentylamine were added under ice cooling and the resultant mixture was stirred for 14 hr. The reaction mixture was treated similarly to that in Comparative Example X (Process 3) to give 235 mg of the title compound.

### [Process 2] Boc-(2S,3S)-AHPBA-Thz-NH-cyclopentyl

Deprotection of 100 mg of the compound obtained by the process 1 was performed similarly to that in Comparative Example X (Process 3), and the obtained product was dissolved in 10 ml of methylene chloride and neutralized with 46 µl of triethylamine under ice cooling. To the neutralized solution, 159 mg of Boc-(2S,3S)-AHPBA-OH DCHA salt, 147 mg of Bop reagent and 46 µl of triethylamine were added and the resultant mixture was stirred for 2 hr. The reaction mixture was treated similarly to that in Example 46 to give 50 mg of the title compound.
TLC: Rf 0.59 (chloroform : methanol = 9:1)

### [Process 3] Boc-Asn-(2S,3S)-AHPBA-Thz-NH-cyclopentyl

Deprotection of 50 mg of the compound obtained by the process 2 was performed similarly to that in Comparative Example X (Process 3), and the obtained product was dissolved in 5 ml of DMF and neutralized with 15 µl of triethylamine under ice cooling. To the neutralized solution, 56 mg of Boc-Asn-ONp, 24 mg of HOBt and 17 µl of N-methylmorpholine were added and the resultant mixture was stirred for 14 hr. The reaction mixture was treated similarly to that in Example 93 (Process 3) to give 20 mg of the title compound.
TLC: Rf 0.66 (chloroform : methanol:water = 8:3:1, lower layer)

### [Process 4] 1-Naphthoxyacetyl-Asn-(2S,3S)-AHPBA-Thz-NH-cyclopentyl

The compound mentioned above was obtained by a similar method to that in Example 127, [process 4].
Analytical HPLC: 24.13 min (For the condition, see: that in Example 35.)
FAB-MS: 676 (M+1)

### Example 129: m-Phenylphenoxyacetyl-Mta-(2S,3S)-AHPBA-Dtc-NH-tBu

The compound mentioned above was obtained by a similar method to that in Example 119.
Analytical HPLC: 29.01 min (For the condition, see: that in Example 119.)
FAB-MS: 721 (M+1)

### Example 130:5-Isoquinolyloxyacetyl-Mta-(2S,3S)-AHPBA-Dtc-NH-tBu.AcOH

The compound mentioned above was obtained by a similar method to that in Example 123 (Process 2) using the compounds obtained in Example 119 (Process 1) and Example 115 (Process 1).
Analytical HPLC: 18.00 min (For the condition, see: that in example 35.)
FAB-MS: 696 (M+1)
¹H NMR (DMSO-d₆, 500 MHz): Fig. 5

### Example 131: 2-Naphthoxyacetyl-Msa-(2S,3S)-AHPBA-Pro-NH-tBu

Deprotection of 23 mg of the compound obtained by Example 90 (Process 1) was performed similarly to that in Comparative Example X (Process 3), and the obtained product was dissolved in 3 ml of DMF and neutralized with 5.4 µl of triethylamine under ice cooling. To the neutralized solution, 8 mg of 2-naphthoxyacetic acid, 17 mg of Bop reagent and 11 µl of triethylamine were added and the resultant mixture was stirred for 2 hr. The reaction mixture was treated similarly to that in Example 30 (Process 4) and treated with hexane to give 22 mg of the crude compound mentioned above. In methanol, 22 mg of the crude crystals were dissolved and, fractionated by a reversed-phase HPLC and lyophilized to give 11.8 mg of the title compound.
TLC: Rf 0.88 (chloroform : methanol = 9:1)
Analytical HPLC: 25.39 min (For the condition, see: that in example 35.)
FAB-MS: 681 (M+1)

### Comparative Example TT : 1-Naphthoxyacetyl-Hse-(2S,3S)-AHPBA-Thz-NH-tBu

### [Process 1] Boc-Hse-(2S,3S)-HPBA-Thz-NH-tBu

Deprotection of 21 mg of the compound obtained by Example 82 (Process 2) was performed similarly to that in Comparative Example X (Process 3), and the obtained product was dissolved in 3 ml of DMF and neutralized with 6.3 µl of triethylamine under ice cooling. To the neutralized solution, 18 mg of Boc-Hse-OH.DCHA, 20 mg of Bop reagent and 6.3 µl of triethylamine were added and the resultant mixture was stirred for 2 hr. The reaction mixture was treated similarly to that in Comparative Example X (Process 2), except for the chromatography solvent (chloroform : methanol = 40:1), to give 20 mg of the title compound.
TLC: Rf 0.83 (chloroform : methanol = 9:1)

### [Process 2] 1-Naphthoxyacetyl-Hse-(2S,3S)-AHPBA-Thz-NH-tBu

Deprotection of 20 mg of the compound obtained by the process 1 was performed similarly to that in Comparative Example X (Process 3), and the obtained product was dissolved in 3 ml of DMF and neutralized with 4.9 µl of triethylamine under ice cooling. To the neutralized solution, 8 mg of 1-naphthoxyacetic acid, 16 mg of Bop reagent and 9.8 µl of triethylamine were added and the resultant mixture was stirred for 2 hr. The reaction mixture was treated similarly to that in the process 1 to give 22 mg of the crude compound mentioned above. In methanol, the crude product was dissolved, fractionated by a reversed-phase HPLC and lyophilized to give 6.1 mg of the title compound.
TLC: Rf 0.57 (chloroform : methanol = 9:1)
Analytical HPLC: 21.31 min (For the condition, see: that in example 35.)
FAB-MS: 651 (M+1)

### Comparative Example UU: 1-Naphthoxyacetyl-Thr-(2S,3S)-AHPBA-Thz-NH-tBu

### [Process 1] Boc-Thr-(2S,3S)-AHPBA-Thz-NH-tBu

Deprotection of 19 mg of the compound obtained by Example 82 (Process 2) was performed similarly to that in Comparative Example X (Process 3), and the obtained product was dissolved in 3 ml of DMF and neutralized with 4.3 µl of triethylamine under ice cooling. To the neutralized solution, 9 mg of Boc-Thr-OH, 18 mg of Bop reagent and 6.2 µl of triethylamine were added and the resultant mixture was stirred for 2 hr. The reaction mixture was treated similarly to that in Example TT (Process 1) to give 24 mg of the title compound.
TLC: Rf 0.66 (chloroform : methanol = 9:1)

### [Process 2] 1-Naphthoxyacetyl-Thr-(2S,3S)-AHPBA-Thz-NH-tBu

Deprotection of 29 mg of the compound obtained by the process 1 was performed similarly to that in Comparative Example X (Process 3), and the obtained product was dissolved in 3 ml of DMF and neutralized with 7.1 µl of triethylamine under ice cooling. To the neutralized solution, 11 mg of 1-naphthoxyacetic acid, 23 mg of Bop reagent and 14.2 µl of triethylamine were added and the resultant mixture was stirred for 2 hr. The reaction mixture was treated similarly to that in Comparative Example TT (Process 2) to give 6.7 mg of the title compound.
TLC: Rf 0.66 (chloroform : methanol = 9:1)
Analytical HPLC: 26.32 min (For the condition, see: that of Example 35.)
FAB-MS: 651 (M+1)

### Example 134: 1-Naphthoxyacetyl-Tle-(2S,3S)-AHPBA-Thz-NH-tBu

### [Process 1] Boc-Tle-(2S,3S)-AHPBA-Thz-NH-tBu

Deprotection of 39 mg of the compound obtained by Example 82 (Process 2) was performed similarly to that in Comparative Example X (Process 3), and the obtained product was dissolved in 3 ml of DMF and neutralized with 11.6 µl of triethylamine under ice cooling. To the neutralized solution, 19.4 mg of Boc-Tle-OH, 37 mg of Bop reagent and 23.3 µl of triethylamine were added and the resultant mixture was stirred for 2 hr. The reaction mixture was treated similarly to that in Comparative Example TT (Process 1) to give 17 mg of the title compound.
TLC: Rf 0.94 (chloroform : methanol = 9:1)

### [Process 2] 1-Naphthoxyacetyl-Tle-(2S,3S)-AHPBA-Thz-NH-tBu

Deprotection of 17 mg of the compound obtained by the process 1 was performed similarly to that in Example 28 (Process 3), and the obtained product was dissolved in 3 ml of DMF and neutralized with 4.1 µl of triethylamine under ice cooling. To the neutralized solution, 11 mg of 1-naphthoxyacetic acid, 13 mg of Bop reagent and 8.2 µl of triethylamine were added and the resultant mixture was stirred for 2 hr. The reaction mixture was treated similarly to that in Comparative Example Z (Process 3) to give 15 mg of the crude compound. The crude product was dissolved in methanol, fractionated by a reversed-phase HPLC and lyophilized to give 2.3 mg of the title compound.
TLC: Rf 0.87 (chloroform : methanol = 9:1)
Analytical HPLC: 31.60 min (For the condition, see: that in example 35.)
FAB-MS: 663 (M+1)

### Example 135: 1-Naphthoxyacetyl-Msa-(2S,3S)-AHPBA-Thz-N-CH(CH(CH₃)₂)-CH₂OH

### [Process 1] Boc-Valinol [Boc-Valol]

In 5 ml of 1,2-dimethoxyethane (DME), 1.086 g of Boc-Val-OH was dissolved and 550 µl of N-methylmorpholine and 650 µl of isobutyl chloroformate were successively added dropwise at -15 °C. After stirring for 1 min, N-methylmorpholine hydrochloride was filtered off and washed twice with 2.5 ml each of DME. The filtrate and washings were combined and a solution of 284 mg of sodium borohydride in 2.5 ml of water was added in one portion. After 30 sec, 125 ml of water was added and the mixture was extracted with 25 ml of ethyl acetate and dried over anhydrous sodium sulfate. The dried solution was evaporated under reduced pressure to give 879 mg of the title compound.
TLC: Rf 0.52 (chloroform : methanol = 20:1)

### [Process 2] Boc-Thz-Valol

Deprotection of 94 mg of the compound obtained by the process 1 was performed similarly to that in Comparative Example Z (Process 2), and the obtained product was dissolved in 3 ml of DMF and neutralized with 88.9 µl of triethylamine under ice cooling. To the neutralized solution, 164 mg of Boc-Thz-OH, 108 mg of HOBt and 147 mg of EDC hydrochloride were added and the resultant mixture was stirred for 2 hr. The reaction mixture was treated similarly to that in Comparative Example TT (Process 1) to give 50 mg of the title compound.
TLC: Rf 0.60 (chloroform : methanol = 9:1)

### [Process 3] Boc-(2S,3S)-AHPBA-Thz-Valol

Deprotection of 50 mg of the compound obtained by the process 2 was performed similarly to that in Example 125 (Process 2), and the obtained product was dissolved in 3 ml of DMF and neutralized with 21.8 µl of triethylamine under ice cooling. To the neutralized solution, 75 mg of Boc-(2S,3S)-AHPBA-OH.DCHA. 70 mg of BOP reagent and 43.6 µl of triethylamine were added and the resultant mixture was stirred for 2 hr. The reaction mixture was treated similarly to that in Comparative Example Z (Process 4) to give 39 mg of the title compound.
TLC: Rf 0.45 (chloroform : methanol = 9:1)

### [Process 4] Boc-Msa-(2S,3S)-AHPBA-Thz-Valol

Deprotection of 39 mg of the compound obtained by the process 3 was performed similarly to that in Comparative Example Z (Process 2), and the obtained product was dissolved in 3 ml of DMF and neutralized with 10.9 µl of triethylamine under ice cooling. To the neutralized solution, 22 mg of Boc-Msa-OH, 35 mg of Bop reagent and 21.9 µl of triethylamine were added and the resultant mixture was stirred for 2 hr. The reaction mixture was treated similarly to that in Comparative Example TT (Process 1) to give 25 mg of the title compound.
TLC: Rf 0.83 (chloroform : methanol = 9:1)

### [Process 5] 1-Naphthoxyacetyl-Msa-(2S,3S)-AHPBA-Thz-Valol

Deprotection of 25 mg of the compound obtained by the process 4 was performed similarly to that in Comparative Example Z (Process 2), and the obtained product was dissolved in 3 ml of DMF and neutralized with 5.4 µl of triethylamine under ice cooling. To the neutralized solution, 8 mg of 1-naphthoxyacetic acid, 18 mg of Bop reagent and 10.8 µl of triethylamine were added and the resultant mixture was stirred for 2 hr. The reaction mixture was treated similarly to that in Comparative Example TT (Process 2) to give 2.8 mg of the title compound.
TLC: Rf 0.47 (chloroform : methanol = 9:1)
Analytical HPLC: 24.06 min (For the condition, see: that of example 35)
FAB-MS: 729 (M+1)

### Example 136: 2-Benzofurancarbonyl-Msa-(2S,3S)-AHPBA-Thz-NH-tBu

Deprotection of 52 mg of the compound obtained by Example 100 (Process 1) was performed similarly to that in Comparative Example X (Process 3), and the obtained product was dissolved in 3 ml of DMF and neutralized with 11.8 µl of triethylamine under ice cooling. To the neutralized solution, 14 mg of 2-benzofurancarboxylic acid, 38 mg of Bop reagent and 23.5 µl of triethylamine were added and the resultant mixture was stirred for 2 hr. The reaction mixture was treated similarly to that in Comparative Example TT (Process 2) to give 1.9 mg of the title compound.
TLC: Rf 0.64 (chloroform : methanol = 9:1)
Analytical HPLC: 24.44 min (For the condition, see: that in example 35.)
FAB-MS:659 (M+1)

### Example 137: 1-Naphthoxyacetyl-Msa-(2S,3S)-AHPBA-Thz-NH-CH(CH(CH₃)-(C₂H₅))-CH₂OH

### [Process 1] Boc-Isoleucinol [Boc-Ileol]

In 5 ml of 1,2-dimethoxyethane (DME), 1.201 g of Boc-Ile-OH.1/2H₂O was dissolved and 550 µl of N-methylmorpholine and 650 µl of isobutyl chloroformate were successively added dropwise at -15 °C. After stirring for 1 min, the formed N-methylmorpholine hydrochloride was filtered off and washed twice with 2.5 ml each of DME. The filtrate and washings were combined and 284 mg of sodium borohydride in 2.5 ml of water was added in one portion. After 30 sec, 125 ml of water was added and the mixture was extracted with 25 ml of ethyl acetate and dried over anhydrous sodium sulfate. The dried solution was evaporated under reduced pressure to give 1.040 g of the title compound.
TLC: Rf 0.46 (chloroform : methanol = 20:1)

### [Process 2] Boc-Thz-Ileol

Deprotection of 127 mg of the compound obtained by the process 1 was performed similarly to that in Comparative Example Z (Process 2), and the obtained product was dissolved in 3 ml of DMF and neutralized with 81.2 µl of triethylamine under ice cooling. To the neutralized solution, 136 mg of Boc-Thz-OH, 258 mg of Bop reagent and 162 µl of triethylamine were added and the resultant mixture was stirred for 2 hr. The reaction mixture was treated similarly to that in Comparative Example TT (Process 1) to give 134 mg of the title compound.
TLC: Rf 0.39 (chloroform : methanol = 20:1)

### [Process 3] Boc-(2S,3S)-AHPBA-Thz-Ileol

Deprotection of 134 mg of the compound obtained by the process 2 was performed similarly to that in Comparative Example Z (Process 2), and the obtained product was dissolved in 3 ml of DMF and neutralized with 56 µl of triethylamine under ice cooling. To the neutralized solution, 119 mg of Boc-(2S,3S)-AHPBA-OH, 178 mg of Bop reagent and 112 µl of triethylamine were added and the resultant mixture was stirred for 2 hr. The reaction mixture was treated similarly to that in Comparative Example Z (Process 4) to give 54 mg of the title compound.
TLC: Rf 0.37 (chloroform : methanol = 20:1)

### [Process 4] Boc-Msa-(2S,3S)-AHPBA-Thz-Ileol

Deprotection of 54 mg of the compound obtained by the process 3 was performed similarly to that in Comparative Example Z (Process 2), and the obtained product was dissolved in 3 ml of DMF and neutralized with 14.7 µl of triethylamine under ice cooling. To the neutralized solution, 29 mg of Boc-Msa-OH, 47 mg of Bop reagent and 29.5 µl of triethylamine were added and the resultant mixture was stirred for 2 hr. The reaction mixture was treated similarly to that in Comparative Example TT (Process 1) to give 22 mg of the title compound.
TLC: Rf 0.18 (chloroform : methanol 20:1)

### [Process 5]: 1-Naphthoxyacetyl-Msa-(2S,3S)-AHPBA-Thz-Ileol

Deprotection of 22 mg of the compound obtained by the process 4 was performed similarly to that in Comparative Example Z (Process 2), and the obtained product was dissolved in 3 ml of DMF and neutralized with 4.6 µl of triethylamine under ice cooling. To the neutralized solution, 7 mg of 1-naphthoxyacetic acid, 15 mg of Bop reagent and 9.3 µl of triethylamine were added and the resultant mixture was stirred for 2 hr. The reaction mixture was evaporated under reduced pressure and dissolved in ethyl acetate. The solution was washed with 5% citric acid aqueous solution, 5% sodium hydrogencarbonate aqueous solution and saturated sodium chloride aqueous solution, successively, and dried over anhydrous sodium sulfate. The dried solution was evaporated under reduced pressure to give 45 mg of a crude product. The crude product was dissolved in methanol, fractionated by a reversed-phase HPLC and lyophilized to give 7.9 mg of the title compound.
TLC: Rf 0.41 (chloroform : methanol = 20:1)
Analytical HPLC: 21.54 min (For the condition, see: that of example 35)
FAB-MS: 743(M+1)

### Example 138: 2-Quinolinecarbonyl-Asn-(2S,3S)-AHPBA-Pro-NH-tBu

Deprotection of 18 mg of the compound obtained by Example 80 (Process 2) was performed similarly to that in Comparative Example X (Process 3), and the obtained product was dissolved in 3 ml of DMF and neutralized with 4.5 µl of triethylamine under ice cooling. To the neutralized solution, 7 mg of 2-quinolinecarboxylic acid, 17 mg of Bop reagent and 9.8 µl of triethylamine were added and the resultant mixture was stirred for 2 hr. The reaction mixture was treated similarly to that in Example 118 to give 6.1 mg of the title compound.
TLC: Rf 0.89 (chloroform : methanol = 9:1)
Analytical HPLC: 20.30 min (For the condition, see: that in example 35.)
FAB-MS: 603 (M+1)

### Comparative Example VV: 1-Naphthoxyacetyl-Asp(NHNH₂)-(2S,3S)-AHPBA-Pro-NH-tBu.AcOH

### [Process 1] Boc-Asp(OBzl)-(2S,3S)-AHPBA-Pro-NH-tBu

Deprotection of 25 mg of the compound obtained by Example 55 (Process 2) was performed similarly to that in Comparative Example X (Process 3), and the obtained product was dissolved in 3 ml of DMF and neutralized with 7.8 µl of triethylamine under ice cooling. To the neutralized solution, 20 mg of Boc-Asp(OBzl)-OH, 27 mg of Bop reagent and 17.1 µl of triethylamine were added and the resultant mixture was stirred for 2 hr. The reaction mixture was treated similarly to that in Comparative Example TT (Process 1) to give 23 mg of the title compound.
TLC: Rf 0.84 (chloroform : methanol = 9:1)

### [Process 2] 1-Naphthoxyacetyl-Asp(OBzl)-(2S,3S)-AHPBA-Pro-NH-tBu

Deprotection of 23 mg of the compound obtained by the process 1 was performed similarly to that in Comparative Example X (Process 3), and the obtained product was dissolved in 3 ml of DMF and neutralized with 4.9 µl of triethylamine under ice cooling. To the neutralized solution, 9 mg of 1-naphthoxyacetic acid, 19 mg of Bop reagent, 11.8 µl of triethylamine and 7 mg of HOBt were added and the resultant mixture was stirred for 2 hr. The reaction mixture was evaporated under reduced pressure and the resultant residue was redissolved in ethyl acetate. The obtained solution was washed with 5% citric acid aqueous solution, 5% sodium hydrogencarbonate aqueous solution and saturated sodium chloride aqueous solution, successively, and dried over anhydrous sodium sulfate. The dried solution was evaporated under reduced pressure to give 24 mg of the title compound.
TLC: Rf 0.86 (chloroform : methanol = 9:1)

### [Process 3] 1-Naphthoxyacetyl-Asp(NHNH₂)-(2S,3S)-AHPBA-Pro-NH-tBu.AcOH

In 3 ml of methanol, 24 mg of the compound obtained by the process 2 was dissolved and 20 µl of hydrazine hydrate was added and the resultant reaction mixture was stirred for 15 hr. The reaction mixture was evaporated under reduced pressure and the residue was subjected to a silica gel column chromatography (chloroform : methanol = 40:1) to give 28 mg of the crude product. In methanol, 10 mg of the crude product was desolved, fractionated by a reverse-phase HPLC [0.1% acetic acid (aq)-acetonitrile], lyophilized to give 2.5 mg of the title compound.
TLC: Rf 0.44 (chloroform : methanol = 9:1)
Analytical HPLC: 21.98 min (For the condition, see: Example 35).
FAB-MS: 661 (M+1)

### Example 140: 1-Isoquinolinecarbonyl-Asn-(2S,3S)-AHPBA-Pro-NH-tBu.AcOH

Deprotection of 19 mg of the compound obtained by Example 80 (Process 2) was performed similarly to that in Comparative Example X (Process 3), and the obtained product was dissolved in 3 ml of DMF and neutralized with 4.7 µl of triethylamine under ice cooling. To the neutralized solution, 6 mg of 1-isoquinolinecarboxylic acid, 15 mg of Bop reagent and 9.4 µl of triethylamine were added and the resultant mixture was stirred for 2 hr. The reaction mixture was evaporated under reduced pressure and the resultant residue was re-dissolved in ethyl acetate. The obtained solution was washed with 5% sodium hydrogencarbonate aqueous solution and saturated sodium chloride aqueous solution, successively, and dried over anhydrous sodium sulfate. The dried solution was evaporated under reduced pressure and subjected to a silica gel column chromatography (chloroform : methanol = 40:1) to give 19 mg of the crude compound. In methanol, 10 mg of the crude compound was dissolved, fractionated by a reversed-phase HPLC [0.1% acetic acid (aq)-acetonitrile system] and lyophilized to give 7.4 mg of the title compound.
TLC: Rf 0.38 (chloroform : methanol = 9:1)
Analytical HPLC: 19.33 min (For the condition, see: Example 35.)
FAB-MS: 603 (M+1)

### Example 141: 1-Naphthalenesulfonyl-Asn-(2S,3S)-AHPBA Pro-NH-tBu

Deprotection of 21 mg of the compound obtained by Example 80 (Process 2) was performed similarly tp that in Comparative Example X (Process 3), and the obtained product was dissolved in 3 ml of DMF and neutralized with 5.2 µl of triethylamine under ice cooling. To the neutralized solution, 9 mg of 1-naphthalenesulfonyl chloride and 5.7 µl of triethylamine were added and the resultant mixture was stirred for 2 hr. The reaction mixture was treated similarly to that in Comparative Example TT (Process 2) to give 6.7 mg of the title compound.
TLC: Rf 0.42 (chloroform : methanol = 9:1)
Analytical HPLC: 22.40 min (For the condition, see: Example 35.)
FAB-MS: 652(M+1)

### Example 142: 1-Naphthoxyacetyl-Msa-(2S,3S)-AHPBA-Thz-NH-tAmyl

### [Process 1] Boc-Thz-NH-tAmyl

In a methylene chloride solution containing 200 mg of Boc-Thz-OH, 100 µl of tert-amylamine and 196 mg of EDC hydrochloride were added and the resultant mixture was stirred for 14 hr. The reaction mixture was treated similarly to that in Comparative Example X (Process 2) to give 167 mg of the title compound.
TLC: Rf 0.76 (chloroform : methanol = 20:1)

### [Process 2] Boc-(2S,3S)-AHPBA-Thz-NH-tAmyl

Deprotection of 63 mg of the compound obtained by the process 1 was performed similarly to that in Comparative Example X (Process 3), and the obtained product was dissolved in 3 ml of DMF and neutralized with 29 µl of triethylamine under ice cooling. To the neutralized solution, 100 mg of Boc-(2S,3S)-AHPBA-OH.DCHA, 93 mg of Bop reagent, and 29 µl of triethylamine were added and the resultant mixture was stirred for 2 hr. The reaction mixture was treated similarly to that in Comparative Example TT (Process 1) to give 51 mg of the title compound.
TLC: Rf 0.71 (chloroform : methanol = 9:1)

### [Process 3] Boc-Msa-(2S,3S)-AHPBA-Thz-NH-tAmyl

Deprotection of 51 mg of the compound obtained by the process 2 was performed similarly to that in Comparative Example X (Process 3), and the obtained product was dissolved in 3 ml of DMF and neutralized with 14.8 µl of triethylamine under ice cooling. To the neutralized solution, 29 mg of Boc-Msa-OH, 47 mg of Bop reagent, and 29.6 µl of triethylamine were added and the resultant mixture was stirred for 2 hr. The reaction mixture was treated similarly to that in Comparative Example TT (Process 1) to give 47 mg of the title compound.
TLC: Rf 0.64 (chloroform : methanol = 9:1)

### [Process 4] 1-Naphthoxyacetyl-Msa-(2S,3S)-AHPBA-Thz-NH-tAmyl

Deprotection of 47 mg of the compound obtained by the process 3 was performed similarly to that in Comparative Example X (Process 3), and the obtained product was dissolved in 3 ml of DMF and neutralized with 10.4 µl of triethylamine under ice cooling. To the neutralized solution, 15 mg of 1-naphthoxy acetic acid, 33 mg of Bop reagent, and 20.7 µl of triethylamine were added and the resultant mixture was stirred for 2 hr. The reaction mixture was treated similarly to that in Comparative Example TT (Process 2) to give 6.7 mg of the title compound.
TLC: Rf 0.39 (chloroform : methanol = 9:1)
Analytical HPLC: 28.66 min (For the condition, see: Example 35.)
FAB-MS: 713 (M+1)

### Example 143: 2-Biphenylcarbonyl-Msa-(2S,3S)-AHPBA-Thz-NH-tBu

Deprotection of 146 mg of the compound obtained by Example 100 (Process 1) was performed similarly to that in Comparative Example X (Process 3), and the obtained product was dissolved in 3 ml of DMF and neutralized with 33 µl of triethylamine under ice cooling. To the neutralized solution, 47 mg of 2-biphenylcarboxylic acid, 105 mg of Bop reagent, and 65.9 µl of triethylamine were added and the resultant mixture was stirred for 2 hr. The reaction mixture was treated similarly to that in Comparative Example TT (Process 1) to give 102 mg of the crude compound. The crude compound was dissolved in methanol and subjected to a reversed-phase HPLC (water-acetonitrile system), fractionated, purified and lyophilized to give the title compound.
TLC: Rf 0.64 (chloroforms : methanol = 9:1)
Analytical HPLC: 23.30 min (For the condition, see: Example 35.)
FAB-MS: 696 (M+1)

### Example 144: 1-Naphthoxyacetyl-Msa-(2S,3S)-AHPBA-3Dic-NH-tBu

### [Process 1] Boc-(DL)-3Dic-OH

In an ethanol solution containing 2.0 g of N-(tert-butoxycarbonyl)-DL-1,2,3,4-tetrahydroisoquinoline-3-carboxylic acid, 5% Rh/Al₂O₃ was added ; the resultant mixture was stirred for 3 days in a hydrogen gas atmosphere at 4.4 x 10⁵ Pa (4.5 kg/cm²), then filtered, and the filtrate was evaporated under reduced pressure. The resultant residue was crystallized by the treatment with ether-hexane to give 1.5 g of the title compound.
TLC: Rf 0.56 (chloroform : methanol:acetic acid = 9:1:0.5)

### [Process 2] Boc-(DL)-3Dic-NH-tBu

In a dichloromethane solution containing 0.2 g of the protected amino acid obtained by the process 1, 60 mg of tert-butylamine and 0.12 g of HOBt and 0.16 g of EDC hydrochloride were added under ice-cooling and the resultant mixture was stirred overnight at room temperature. The reaction mixture was evaporated under reduced pressure and the resultant residue was re-dissolved in ethyl acetate. The obtained solution was washed with 5% sodium hydrogencarbonate aqueous solution, 10% citric acid aqueous solution and saturated sodium chloride aqueous solution, successively, and dried over anhydrous sodium sulfate. The dried solution was evaporated under reduced pressure and crystallized from ether-hexane to give 0.21 g of the title compound.
TLC: Rf 0.88 (chloroform : methanol:acetic acid = 9:1:0.5)

### [Process 3] Boc-(2S,3S)-AHPBA-(DL)-3Dic-NH-tBu

Deprotection of 210 mg of the compound obtained by the process 2 was performed similarly to that in Comparative Example X (Process 3), and the obtained product was dissolved in dichloromethane and neutralized with 90 µl of triethylamine under ice cooling. To the neutralized solution, 0.18 g of Boc-(2S, 3S)-AHPBA-OH and 0.33 g of Bop reagent were added and the resultant mixture was stirred overnight at room temperature. The reaction mixture was treated similarly to that in the process 2 to give 150 mg of the title compound.
TLC: Rf 0.63 (chloroform : methanol = 40:1)

### [Process 4] Boc-Msa-(2S,3S)-AHPBA-(DL)-3Dic-NH-tBu

Deprotection of 150 mg of the compound obtained by the process 3 was performed similarly to that in Comparative Example X (Process 3), and the obtained product was dissolved in DMF and neutralized with 40 µl of triethylamine under ice cooling. To the neutralized solution, 72 mg of Boc-Msa-OH and 146 mg of Bop reagent were added and the resultant mixture was stirred overnight at room temperature. The reaction mixture was treated similarly to that in the process 2 to give 0.11 g of the title compound.
TLC: Rf 0.76 (chloroform : methanol:acetic acid = 9:1:0.5)

### [Process 5] 1-Naphthoxyacetyl-Msa-(2S,3S)-AHPBA-3Dic-NH-tBu

Deprotection of 110 mg of the compound obtained by the process 4 was performed similarly to that in Example 125 (Process 2), and the obtained product was dissolved in DMF and neutralized with 20 µl of triethylamine under ice cooling. To the neutralized solution, 30 mg of 1-naphthoxyacetic acid and 60 mg of Bop reagent were added and the resultant mixture was stirred overnight at room temperature. The reaction mixture was treated similarly to that in Example 122 to give 8 mg of the title compound.
FAB-MS: 749 (M+1)

### Example 145: 1-Naphthoxyacetyl-Msa-(2S,3S)-AHPBA-1Dic-NH-tBu

### [Process 1] Boc-(DL)-1Dic-OH

In an acetic acid solution containing 2.0 g of isoquinnline-1-carboxylic acid, 5% Rh/Al₂O₃ was added and the resultant mixture was stirred for 3 days in a hydrogen gas atmosphere (4.5 kg/cm²). The reaction mixture was filtered and the filtrate was evaporated under reduced pressure. The resultant residue was tert-butoxycarbonylated, dissolved in ethanol, and the resultant mixture was stirred with 5% Rh/Al₂O₃ for 3 days in a hydrogen gas atmosphere at 4.4 x 10⁵Pa (4.5 kg/cm²) and then treated similarly to that in Example 144 (Process 1) to give 0.8 g of the title compound.
TLC: Rf 0.71 (chloroform : methanol:acetic acid = 9:1:0.5)

### [Process 2] Boc-(DL)-1Dic-NH-tBu

In a dichloromethane solution containing 0.29 g of the protected amino acid obtained by the process 1, 90 mg of tert-butylamine and 0.17 g of HOBt and 0.23 g of EDC hydrochloride were added under ice cooling and the resultant mixture was stirred overnight at room temperature. The reaction mixture was treated similarly to that in Example 144 (Process 2) to give 0.13 g of the title compound.
TLC: Rf 0.76 (chloroform : methanol:acetic acid = 9:1:0.5)

### [Process 3] Boc-(2S,3S)-AHPBA-(DL)-1Dic-NH-tBu

Deprotection of 130 mg of the compound obtained by the process 2 was performed similarly to that in Comparative Example X (Process 3), and the obtained product was dissolved in dichloromethane and neutralized with 50 µl of triethylamine under ice cooling. To the neutralized solution, 0.1 g of Boc-(2S, 3S)-AHPBA-OH and 0.18 g of Bop reagent were added and the resultant mixture was stirred overnight at room temperature. The reaction mixture was treated similarly to that in Example 144 (Process 2)to give 90 mg of the title compound.
TLC: Rf 0.68 (chloroform : methanol = 40:1)

### [Process 4] Boc-Msa-(2S,3S)-AHPBA-(DL)-1Dic-NH-tBu

Deprotection of 90 mg of the compound obtained by the process 3 was performed similarly to that in Comparative Example X (Process 3), and the obtained product was dissolved in DMF and neutralized with 20 µl of triethylamine under ice cooling. To the neutralized solution, 42 mg of Boc-Msa-OH and 80 mg of Bop reagent were added and the resultant mixture was stirred overnight at room temperature. The reaction mixture was treated similarly to that in Example 144 (Process 2) to give 90 mg of the title compound.
TLC: Rf 0.83 (chloroform : methanol:acetic acid = 9:1:0.5)

### [Process 5] 1-Naphthoxyacetyl-Msa-(2S,3S)-AHPBA-1Dic-NH-tBu

Deprotection of 90 mg of the compound obtained by the process 4 was performed similarly to that in Example 125 (Process 2), and the obtained product was dissolved in DMF and neutralized with 35 µl of triethylamine under ice cooling. To the neutralized solution, 30 mg of 1-naphthoxyacetic acid and 60 mg of Bop reagent were added and the resultant mixture was stirred overnight at room temperature. The reaction mixture was treated similarly to that in Example 122 to give 3 mg of the title compound.
FAB-MS: 749 (M+1)

### Example 146: 1-Naphthoxyacetyl-Msa-(2S,3S)-AHPBA-Oic-NH-tBu

### [Process 1] Boc-Oic-OH

In an acetic acid solution containing 2.0 g of L-indoline-2-carboxylic acid, 5% Rh/Al₂O₃ was added and the resultant mixture was stirred for 3 days in a hydrogen gas atmosphere at 4.4 x 10⁵Pa (4.5 kg/cm²), then filtered, and the filtrate was evaporated under reduced pressure. The resultant residue was tert-butoxycarbonylated to give 0.6 g of the title compound.

### [Process 2] Boc-Oic-NH-tBu

In a dichloromethane solution containing the protected amino acid obtained by the process 1, tert-butylamine, HOBt and EDC hydrochloride were added under ice cooling and the resultant mixture was stirred overnight at room temperature. The reaction mixture was treated similarly to that in Example 144 (Process 2) to give the title compound.
TLC: Rf 0.89 (chloroform : methanol = 9:1)

### [Process 3] Boc-(2S,3S)-AHPBA-Oic-NH-tBu

Deprotection of 63 mg of the compound obtained by the process 2 was performed similarly to that in Comparative Example X (Process 3), and the obtained product was dissolved in dichloromethane and neutralized with 20 µl of triethylamine under ice cooling. To the neutralized solution, 86 mg of Boc-(2S, 3S)-AHPBA-OH and 93 mg of Bop reagent were added and the resultant mixture was stirred overnight at room temperature. The reaction mixture was treated similarly to that in Example 144 (Process 2) to give 10 mg of the title compound.
TLC: Rf 0.35 (hexane:ether = 2:1)

### [Process 4] Boc-Msa-(2S,3S)-AHPBA-Oic-NH-tBu

Deprotection of 10 mg of the compound obtained by the process 3 was performed similarly to that in Comparative Example X (Process 3), and the obtained product was dissolved in DMF and neutralized with 3 µl of triethylamine under ice cooling. To the neutralized solution, 10 mg of Boc-Msa-OH and 12 mg of Bop reagent were added under ice cooling and the resultant mixture was stirred overnight at room temperature. The reaction mixture was treated similarly to that in Example 46 to give 20 mg of the title compound.
TLC: Rf 0.76 (chloroform : methanol = 9:1)

### [Process 5] 1-Naphthoxyacetyl-Msa-(2S,3S)-AHPBA-Oic-NH-tBu

Deprotection of 20 mg of the compound obtained by the process 4 was performed similarly to that in Example 125 (Process 2), and the obtained product was dissolved in DMF and neutralized with 3 µl of triethylamine under ice cooling. To the neutralized solution, 4 mg of 1-naphthoxyacetic acid and 11 mg of Bop reagent were added and the resultant mixture was stirred overnight at room temperature. The reaction mixture was treated similarly to that in Example 122 to give 1 mg of the title compound.
Analytical HPLC: 27.47 min (For the condition, see: Example 35.)
FAB-MS: 735 (M+1)

### Example 147: 1-Naphthoxyacetyl-Msa-(2S,3S)-AHPBA-Pro-NH-Ph(o-OH)

### [Process 1] Boc-Pro-NH-Ph(o-OH)

In a dichloromethane solution containing 1.07 g of Boc-Pro-OH, 0.55 g of o-aminophenol, 0.84 g of HOBt and 1.15 g of EDC hydrochloride were added under ice cooling and the resultant mixture was stirred overnight at room temperature. The reaction mixture was treated similarly to that in Example 144 (Process 2) to give 0.25 g of the title compound.
TLC: Rf 0.60 (chloroform : methanol = 9:1)

### [Process 2] Boc-(2S,3S)-AHPBA-Pro-NH-Ph(o-OH)

Deprotection of 100 mg of the compound obtained by the process 1 was performed similarly to that in Comparative Example X (Process 3), and the obtained product was dissolved in dichloromethane and neutralized with 50 µl of triethylamine under ice cooling. To the neutralized solution, 155 mg g of Boc-(2S,3S)-AHPBA-OH and 175 mg of Bop reagent were added and the resultant mixture was stirred overnight at room temperature. The reaction mixture was treated similarly to that in Example 55 (Process 3) to give 70 mg of the title compound.
TLC: Rf 0.51 (chloroform : methanol = 9:1)

### [Process 3] Boc-Msa-(2S,3S)-AHPBA-Pro-NH-Ph(o-OH)

Deprotection of 50 mg of the compound obtained by the process 2 was performed similarly to that in Comparative Example X (Process 3), and the obtained product was dissolved in DMF and neutralized with 14 µl of triethylamine under ice cooling. To the neutralized solution, 27 mg of Boc-Msa-OH and 53 mg of Bop reagent were added and the resultant mixture was stirred overnight at room temperature. The reaction mixture was treated similarly to that in Example 93 (Process 3) to give 20 mg of the title compound.
TLC: Rf 0.53 (chloroform : methanol = 9:1)

### [Process 4] 1-Naphthoxyacetyl-Msa-(2S,3S)-AHPBA-Pro-NH-Ph(o-OH)

Deprotection of 20 mg of the compound obtained by the process 3 was performed similarly to that in Example 125 (Process 2), and the obtained product was dissolved in DMF and neutralized with 4.2 µl of triethylamine under ice cooling. To the neutralized solution, 6 mg of 1-naphthoxyacetic acid and 18 mg of Bop reagent were added and the resultant mixture was stirred overnight at room temperature. The reaction mixture was treated similarly to that in Example 122 to give 6 mg of the title compound.
Analytical HPLC: 25.83 min (For the condition, see: Example 35.)
FAB-MS: 717 (M+1)

### Example 148: 1-Naphthoxyacetyl-Msa-(2S,3S)-AHPBA-Pro-NH-Ph(m-OH)

### [Process 1] Boc-Pro-NH-Ph(m-OH)

From 1.07 g of Boc-Pro-OH, and 0.55 g of m-aminophenol, 0.13 g of the title compound was synthesized by the similar method to Example 147 (Process 1).
TLC: Rf 0.54 (chloroform : methanol = 9:1)

### [Process 2] Boc-(2S,3S)-AHPBA-Pro-NH-Ph(m-OH)

From 0.13 g of the protected amino acid obtained by the process 1, 114 mg of the title compound was systhesized by the similar method to Example 147 (Process 2).
TLC: Rf 0.38 (chloroform : methanol = 9:1)

### [Process 3] Boc-Msa-(2S,3S)-AHPBA-Pro-NH-Ph(m-OH)

From 50 mg of the protected peptide obtained by the process 2, 60 mg of the title compound was synthesized by the similar method to Example 147 (Process 3)
TLC: Rf 0.45 (chloroform : methanol = 9:1)

### [Process 4] 1-Naphthoxyacetyl-Msa-(2S,3S)-AHPBA-Pro-NH-Ph(m-OH)

From 60 mg of the protected peptide obtained by the process 3, 5 mg of the title compound was synthesized by the similar method to Example 147 (Process 3).
Analytical HPLC: 24.23 min (For the condition, see: Example 35.)
FAB-MS: 717 (M+1)

### Example 149: 1-Naphthoxyacetyl-Msa-(2S,3S)-AHPBA-Pro-NH-Ph(p-OH)

### [Process 1] Boc-Pro-NH-Ph(p-OH)

From 1.07 g of Boc-Pro-OH and 0.55 g of p-aminophenol, 0.13 g of the title compound was synthesized by the similar method to Example 147 (Process 1)
TLC: Rf 0.72 (chloroform : methanol:H₂O = 8:3:1, lower layer)

### [Process 2] Boc-(2S,3S)-AHPBA-Pro-NH-Ph(p-OH)

From 0.1 g of the protected amino acid obtained by the process 1, 151 mg of the title compound was synthesized by the similar method to Example 147 (Process 2)
TLC: Rf 0.50 (chloroform : methanol = 9:1)

### [Process 3] Boc-Msa-(2S,3S)-AHPBA-Pro-NH-Ph(P-OH)

From 50 mg of the protected peptide obtained by the process 2, 10 mg of the title compound was synthesized by the similar method to Example 147 (Process 3)
TLC: Rf 0.45 (chloroform : methanol = 9:1)

### [Process 4] 1-Naphthoxyacetyl-Msa-(2S,3S)-AHPBA-Pro-NH-Ph(p-OH)

From 10 mg of the protected peptide obtained by the process 3, 1 mg of the title compound was synthesized by the similar method to Example 147 (Process 4).
Analytical HPLC: 24.83 min (For the condition, see: Example 35.)
FAB-MS: 717 (M+1)

### Example 150: 1-Naphthoxyacetyl-Msa-(2S,3S)-AHPBA-Hyp-NH-tBu

### [Process 1] Boc-Hyp-NH-tBu

In a dichloromethane solution containing 5.1 g of N-Boc-hydroxyproline (Boc-Hyp-OH), 0.35 g of tert-butylamine, 7.4 g of HOBt and 6.0 g of EDC hydrochloride were added under ice cooling and the resultant mixture was stirred overnight at room temperature. The reaction mixture was treated similarly to that in Example 144 (Process 2) to give 2.59 g of the title compound.
TLC: Rf 0.67 (chloroform : methanol = 9:1)

### [Process 2] Boc-(2S,3S)-AHPBA-Hyp-NH-tBu

Deprotection of 170 mg of the compound obtained by the process 1 was performed similarly to that in Example 125 (Process 2), and the obtained product was dissolved in dichloromethane and neutralized with 50 µl of triethylamine under ice cooling. To the neutralized solution, 138 mg of Boc-(2S,3S)-AHPBA-OH and 200 mg of Bop reagent were added and the resultant mixture was stirred overnight at room temperature. The reaction mixture was treated similarly to that in Example 144 (Process 2) to give 0.13 g of the title compound.
TLC: Rf 0.41 (chloroform : methanol = 9:1)

### [Process 3] Boc-Msa-(2S,3S)-AHPBA-Hyp-NH-tBu

Deprotection of 100 mg of the compound obtained by the process 2 was performed similarly to that in Example 125 (Process 2), and the obtained product was dissolved in DMF and neutralized with 30 µl of triethylamine under ice cooling. To the neutralized solution, 59 mg of Boc-Msa-OH and 116 mg of Bop reagent were added and the resultant mixture was stirred overnight at room temperature. The reaction mixture was treated similarly to that in Example 93 (Process 3) to give 33 mg of the title compound.
TLC: Rf 0.18 (chloroform : methanol = 9:1)

### [Process 4] 1-Naphthoxyacetyl-Msa-(2S,3S)-AHPBA-Hyp-NH-tBu

Deprotection of 20 mg of the compound obtained by the process 3 was performed similarly to that in Example 125 (Process 2), and the obtained product was dissolved in DMF and neutralized with 4 µl of triethylamine under ice cooling. To the neutralized solution, 6 mg of 1-naphthoxyacetic acid and 18 mg of Bop reagent were added and the resultant mixture was stirred overnight at room temperature. The reaction mixture was treated similarly to that in Example 122 to give 1 mg of the title compound.
Analytical HPLC: 26.27 min (For the condition, see: Example 35.)
FAB-MS: 697 (M+1)

### Example 151 1-Naphthoxyacetyl-Msa-(2S,3S)-AHPBA-Hyp(Me)-NH-tBu

### [Process 1] Boc-Hyp(Me)-NH-tBu

In a THF solution containing 0.6 g of the protected amino acid obtained by the Example 150, (Process 1), 94 mg of sodium hydride (60% in oil) was added under ice cooling and the resultant mixture was stirred for 1 hr at room temperature. Further, 2.0 ml of methyl iodide was added and the obtained reaction mixture was stirred for 3 hr at room temperature. To the reaction mixture, 5% potassium hydrogensulfate aqueous solution was added and extracted with ethyl acetate. The organic layer was washed with water and saturated sodium chloride aqueous solution, successively and dried over anhydrous sodium sulfate. The dried solution was evaporated under reduced pressure and treated with hexane to crystallize 0.4 g of the title compound.
TLC: Rf 0.74 (chloroform : methanol = 9:1)

### [Process 2] Boc-(2S,3S)-AHPBA-Hyp(Me)-NH-tBu

From 0.1 g of the protected amino acid obtained by the process 1, 0.14 g of the title compound was synthesized by the similar method to Example 150 (Process 2).
TLC: Rf 0.62 (chloroform : methanol = 9:1)

### [Process 3] Boc-Msa-(2S,3S)-AHPBA-Hyp(Me)-NH-tBu

From 0.14 g of the protected peptide obtained by the process 2, 0.14 g of the title compound was synthesized by the similar method to Example 150 (Process 3).
TLC: Rf 0.76 (chloroform : methanol = 9:1)

### [Process 4] 1-Naphthoxyacetyl-Msa-(2S,3S)-AHPBA -Hyp(Me)-NH-tBu

From 20 mg of the protected peptide obtained by the process 3, 11 mg of the title compound was synthesized by the similar method to Example 150 (Process 4).
Analytical HPLC: 26.43 min (For the condition, see: Example 35.)
FAB-MS: 711 (M+1)

### Example 152: 1-Naphthoxyacetyl-Msa-(2S,3S)-AHPBA-Hyp(Et)-NH-tBu

### [Process 1] Boc-Hyp(Et)-NH-tBu

In a THF solution containing 1.0 g of the protected amino acid obtained by the Example 150 (Process 1), 160 mg of sodium hydride (60% in oil) was added under ice cooling and the resultant mixture was stirred for 1 hr at room temperature. Further, 1 ml of ethyl bromide was added and stirred for 3 hr at room temperature. the obtained reaction mixture was treated similarly to that in Example 151 (Process 1) to give 0.8 g of the title compound.
TLC: Rf 0.78 (chloroform : methanol = 9:1)

### [Process 2] Boc-(2S,3S)-AHPBA-Hyp(Et)-NH-tBu

From 0.8 g of the protected amino acid obtained by the process 1, 80 mg of the title compound was synthesized by the similar method to Example 150 (Process 2).
TLC: Rf 0.70 (chloroform : methanol = 9:1)

### [Process 3] Boc-Msa-(2S,3S)-AHPBA-Hyp(Et)-NH-tBu

From 80 mg of the protected peptide obtained by the process 2, 87 mg of the title compound was synthesized by the similar method to Example 150 (Process 3).
TLC: Rf 0.76 (chloroform : methanol = 9:1)

### [Process 4] 1-Naphthoxyacetyl-Msa-(2S,3S)-AHPBA-Hyp(Et)-NH-tBu

From 20 mg of the protected peptide obtained by the process 3, 2 mg of the title compound was synthesized by the similar method to Example 150 (Process 4).
Analytical HPLC: 27.42 min (For the condition. see: Example 35.)
FAB-MS: 725 (M+1)

### Example 153: 1-Naphthoxyacetyl-Msa-(2S,3S)-AHPBA-Hyp(Allyl)-NH-tBu

### [Process 1] Boc-Hyp(Allyl)-NH-tBu

In a THF solution containing 1.0 g of the protected amino acid obtained by the Example 150 (Process 1), 160 mg of sodium hydride (60% in oil) was added under ice cooling and the resultant mixture was stirred for 1 hr at room temperature. Further, 0.5 ml of allyl bromide was added and stirred for 3 hr at room temperature. the obtained reaction mixture was treated similarly to that in Example 151 (Process 1) to give 0.95 g of the title compound.
TLC: Rf 0.87 (chloroform : methanol = 9:1)

### [Process 2] Boc-(2S,3S)-AHPBA-Hyp(Allyl)-NH-tBu

From 0.13 g of the protected amino acid obtained by the process 1, 156 mg of the title compound was synthesized by the similar method to Example 150 (Process 2).
TLC: Rf 0.82 (chloroform : methanol = 9:1)

### [Process 3] Boc-Msa-(2S,3S)-AHPBA-Hyp(Allyl)-NH-tBu

From 50 mg of the protected peptide obtained by the process 2, 41 mg of the title compound was synthesized by the similar method to Example 150 (Process 3).
TLC: Rf 0.59 (chloroform : methanol = 9:1)

### [Process 4] 1-Naphthoxyacetyl-Msa-(2S,3S)-AHPBA-Hyp(Allyl)-NH-tBu

From 21 mg of the protected peptide obtained by the process 3, 4 mg of the title compound was synthesized by the similar method to Example 150 (Process 4).
Analytical HPLC: 28.27 min (For the condition, see: Example 35.)
FAB-MS: 737 (M+1)

### Comparative Example WW: 1-Naphthoxyacetyl-Mtv-(2S,3S)-AHPBA-Thz-NH-tBu

### [Process 1] N-Boc-β-(methylthio)valine [Boc-Mtv-OH]

In 70 ml of a mixture of 1N-NaOH-ethanol (1:1) solution containing 1.04 g of L-penicillamine, 0.48 ml of methyl iodide was added under ice cooling and the obtained reaction mixture was stirred overnight at room temperature. The reaction mixture was evaporated under reduced pressure and the resultant residue was redissolved in ethyl acetate, washed with 5% sodium hydrogensulfite aqueous solution and saturated sodium chloride aqueous solution, successively, and dried over anhydrous sodium sulfate. The dried solution was evaporated under reduced pressure. The obtained residue was tert-butoxycarbonylated and 2.83 g of the title compound was obtained as its DCHA salt.
TLC: Rf 0.68 (chloroform : methanol:H₂O = 8:3:1, lower layer)

### [Process 2] Boc-Mtv-(2S,3S)-AHPBA-Thz-NH-tBu

Deprotection of 50 mg of the compound obtained by Example 82 (Process 2) was performed similarly to that in Example 125 (Process 2), and the obtained product was dissolved in DMF and neutralized with 14 µl of triethylamine under ice cooling. To the neutralized solution, 45 mg of the protected amino acid DCHA salt obtained by the process 1 and 53 mg of Bop reagent were added and the resultant mixture was stirred overnight at room temperature. The reaction mixture was treated similarly to that in Example 144 (Process 2) to give 30 mg of the title compound.
TLC: Rf 0.86 (chloroform : methanol = 9:1)

### [Process 3] 1-Naphthoxyacetyl-Mtv-AHPBA-Thz-NH-tBu

Deprotection of 30 mg of the compound obtained by the process 2 was performed similarly to that in Example 125 (Process 2), and the obtained product was dissolved in DMF and neutralized with 7 µl of triethylamine under ice cooling. To the neutralized solution, 10 mg of 1-naphthoxyacetic acid and 26 mg of Bop reagent were added and the resultant mixture was stirred overnight at room temperature. The reaction mixture was treated similarly to that in Example 122 to give l mg of the title compound.
Analytical HPLC: 33.23 min (For the condition, see: Example 35.)
FAB-MS: 695 (M+1)

### Comparative Example XX: 1-Naphthoxyacetyl-Msv-(2S,3S)-AHPBA-Thz-NH-tBu

### [Process 1] N-Boc-β-(methanesulfonyl)valine [Boc-Msv-OH]

In a chloroform solution containing 0.44 g of the protected amino acid obtained by the Comparative Example WW, (Process 1), 0.52 g of m-chloroperbenzoic acid was added under ice cooling and the reaction mixture was stirred for 2 hr at room temperature. The reaction mixture was mixed with methyl sulfide and filtered. DCHA was added to the filtrate, and the solution was evaporated under reduced pressure and crystallized by the addition of ether-hexane. The obtained solid was recrystallized from ether-hexane to give 0.23 g of the title compound as its DCHA salt.
TLC: Rf 0.33 (chloroform : methanol = 9:1)

### [Process 2] Boc-Msv-(2S,3S)-AHPBA-Thz-NH-tBu

Deprotection of 50 mg of the compound obtained by Example 82 (Process 2) was performed similarly to that in Example 125 (Process 2), and the obtained product was dissolved in DMF and neutralized with 14 µl of triethylamine, 30 mg of the protected amino acid obtained by the process 1 and 53 mg of Bop reagent were added and the resultant mixture was stirred overnight at room temperature. The reaction mixture was treated similarly to that in Example 144 (Process 2) to give 20 mg of the title compound.
TLC: Rf 0.50 (chloroform : methanol = 9:1)

### [Process 3] 1-Naphthoxyacetyl-Msv-(2S,3S)-AHPBA-Thz-NH-tBu

Deprotection of 20 mg of the compound obtained by the process 2 was performed similarly to that in Example 125 (Process 2), and the obtained product was dissolved in DMF and neutralized with 4 µl of triethylamine under ice cooling. To the neutralized solution, 6 mg of 1-naphthoxyacetic acid and 18 mg of Bop reagent were added under ice cooling and the resultant mixture was stirred overnight at room temperature. The reaction mixture was treated similarly to that in Example 122 to give 0.3 mg of the title compound.
Analytical HPLC: 28.96 min (For the condition, see: Example 35.)
FAB-MS: 727 (M+1)

### Example 156: 1-Naphthoxyacetyl-Msa-(2S,3S)-AHPBA-Thz-NH-CH(C₆H₅)CH₂ OH

### [Process 1] N-Boc-Phenylglycinol [Boc-Phgol]

In a THF solution containing 0.30 g of lithium borohydride, 1.30 g of N-(tert-butoxycarbonyl)phenylglycine methyl ester was added under ice cooling. To the resultant solution, methanol was added dropwise under ice cooling and stirred overnight at room temperature. Water was added to the reaction mixture, extracted with ethyl acetate. The organic layer was washed with water and saturated sodium chloride aqueous solution, successively, and dried over anhydrous sodium sulfate. The dried solution was evaporated under reduced pressure and the obtained residue was subjected to a silica gel column chromatography (hexane:ether = 1:1) to give 0.41 g of the title compound.
TLC: Rf 0.11 (hexane:ether = 1:1)

### [Process 2] Boc-Thz-Phgol

Deprotection of 240 mg of the compound obtained by the process 1 was performed similarly to that in Example 125 (Process 2), and the obtained product was dissolved in dichloromethane and 0.14 ml of triethylamine, 0.23 g of Boc-Thz-OH, 0.17 g of HOBt and 0.23 g of EDC hydrochloride were added under ice cooling and the resultant mixture was stirred overnight at room temperature. The reaction mixture was treated similarly to that in Example 144 (Process 2) to give 0.15 g of the title compound.
TLC: Rf 0.53 (chloroform : methanol = 9:1)

### [Process 3] Boc-(2S,3S)-AHPBA-Thz-Phgol

Deprotection of 35 mg of the compound obtained by the process 2 was performed similarly to that in Example 125 (Process 2), and the obtained product was dissolved in DMF and neutralized with 14 µl of triethylamine under ice cooling. To the neutralized solution, 30 mg of Boc-(2S,3S)-AHPBA-OH, 53 mg of Bop reagent were added and the resultant mixture was stirred overnight at room temperature. The reaction mixture was treated similarly to that in Example 144 (Process 2) to give 40 mg of the title compound.
TLC: Rf 0.48 (chloroform : methanol = 9:1)

### [Process 4] Boc-Msa-(2S,3S)-AHPBA-Thz-Phgol

Deprotection of 40 mg of the compound obtained by the process 3 was performed similarly to that in Example 125 (Process 2), and the obtained product was dissolved in DMF and neutralized with 11 µl of triethylamine under ice cooling. To the neutralized solution, 20 mg of Boc-Msa-OH, 40 mg of Bop reagent were added and the resultant mixture was stirred overnight at room temperature. The reaction mixture was treated similarly to that in Example 93 (Process 3) to give 10 mg of the title compound.
TLC: Rf 0.43 (chloroform : methanol = 9:1)

### [Process 5] 1-Naphthoxyacetyl-Msa-(2S,3S)-AHPBA-Thz-NH-CH(C₆H₅)CH₂OH

Deprotection of 10 mg of the compound obtained by the process 4 was performed similarly to that in Example 125 (Process 2), and the obtained product was dissolved in DMF and neutralized with 1.5 µl of triethylamine under ice cooling. To the neutralized solution, 3 mg of 1-naphthoxyacetic acid and 12 mg of Bop reagent were added and the resultant mixture was stirred overnight at room temperature. The reaction mixture was treated similarly to that in Example 122 to give 0.3 mg of the title compound.
Analytical HPLC: 26.03 min (For the condition, see: Example 35.)
FAB-MS: 763 (M+1)

### Example 157: 1-Naphthoxyacetyl-Msa-(2S,3S)-AHPBA-Thz-Phg-NH₂

### [Process 1] Boc-Phg-NH₂

In a dichloromethane solution containing 4.3 g of N-(tert-butoxycarbonyl)phenylglycine, 5 ml of ammonia water, 1.5 g of HOBt and 2.3 g of EDC hydrochloride were added under ice cooling and the resultant mixture was stirred overnight at room temperature. The reaction mixture was treated similarly to that in Example 144 (Process 2) to give 0.42 g of the title compound.
TLC: Rf 0.73 (chloroform : methanol = 5:1)

### [Process 2] Boc-Thz-Phg-NH₂

Deprotection of 250 mg of the compound obtained by the process 1 was performed similarly to that in Comparative Example X (Process 3), and the obtained product was dissolved in dichloromethane and 0.14 ml of triethylamine, 0.23 g of Boc-Thz-OH, 0.17 g of HOBt and 0.23 g of EDC hydrochloride were added and the resultant mixture was stirred overnight at room temperature. The reaction mixture was treated similarly to that in Example 144 (Process 2) to give 0.316 g of the title compound.
TLC: Rf 0.61 (chloroform : methanol = 9:1)

### [Process 3] Boc-(2S,3S)-AHPBA-Thz-Phg-NH₂

Deprotection of 37 mg of the compound obtained by the process 2 was performed similarly to that in Comparative Example X (Process 3), and the obtained product was dissolved in DMF and neutralized with 14 µl of triethylamine under ice cooling. To the neutralized solution, 30 mg of Boc-(2S, 3S)-AHPBA-OH, 53 mg of Bop reagent were added and the resultant mixture was stirred overnight at room temperature. The reaction mixture was treated similarly to that in Example 144 (Process 2) to give 30 mg of the title compound.
TLC: Rf 0.58 (chloroform : methanol = 9:1)

### [Process 4] Boc-Msa-(2S,3S)-AHPBA-Thz-Phg-NH₂

Deprotection of 30 mg of the compound obtained by the process 3 was performed similarly to that in Example 125 (Process 2), and the obtained product was dissolved in DMF and neutralized with 8.4 µl of triethylamine under ice cooling. To the neutralized solution, 16 mg of Boc-Msa-OH, 32 mg of Bop reagent were added and the resultant mixture was stirred overnight at room temperature. The reaction mixture was treated similarly to that in Example 144 (Process 2) to give 30 mg of the title compound.
TLC: Rf 0.47 (chloroform : methanol = 9:1)

### [Process 5] 1-Naphthoxyacetyl-Msa-(2S,3S)-AHPBA-Thz-Phg-NH₂

Deprotection of 30 mg of the compound obtained by the process 4 was performed similarly to that in Example 125 (Process 2), and the obtained product was dissolved in DMF and neutralized with 6 µl of triethylamine under ice cooling. To the neutralized solution, 9 mg of 1-naphthoxyacetic acid and 23 mg of Bop reagent were added and the resultant mixture was stirred overnight at room temperature. The reaction mixture was treated similarly to that in Example 122 to give 2 mg of the title compound.
Analytical HPLC: 23.79 min (For the condition, see: Example 35.)
FAB-MS: 776 (M+1)

### Example 158: 1-Naphthoxyacetyl-Msa-(2S,3S)-AHPBA-Thz-NH-chex-ol

### [Process 1] Boc-Thz-NH-chex-ol

In a dichloromethane solution containing 0.23 g of Boc-Thz-OH, 0.12 g of trans-4-aminocyclohexanol, 0.17 g of HOBt and 0.23 g of EDC hydrochloride were added under ice cooling and the resultant mixture was stirred overnight at room temperature. The reaction mixture was treated similarly to that in Example 144 (Process 2) to give 0.12 g of the title compound.
TLC: Rf 0.38 (chloroform : methanol = 9:1)

### [Process 2] Boc-(2S,3S)-AHPBA-Thz-NH-chex-ol

Deprotection of 100 mg of the compound obtained by the process 1 was performed similarly to that in Comparative Example X (Process 3), and the obtained product was dissolved in DMF and neutralized with 56 µl of triethylamine. To the neutralized solution, 190 mg of Boc-(2S,3S)-AHPBA-OH and 221 mg of Bop reagent were added and the resultant mixture was stirred overnight at room temperature. The reaction mixture was treated similarly to that in Example 144 (Process 2) to give 120 mg of the title compound.
TLC: Rf 0.48 (chloroform : methanol = 9:1)

### [Process 3] Boc-Msa-(2S,3S)-AHPBA-Thz-NH-chex-ol

Deprotection of 50 mg of the compound obtained by the process 2 was performed similarly to that in Example 125 (Process 2), and the obtained product was dissolved in DMF and neutralized with 14 µl of triethylamine under ice cooling. To the neutralized solution, 27 mg of Boc-Msa-OH and 53 mg of Bop reagent were added and the resultant mixture was stirred overnight at room temperature. The reaction mixture was treated similarly to that in Example 93 (Process 3) to give 10 mg of the title compound.
TLC: Rf 0.56 (chloroform : methanol = 9:1)

### [Process 4] 1-Naphthoxyacetyl-Msa-(2S,3S)-AHPBA-Thz-NH-chex-OH

Deprotection of 10 mg of the compound obtained by the process 3 was performed similarly to that in Example 125 (Process 2), and the obtained product was dissolved in DMF and neutralized with 2 µl of triethylamine under ice cooling. To the neutralized solution, 3 mg of 1-naphthoxyacetic acid and 12 mg of Bop reagent were added and the resultant mixture was stirred overnight at room temperature. The reaction mixture was treated similarly to that in Example 122 to give 2 mg of the title compound.
Analytical HPLC: 25.58 min (For the condition, see: Example 35).
FAB-MS: 741 (M+1)

### Example 159: 1-Naphthoxyacetyl-Msa-(2S,3S)-AHPBA-Thz-Pip-O-CH₃

### [Process 1] Boc-Thz-(DL)-Pip-O-CH₃

In a dichloromethane solution containing 0.20 g of hydrochloride of methyl (DL)-pipecolinate, 0.17 ml of triethylamine, 0.28 g of Boc-Thz-OH, 0.20 g of HOBt and 0.28 g of EDC hydrochloride were added under ice cooling and the resultant mixture was stirred overnight at room temperature. The reaction mixture was treated similarly to that in Comparative Example VV (Process 2) to give 0.34 g of the title compound as oil.
TLC: Rf 0.83 (chloroform : methanol = 9:1)

### [Process 2] Boc-(2S,3S)-AHPBA-(DL)-Pip-O-CH₃

Deprotection of 100 mg of the compound obtained by the process 1 was performed similarly to that in Comparative Example X (Process 3), and the obtained product was dissolved in DMF and neutralized with 24 µl of triethylamine under ice cooling. To the neutralized solution, 50 mg of Boc-(2S, 3S)-AHPBA-OH and 88 mg of Bop reagent were added and the resultant mixture was stirred overnight at room temperature. The reaction mixture was treated similarly to that in Example 144 (Process 2) to give 40 mg of the title compound.
TLC: Rf 0.58 (chloroform : methanol = 9:1)

### [Process 3] Boc-Msa-(2S,3S)-AHPBA-Thz-(DL)-Pip-O-CH₃

Deprotection of 40 mg of the compound obtained by the process 2 was performed similarly to that in Example 125 (Process 2), and the obtained product was dissolved in DMF and neutralized with 11 µl of triethylamine under ice cooling. To the neutralized solution, 20 mg of Boc-Msa-OH and 40 mg of Bop reagent were added and the resultant mixture was stirred overnight at room temperature. The reaction mixture was treated similarly to that in Example 144 (Process 2) to give 40 mg of the title compound.
TLC: Rf 0.47 (chloroform : methanol = 9:1)

### [Process 4] 1-Naphthoxyacetyl-Msa-(2S,3S)-AHPBA-Thz-Pip-O-CH₃

Deprotection of 20 mg of the compound obtained by the process 3 was performed similarly to that in Example 125 (Process 2), and the obtained product was dissolved in DMF and neutralized with 3 µl of triethylamine under ice cooling. To the neutralized solution, 4 mg of 1-naphthoxyacetic acid and 10 mg of Bop reagent were added and the resultant mixture was stirred overnight at room temperature. The reaction mixture was treated similarly to that in Example 122 to give 2 mg of 1-naphthoxyacetyl-Msa-(2S,3S)-AHPBA-Thz-(D)-Pip-O-CH₃ and 1 mg of 1-naphthoxyacetyl-Msa-(2S,3S)-AHPBA-Thz-(L)-Pip-O-CH₃.
Analytical HPLC: 21.31, 27.22 min (For the condition, see: Example 35).
FAB-MS: 769 (M+1)

### Comparative Example YY : 1-Naphthoxyacetyl-Phg-(2S,3S)-AHPBA-Thz-NH-tBu

### [Process 1] Boc-Phg-(2S,3S)-AHPBA-Thz-NH-tBu

Deprotection of 38 mg of the compound obtained by Example 82 (Process 2) was performed similarly to that in Example 125 (Process 2), and the obtained product was dissolved in DMF and 11 µl of triethylamine, 36 mg of Boc-Phg-OH and 44 mg of Bop reagent were added under ice cooling and the resultant mixture was stirred overnight at room temperature. The reaction mixture was treated similarly to that in Example 93 (Process 3) to give 10 mg of the title compound.
TLC: Rf 0.90 (chloroform : methanol = 9:1)

### [Process 2] 1-Naphthoxyacetyl-Phg-(2S,3S)-AHPBA-Thz-NH-tBu

Deprotection of 10 mg of the compound obtained by the process 1 was performed similarly to that in Example 125 (Process 2), and the obtained product was dissolved in DMF and neutralized with 37 µl of triethylamine under ice cooling. To the neutralized solution, 3 mg of 1-naphthoxyacetic acid and 12 mg of Bop reagent were added and the resultant mixture was stirred overnight at room temperature. The reaction mixture was treated similarly to that in Example 122 to give 0.4 mg of the title compound.
Analytical HPLC: 26.12 min (For the condition, see: Example 35).
FAB-MS: 683 (M+1)

### Example 161: 1-Naphthoxyacetyl-Ile-(2S,3S)-AHPBA-Thz-NH-tBu

### [Process 1] Boc-Ile-(2S,3S)-AHPBA-Thz-NH-tBu

Deprotection of 100 mg of the compound obtained by Example 82 (Process 2) was performed similarly to that in Example 125 (Process 2), and the obtained product was dissolved in DMF and 29 µl of triethylamine, 50 mg of Boc-Ile-OH and 106 mg of Bop reagent were added under ice cooling and the resultant mixture was stirred overnight at room temperature. The reaction mixture was treated similarly to that in Example 93 (Process 3) to give 10 mg of the title compound.
TLC: Rf 0.90 (chloroform : methanol = 9:1)

### [Process 2] 1-Naphthoxyacetyl-Ile-(2S,3S)-AHPBA-Thz-NH-tBu

Deprotection of 10 mg of the compound obtained by the process 1 was performed similarly to that in Example 125 (Process 2), and the obtained product was dissolved in DMF and neutralized with 37 µl of triethylamine under ice cooling. To the neutralized solution, 3 mg of 1-naphthoxyacetic acid and 12 mg of Bop reagent were added and the resultant mixture was stirred overnight at room temperature. The reaction mixture was treated similarly to that in Example 122 to give 3 mg of the title compound.
Analytical HPLC: 31.15 min (For the condition, see: Example 35).
FAB-MS: 663 (M+1)

### Example 162: 1-Naphthoxyacetyl-Mta-(2S,3S)-AHPBA-Thz-NH-tBu

### [Process 1] Boc-Mta-(2S, 3S)-AHPBA-Thz-NH-tBu

Deprotection of 100 mg of the compound obtained by Example 82 (Process 2) was performed similarly to that in Example 125 (Process 2), and the obtained product was dissolved in DMF and 29 µl of triethylamine, 50 mg of Boc-Ile-OH and 106 mg of Bop reagent were added under ice cooling and the resultant mixture was stirred overnight at room temperature. The reaction mixture was treated similarly to that in Example 93 (Process 3) to give 6.5 mg of the title compound.
TLC: Rf 0.90 (chloroform : methanol = 9:1)

### [Process 2] 1-Naphthoxyacetyl-Mta-(2S,3S)-AHPBA-Thz-NH-tBu

Deprotection of 6.5 mg of the compound obtained by the process 1 was performed similarly to that in Example 125 (Process 2), and the obtained product was dissolved in DMF and neutralized with 30 µl of triethylamine under ice cooling. To the neutralized solution, 3 mg of 1-naphthoxyacetic acid and 12 mg of Bop reagent were added and the resultant mixture was stirred overnight at room temperature. The reaction mixture was treated similarly to that in Example 122 to give 0.7 mg of the title compound.
Analytical HPLC: 30.54 min (For the condition, see: Example 35).
FAB-MS: 667 (M+1)

### Example 163: 1-Naphthoxyacetyl-Thr(Me)-(2S,3S)-AHPBA-Thz-NH-tBu

### [Process 1] Boc-Thr(Me)-OH

In a THF solution containing 0.8 g of Boc-Thr-OH, 100 mg of sodium hydride (60% in oil) was added under ice cooling and the resultant mixture was stirred for 30 min at room temperature. Further, 2.8 ml of methyl iodide was added and the obtained reaction mixture was stirred overnight at room temperature. The reaction mixture was evaporated under reduced pressure and the resultant residue was redissolved in ethyl acetate, washed with 5% sodium hydrogensulfite aqueous solution, water and saturated sodium chloride aqueous solution, successively, and dried over anhydrous sodium sulfate. The dried solution was evaporated under reduced pressure, dissolved in methanol, mixed with DCHA and reevaporated. Ether-hexane was added to the residue to give 0.55 g of the title compound as its DCHA salt.
TLC: Rf 0.63 (chloroform : methanol = 9:1)

### [Process 2] Boc-Thr(Me)-(2S,3S)-AHPBA-Thz-NH-tBu

Deprotection of 100 mg of the compound obtained by Example 82 (Process 2) was performed similarly to that in Example 125 (Process 2), and the obtained product was dissolved in DMF, and 29 µl of triethylamine, 50 mg of Boc-Thr(Me)-OH and 106 mg of Bop reagent were added and the resultant mixture was stirred overnight at room temperature. The reaction mixture was treated similarly to that in Example 93 (Process 3) to give 27.1 mg of the title compound.
TLC: Rf 0.50 (chloroform : methanol = 9:1)

### [Process 3] 1-Naphthoxyacetyl-Thr(Me)-(2S,3S)-AHPBA-Thz-NH-tBu

Deprotection of 27.1 mg of the compound obtained by the process 2 was performed similarly to that in Example 125 (Process 2), and the obtained product was dissolved in DMF and neutralized with 50 µl of triethylamine under ice cooling. To the neutralized solution, 18 mg of 1-naphthoxyacetic acid and 30 mg of Bop reagent were added and the resultant mixture was stirred overnight at room temperature. The reaction mixture was treated similarly to that in Example 122 to give 3 mg of the title compound.
Analytical HPLC: 29.20 min (For the condition, see: Example 35).
FAB-MS: 665 (M+1)

### Example 164: Benzyloxycarbonyl-Asn-(2S,3S)-AHPBA-Pdp-NH-tBu

### [Process 1] Benzyloxycarbonyl-Pdp-NH-tBu

In a dichloromethane solution containing 100 mg of 1-(Benzyloxycarbonyl)-4-phenyl-2,5-dihydropyrrole-2-carboxylic acid [C₆H₅-CH₂O-CO-Pdp-OH], 43 µl of triethylamine, 86 mg of 2-chloro-1, 3-dimethylimidazolinium hexafluorophosphate and 64 µl of tert-butylamine were added under ice cooling and the resultant mixture was stirred for 14 hr. The reaction mixture was treated similarly to that in Comparative Example X (Process 2) to give 66 mg of the title compound.
TLC: Rf 0.62 (chloroform : methanol = 40:1)

### [Process 2] Boc-(2S,3S)-AHPBA-Pdp-NH-tBu

To 66 mg of the protected peptide obtained by the process 1, 3 ml of 30% HBr in acetic acid was added in the presence of 30 µl of anisole and the mixture was stirred for 2 hr at room temperature. The reaction mixture was evaporated under reduced pressure and the resultant residue was redissolved in 3 ml of DMF, and neutralized with 24 µl of triethylamine under ice cooling. To the neutralized solution, 81 mg of Boc-(2S,3S)-AHPBA-OH.DCHA salt, 75 mg of Bop reagent and 24 µl of triethylamine were added and the resultant mixture was stirred overnight. The reaction mixture was treated similarly to that in Comparative Example Z (Process 4) to give 25 mg of the title compound.
TLC: Rf 0.71 (chloroform : methanol = 9:1)

### [Process 3] Benzyloxycarbonyl-Asn-(2S,3S)-AHPBA-Pdp-NH-tBu

Deprotection of 25 mg of the compound obtained by the process 2 was performed similarly to that in Comparative Example X (Process 3), and the obtained product was dissolved in 3 ml of DMF and neutralized with 7 µl of triethylamine under ice cooling. To the neutralized solution, 28 mg of benzyloxy-carbonyl-Asn-ONp, 11 mg of HOBt and 8 µl of N-methylmorpholine were added and the resultant mixture was stirred for 14 hr. The reaction mixture was treated similarly to that in Example 101 (Process 2) to give 9.7 mg of the title compound.
Analytical HPLC: 25.30 min (For the condition, see: Example 35).
FAB-MS: 670 (M+1)

### Example 165 1-Naphthoxyacetyl-Nva-(2S,3S)-AHPBA-Thz-NH-tBu

### [Process 1] Boc-Nva-(2S,3S)-AHPBA-Thz-NH-tBu

Deprotection of 32 mg of the compound obtained by Example 82 (Process 2) was performed similarly to that in Comparative Example X (Process 3), and the obtained product was dissolved in 3 ml of DMF, and neutralized with 9.6 µl of triethylamine under ice cooling. To the neutralized solution, 15 mg of N-Boc-norvaline (Boc-Nva-OH), 30 mg of Bop reagent and 19.2 µl of triethylamine were added and the resultant mixture was stirred 2 hr. The reaction mixture was treated similarly to that in Comparative Example TT (Process 1) to give 25 mg of the title compound.
TLC: Rf 0.93 (chloroform : methanol = 9:1)

### [Process 2] 1-Naphthoxyacetyl-Nva-(2S,3S)-AHPBA-Thz-NH-tBu

Deprotection of 25 mg of the compound obtained by the process 1 was performed similarly to that in Comparative Example X (Process 3), and the obtained product was dissolved in 3 ml of DMF and neutralized with 7.5 µl of triethylamine under ice cooling. To the neutralized solution, 11 mg of 1-naphthoxyacetic acid, 24 mg of Bop reagent and 14.9 µl of triethylamine were added and the resultant mixture was stirred for 2 hr. The reaction mixture was treated similarly to that in Example 137 (Process 5) to give the title compound.
TLC: Rf 0.90 (chloroform : methanol = 9:1)
Analytical HPLC: 31.20 min (For the condition, see: Example 35).
FAB-MS: 649 (M+1)

### Example 166: m-Isopropyloxyphenoxyacetyl-Msa-(2S,3S)-AHPBA-Dtc-NH-tBu

### [Process 1] m-Isopropyloxyphenol [m-(iPro)-Ph-OH]

In 10 ml of THF, 1.5 g of resorcinol was dissolved and 2.24 ml of DBU was added under ice cooling. The resultant mixture was stirred for 10 min at room temperture. To the mixture, 1.92 ml of isopropyl bromide was added and refluxed for 2 hr. The reaction mixture was neutralized with acetic acid and evaporated under reduced pressure. The evaporated residue was redissolved in ethyl acetate and washed with 5% aqueous citric acid solution and saturated aqueous sodium chloride solution, successively, and dried over anhydrous sodium sulfate. The dried solution was evaporated under reduced pressure and the residue was subjected to a silica gel column chromatography (chloroform : methanol = 40:1) to give 466 mg of the title compoucd.
TLC: Rf 0.78 (choroform:methanol = 9:1)

### [Process 2] m-(iPro)-Ph-O-CH₂-CO₂C₂H₅

In 5 ml of THF, 466 mg of the product obtained by the process 1 and 0.67 ml methanol solution of sodium metanolate was added under ice cooling followed by stirring for 10 min. To the reaction mixture, 375 µl of ethyl bromoacetate was added and the mixture was refluxed for 2 hr. The reaction mixture was neutralized with acetic acid and evaporated under reduced pressure. The obtained residue was redissolved in ethyl acetate and washed with 5% aqueous citric acid solution and saturated aqueous sodium chloride solution, successively, and dried over anhydrous sodium sulfate. The dried solution was evaporated under reduced pressure and the residue was subjected to a silica gel column chromatography (chloroform) to give 366 mg of the title compound.
TLC: Rf 0.94 (choroform:methanol = 9:1)

### [Process 3] m-(iPro)-Ph-O-CH₂-CO₂H

In 4 ml of methanol, 366 mg of the compound obtained by the process 2, 764 µl of 4N-NaOH aqueous solution was added under ice cooling and the resultant solution was stirred for 60 min at room temperature. The reaction mixture was neutralized with acetic acid and evaporated under reduce pressure. The resultant residue was redissolved in ethyl acetate and washed with 5% aqueous citric acid solution and saturated aqueous sodium chloride solution, successively and dried over anhydrous sodium sulfate. The dried solution was evaporated under reduced pressure and the residue was crystallized by an addition of ether to give 310 mg of the title compound.
TLC: Rf 0.19 (choroform:methanol = 9:1)

### [Process 4] m-(iPro)-Ph-O-CH₂-CO-Msa-(2S,3S)-AHPBA-Dtc-NH-tBu

Deprotection of 105 mg of the compound obtained by Example 105 (Process 1) was performed similarly to that in Comparative Example X (Process 3), and the obtained product was dissolved in 3 ml of DMF and neutralized with 24 µl of triethylamine under ice cooling. To the neutralized solution, 38 mg of m-(iPro)-Ph-O-CH₂-CO₂H, 80 mg of Bop reagent and 48 µl of triethylamine were added and the resultant mixture was stirred for 2 hr. The reaction mixture was treated similarly to that in Comparative Example RR to give 5.1 mg of the title compound.
Analytical HPLC: 29.51 min (For the condition, see: Example 35).
FAB-MS: 735 (M+1)

### Example 167: m-(Piper-CO-)Ph-O-CH₂-CO-Msa-(2S,3S)-AHPBA-Thz-NH-tBu

### [Process 1] m-(Piperidinocarbonyl)phenol [m-(Piper-CO)-Ph-OH]

In 15 ml of DMF, 700 mg of m-hydroxybenzoic acid was dissolved, and 2.84 ml of piperidine, 3.80 g of Bop reagent and 70 mg of 4-dimethylaminopyridine were added under ice cooling, and the resultant mixture was stirred for 15 hr. The reaction mixture was evaporated under reduced pressure and the residue was redissolved in ethyl acetate. The ethyl acetate solution was washed with 5% citric acid aqueous solution and saturated sodium chloride aqueous solution, successively, and dried over anhydrous sodium sulfate. The dried solution was evaporated under reduced pressure and subjected to a silica gel column chromatography (chloroform) to give 267 mg of the title compound.
TLC: Rf 0.83 (chloroform : methanol:acetic acid = 9:1:0.5)

### [Process 2] m-(Piper-CO)-Ph-O-CH₂-CO₂C₂H₅

In 5 ml of THF, 267 mg of the compound obtained by the process 1 was dissolved and 316 µl of DBU was added under ice cooling. The resultant mixture was stirred for 10 min. To the mixture, 173 µl of ethyl bromoacetate was added and refluxed for 2 hr. The reaction mixture was neutralized with acetic acid and treated similarly to that in the process 1 to give 112 mg of the title compound.
TLC: Rf 0.69 (choroform:methanol = 20:1)

### [Process 3] m-(Piper-CO)-Ph-O-CH₂CO₂H

In 3 ml of methanol, 110 mg of the product obtained by the process 2 was dissolved and 303 µl of 4N-NaOH aqueous solution was added under ice cooling. The resultant solution was stirred for 60 min at room temperature. The reaction mixture was evaporated under reduced pressure, redissolved in ethyl acetate, washed with 5% aqueous citric acid solution and saturated aqueous sodium chloride solution and dried over anhydrous sodium sulfate. The dried solution was evaporated under reduced pressure and crystallized from ether to give 92 mg of the title compound.
TLC: Rf 0.23 (choroform:methanol:water = 8:3:1, lower layer)

### [Process 4] m-(Piper-CO)-Ph-O-CH₂-CO-Msa-(2S,3S)-AHPBA-Thz-NH-tBu

Deprotection of 42 mg of the compound obtained by Example 100 (Process 1) was performed similarly to that in Comparative Example X (Process 3), and the obtained product was dissolved in 3 ml of DMF and neutralized with 9.1 µl of triethylamine under ice cooling. To the neutralized solution, 18 mg of m-(piperidinocarbonyl)-phenoxyacetic acid, 29 mg of Bop reagent and 18.2 µl of triethylamine were added and the resultant mixture was stirred for 2 hr. The reaction mixture was treated similarly to that in Comparative Example RR to give 20.4 mg of the title compound.
TLC: Rf 0.17 (chloroform : methanol = 20:1)
Analytical HPLC: 23.31 min (For the condition, see: Example 35).
FAB-MS: 760 (M+1)

### Example 168: m-(Morph-CO)-Ph-O-CH₂-CO-Msa-(2S,3S)-AHPBA-Thz-NH-tBu

### [Process 1] m-(Morpholinocarbonyl)phenol [m-(Morph-CO)-Ph-OH]

In 15 ml of DMF, 700 mg of m-hydroxybenzoic acid was dissolved, and 2.50 ml of morpholine, 3.04 g of Bop reagent were added under ice cooling, and the resultant mixture was stirred for 3 hr. The reaction mixture was treated similarly to that in Comparative Example X (Process 2) to give 1.10 g of the title compound.
TLC: Rf 0.34 (chloroform : methanol = 20:1)

### [Process 2] m-(Morph-CO)-Ph-O-CH₂CO₂C₂H₅

In THF, 500 mg of the compound obtained by the process 1 was dissolved and 531 µl of methanol solution of sodium methanolate was added under ice cooling. The resultant mixture was stirred for 10 min. To the mixture, 295 µl of ethyl bromoacetate was added and refluxed for 2 hr. The reaction mixture was treated similarly to that in Example 167 (Precess 2) except for the chromatography solvent (chloroform : methanol = 40:1) to give 243 mg of the title compound.
TLC: Rf 0.82 (choroform:methanol = 9:1)

### [Process 3] m-(Morph-CO)-Ph-O-CH₂-CO₂H

In methanol, 243 mg of the product obtained by the process 2 was dissolved and 621 µl of 4N-NaOH aqueous solution was added under ice cooling. The resultant solution was stirred for 60 min at room temperature. The reaction mixture was treated similarly to that in Example 167 (Process 3) to give 56 mg of the title compound.
TLC: Rf 0.82 (choroform:methanol = 9:1)

### [Process 4] m-(Morph-CO)-Ph-O-CH₂-CO-Msa-(2S,3S)-AHPBA-Thz-NH-tBu

Deprotection of 28 mg of the compound obtained by Example 100 (Process 1) was performed similarly to that in Comparative Example X (Process 3), and the obtained product was dissolved in 3 ml of DMF and neutralized with 6.1 µl of triethylamine under ice cooling. To the neutralized solution, 12 mg of m-(Morph-CO)-Ph-O-CH₂-CO₂H, 20 mg of Bop reagent and 12.1 µl of triethylamine were added and the resultant mixture was stirred for 2 hr. The reaction mixture was treated similarly to that in Comparative Example TT (Process 2) to give 12.1 mg of the title compound.
TLC: Rf 0.30 (chloroform : methanol = 20:1)
Analytical HPLC: 19.10 min (For the condition, see: Example 35).
FAB-MS: 762 (M+1)

### Example 169: m-(iPrO)-Ph-O-CH₂-CO-Asn-(2S,3S)-AHPBA-Dtc-NH-tBu

Deprotection of 32 mg of the compound obtained by Example 106 (Process 1) was performed similarly to that in Comparative Example X (Process 3), and the obtained product was dissolved in 3 ml of DMF and neutralized with 7.3 µl of triethylamine under ice cooling. To the neutralized solution, 11 mg of m-isopropyloxyphenoxyacetic acid obtained in Example 166 (Process 3), 24 mg of Bop reagent and 14.6 µl of triethylamine were added and the resultant mixture was stirred for 2 hr. The reaction mixture was treated similarly to that in Comparative Example TT (Process 2) to give 7.8 mg of the title compound.
Analytical HPLC: 26.68 min (For the condition, see: Example 35).
FAB-MS: 700 (M+1)

### Comparative Example ZZ: 1-Naphthoxyacetyl-Alg-(2S,3S)-AHPBA-Thz-NH-tBu

### [Process 1] Boc-Alg-(2S,3S)-AHPBA-Thz-NH-tBu

Deprotection of 58 mg of the compound obtained by Example 82 (Process 2) was performed similarly to that in Comparative Example X (Process 3), and the obtained product was dissolved in 3 ml of DMF and neutralized with 17.3 µl of triethylamine under ice cooling. To the neutralized solution, 54 mg of Boc-L-allylglycine.DCHA, 61 mg of Bop reagent and 19 µl of triethylamine were added and the resultant mixture was stirred for 2 hr. The reaction mixture was treated similarly to that in Comparative Example VV (Process 2) to give 76 mg of the title compound.
TLC: Rf 0.66 (chloroform : methanol = 20:1)

### [Process 2] 1-Naphthoxyacetyl-Alg-(2S,3S)-AHPBA-Thz-NH-tBu

Deprotection of 76 mg of the compound obtained by the process 1 was performed similarly to that in Comparative Example Z (Process 2), and the obtained product was dissolved in 3 ml of DMF and neutralized with 18.8 µl of triethylamine under ice cooling. To the neutralized solution, 30 mg of 1-naphthoxyacetic acid, 66 mg of Bop reagent and 39.4 µl of triethylamine were added and the resultant mixture was stirred for 2 hr. The reaction mixture was treated similarly to that in Comparative Example TT (Process 1) to give 39 mg of a crude product. In methanol, 19 mg of the product was dissolved, fractionated by reversed-phase HPLC anl lyophilized to give 7.7 mg of the title compound.
TLC: Rf 0.53 (chloroform : methanol = 20:1)
Analytical HPLC: 31.26 min (For the condition, see: Example 35).
FAB-MS: 647 (M+1)

### Example 171: 2,3-diMe-Ph-O-CH₂-CO-Asn-(2S,3S)-AHPBA-Dtc-NH-tBu

### [Process 1] 2,3-diMe-Ph-O-CH₂CO₂C₂H₅

To a THF solution of 500 mg of 2,3-dimethylphenol (2,3-diMe-Ph-OH), 672 µl of DBU was added under ice cooling. The resultant mixture was stirred for 10 min at room temperture. To the mixture, 50 µl of ethyl bromoacetate was added and refluxed for 2 hr. The reaction mixture was treated similarly to that in Example 167 (Process 2) to give 156 mg of the title compound.
TLC: Rf 0.89 (choroform:methanol = 20:1)

### [Process 2] 2,3-diMe-Ph-O-CH₂-CO₂H

In a methanol solution containing the compound obtained by the process 1, 375 µl of 4N-NaOH aqueous solution was added under ice cooling and the resultant mixture was stirred for 60 min at room temperature and treated similarly to that in Example 166 (Process 3) to give 126 mg of the title compound.
TLC: Rf 0.20 (choroform:methanol = 9:1)

### [Process 3] 2,3-diMe-Ph-O-CH₂-CO-Asn-(2S,3S)-AHPBA-Dtc-NH-tBu

Deprotection of 33 mg of the compound obtained by Example 106 (Process 1) was performed similarly to that in Comparative Example X (Process 3), and the obtained product was dissolved in 3 ml of DMF and neutralized with 10.4 µl of triethylamine under ice cooling. To the neutralized solution, 14 mg of 2,3-dimethylphenoxyacetic acid, 33 mg of Bop reagent and 20.7 µl of triethylamine were added and obtained mixture was stirred for 2 hr. The reaction mixture was treated similarly to that in Comparative Example TT (Process 2) to give 6.1 mg of the title compound.
TLC: Rf 0.43 (choroform:methanol = 9:1)
Analytical HPLC: 26.55 min (For the condition, see: Example 35).
FAB-MS: 671 (M+1)

### Example 172: 1-Naphthoxyacetyl-Msa-(2S,3S)-AHPBA-Thz-Gly-NH₂

### [Process 1] Boc-Thz-Gly-NH₂

Deprotection of 500 mg of Boc-Gly-NH₂ was performed similarly to that in Comparative Example X (Process 3), and the obtained product was dissolved in dichloromethane, and 0.39 ml of tri-ethylamine, 0.67 g of Boc-Thz-OH, 0.47 g of HOBt and 0.64 g of EDC hydrochloride were added under ice cooling and the mixture was stirred overnight at room temperature. The reaction mixture was treated similarly to that in Comparative Example VV (Process 2) to give 0.54 g of the title compound as oil.
TLC: Rf 0.63 (chloroform : methanol = 9:1)

### [Process 2] Boc-(2S,3S)-AHPBA-Thz-Gly-NH₂

Deprotection of 540 mg of the compound obtained by the process 1 was performed similarly to that in Example 125 (Process 2), and the obtained product was dissolved in DMF. To the solution, 90 µl of triethylamine, 100 mg of Boc-(2S,3S)-AHPBA-OH and 160 mg of Bop reagent were added under ice cooling and the mixture was stirred overnight at room temperature. The reaction mixture was treated similarly to that in Example 93 (Process 3) to give 13.5 mg of title compound.
TLC: Rf 0.48 (choroform:methanol = 9:1)

### [Process 3] Boc-Msa-(2S,3S)-AHPBA-Thz-Gly-NH₂

Deprotection of 13.5 mg of the compound obtained by the process 2 was performed similarly to that in Example 125 (Process 2), and the obtained product was dissolved in DMF. To the solution, 5 µl of triethylamine, 13 mg of Boc-Msa-OH and 21 mg of Bop reagent were added under ice cooling and the mixture was stirred overnight at room temperature. The reaction mixture was treated similarly to that in Example 93 (Process 3) to give 20 mg of title compound.
TLC: Rf 0.15 (choroform:methanol = 9:1)

### [Process 4] 1-Naphthoxyacetyl-Msa-(2S,3S)-AHPBA-Thz-Gly-NH₂

Deprotection of 20 mg of the compound obtained by the process 3 was performed similarly to that in Example 125 (Process 2), and the obtained product was dissolved in DMF and neutralized with 6 µl of triethylamine under ice cooling. To the neutralized solution, 9 mg of 1-naphthoxyacetic acid and 21 mg of Bop reagent were added and the mixture was stirred overnight at room temperature. The reaction mixture was treated similarly to that in Example 122 to give 3 mg of the title compound.
Analytical HPLC: 20.06 min (For the condition, see: Example 35).
FAB-MS: 700 (M+1)

### Example 173: 1-Naphthoxyacetyl-Msa-(2S,3S)-AHPBA-Thz-GABA-NH₂

### [Process 1] Boc-Thz-GABA-NH₂

Deprotection of 400 mg of 4-N-t-butoxycarbonylamino-butanamide [Boc-GABA-NH₂] was performed similarly to that in Example 125 (Process 2), and the obtained product was dissolved in dichloromethane, and 0.28 ml of tri-ethylamine, 0.47 g of Boc-Thz-OH, 1.06 g of Bop reagent were added under ice cooling and the mixture was stirred overnight at room temperature. The reaction mixture was treated similarly to that in Example 144 (Process 2) to give 27 mg of the title compound.
TLC: Rf 0.56 (chloroform : methanol = 9:1)

### [Process 2] Boc-(2S,3S)-AHPBA-Thz-GABA-NH₂

Deprotection of 95 mg of the compound obtained by the process 1 was performed similarly to that in Example 125 (Process 2), and the obtained product was dissolved in DMF. To the solution, 42 µl of triethylamine, 89 mg of Boc-(2S, 3S)-AHPBA-OH and 146 mg of Bop reagent were added under ice cooling and the mixture was stirred overnight at room temperature. The reaction mixture was treated similarly to that in Example 93 (Process 3) to give 62 mg of title compound.
TLC: Rf 0.30 (choroform:methanol = 9:1)

### [Process 3] Boc-Msa-(2S,3S)-AHPBA-Thz-GABA-NH₂

Deprotection of 62 mg of the compound obtained by the process 2 was performed similarly to that in Example 125 (Process 2), and the obtained product was dissolved in DMF. To the solution, 17.5 µl of triethylamine, 37 mg of Boc-Msa-OH and 61 mg of Bop reagent were added under ice cooling and the mixture was stirred overnight at room temperature. The reaction mixture was treated similarly to that in Example 144 (Process 2) to give 30 mg of the title compound.
TLC: Rf 0.18 (choroform:methanol = 9:1)

### [Process 4] 1-Naphthoxyacetyl-Msa-(2S,3S)-AHPBA-Thz-GABA-NH₂

Deprotection of 30 mg of the compound obtained by the process 3 was performed similarly to that in Example 125 (Process 2), and the obtained product was dissolved in DMF and neutralized with 6.6 µl of triethylamine under ice cooling. To the neutralized solution, 9.5 mg of 1-naphthoxyacetic acid and 23 mg of Bop reagent were added and the mixture was stirred overnight at room temperature. The reaction mixture was treated similarly to that in Example 122 to give 2 mg of the title compound.
Analytical HPLC: 20.58 min (For the condition, see: Example 35).
FAB-MS: 728 (M+1)

### Example 174: 1-Naphthoxyacetyl-Msa-(2S,3S)-AHPBA-Thz-BAIB-NH₂

### [Process 1] Boc-Thz-BAIB-NH₂

Deprotection of 400 mg of 3-N-t-butoxycarbonylamino-2-methylpropanamide [Boc-BAIB-NH₂] was performed similarly to that in Example 125 (Process 2), and the obtained product was dissolved in dichloromethane, and 0.29 ml of triethylamine, 0.47 g of Boc-Thz-OH, 1.06 g of Bop reagent were added under ice cooling and the mixture was stirred overnight at room temperature. The reaction mixture was treated similarly to that in Example 93 (Process 3] to give 0.52 g of the title compound.
TLC: Rf 0.83 (chloroform : methanol = 9:1)

### [Process 2] Boc-(2S,3S)-AHPBA-Thz-BAIB-NH₂

Deprotection of 130 mg of the compound obtained by the process 1 was performed similarly to that in Example 125 (Process 2), and the obtained product was dissolved in DMF. To the solution, 56 µl of triethylamine, 180 mg of Boc-(2S,3S)-AHPBA-OH and 194 mg of Bop reagent were added under ice cooling and the mixture was stirred overnight at room temperature. The reaction mixture was treated similarly to that in Example 144 (Process 2) to give 40 mg of the title compound.
TLC: Rf 0.48 (choroform:methanol = 9:1)

### [Process 3] Boc-Msa-(2S,3S)-AHPBA-Thz-BAIB-NH₂

Deprotection of 40 mg of the compound obtained by the process 2 was performed similarly to that in Example 125 (Process 2), and the obtained product was dissolved in DMF. To the solution, 11 µl of triethylamine, 24 mg of Boc-Msa-OH and 39 mg of Bop reagent were added under ice cooling and the mixture was stirred overnight at room temperature. The reaction mixture was treated similarly to that in Example 93 (Process 3] to give 30 mg of the title compound.
TLC: Rf 0.52 (choroform:methanol = 9:1)

### [Process 4] 1-Naphthoxyacetyl-Msa-(2S,3S)-AHPBA-Thz-BAIB-NH₂

Deprotection of 30 mg of the compound obtained by the process 3 was performed similarly to that in Example 125 (Process 2), and the obtained product was dissolved in DMF and neutralized with 6.6 µl of triethylamine under ice cooling. To the neutralized solution, 10 mg of 1-naphthoxyacetic acid and 25 mg of Bop reagent were added and the mixture was stirred overnight at room temperature. The reaction mixture was treated similarly to that in Example 122 to give 5 mg of the title compound.
Analytical HPLC: 21.27 min (For the condition, see: Example 35).
FAB-MS: 728 (M+1)

### Example 175: 1-Naphthoxyacetyl-Msa-(2S,3S)-AHPBA-Thz-BANB-NH₂

### [Process 1] Boc-Thz-BANB-NH₂

Deprotection of 400 mg of 3-N-t-butoxycarbonylaminobutanamide [Boc-BANB-NH₂] was performed similarly to that in Example 125 (Process 2), and the obtained product was dissolved in dichloromethane, and 0.29 ml of triethylamine, 0.47 g of Boc-Thz-OH, 1.06 g of Bop reagent were added under ice cooling and the mixture was stirred overnight at room temperature. The reaction mixture was treated similarly to that in Example 93 (Process 3) to give 0.38 g of the title compound.
TLC: Rf 0.67 (chloroform : methanol = 9:1)

### [Process 2] Boc-(2S,3S)-AHPBA-Thz-BANB-NH₂

Deprotection of 130 mg of the compound obtained by the process 1 was performed similarly to that in Example 125 (Process 2), and the obtained product was dissolved in DMF. To the solution, 77 µl of triethylamine, 162 mg of Boc-(2S,3S)-AHPBA-OH and 270 mg of Bop reagent were added under ice cooling and the mixture was stirred overnight at room temperature. The reaction mixture was treated similarly to that in Example 144 (Process 2) to give 60 mg of the title compound.
TLC: Rf 0.48 (choroform:methanol = 9:1)

### [Process 3] Boc-Msa-(2S,3S)-AHPBA-Thz-BANB-NH₂

Deprotection of 60 mg of the compound obtained by the process 2 was performed similarly to that in Example 125 (Process 2), and the obtained product was dissolved in DMF. To the solution, 17 µl of triethylamine, 36 mg of Boc-Msa-OH and 58 mg of Bop reagent were added under ice cooling and the mixture was stirred overnight at room temperature. The reaction mixture was treated similarly to that in Example 93 (Process 3) to give 30 mg of the title compound.
TLC: Rf 0.51 (choroform:methanol = 9:1)

### [Process 4] 1-Naphthoxyacetyl-Msa-(2S,3S)-AHPBA-Thz-BANB-NH₂

Deprotection of 30 mg of the compound obtained by the process 3 was performed similarly to that in Example 125 (Process 2), and the obtained product was dissolved in DMF and neutralized with 6.6 µl of triethylamine under ice cooling. To the neutralized solution, 10 mg of 1-naphthoxyacetic acid and 25 mg of Bop reagent were added and the mixture was stirred overnight at room temperature. The reaction mixture was treated similarly to that in Example 122 to give 3 mg of the title compound.
Analytical HPLC: 20.74 min (For the condition, see: Example 35).
FAB-MS: 728 (M+1)

### Example 176: Benzyloxycarbonyl-Asn-(2S,3S)-AHPBA-Pro-NH-sBu

### [Process 1] Boc-Pro-NH-CH(CH₃)(C₂H₅) [Boc-Pro-NH-sBu]

In dichloromethane solution containing 0.1 g of sec-butylamine, 0.5 g of Boc-Pro-OH, 0.2 g of HOBt and 0.3 g of EDC hydrochloride were added under ice cooling and the mixture was stirred overnight at room temperature. The reaction mixture was treated similarly to that in Example 144 (Process 2) to give 0.4 g of the title compound.
TLC: Rf 0.84 (chloroform : methanol = 9:1)

### [Process 2] Boc-(2S,3S)-AHPBA-Pro-NH-sBu

Deprotection of 57 mg of the compound obtained by the process 1 was performed similarly to that in Example 125 (Process 2), and the obtained product was dissolved in dichloromethane. To the solution, 29 µl of triethylamine, 100 mg of Boc-(2S,3S)-AHPBA-OH and 112 mg of Bop reagent were added under ice cooling and the mixture was stirred overnight at room temperature. The reaction mixture was treated similarly to that in Example 144 (Process 2) to give 0.12 g of the title compound.
TLC: Rf 0.46 (choroform:methanol = 9:1)

### [Process 3] Benzyloxycarbonyl-Asn-(2S,3S)-AHPBA-Pro-NH-sBu

Deprotection of 60 mg of the compound obtained by the process 2 was performed similarly to that in Example 125 (Process 2), and the obtained product was dissolved in dichloromethane. To the solution, 19 µl of triethylamine and 52 mg of benzyloxycarbonyl-Asn-ONp were added under ice cooling and the mixture was stirred overnight at room temperature. The reaction mixture was treated similarly to that in Example 122 to give 2 mg of the title compound.
Analytical HPLC: 20.14 min (For the condition, see: Example 35).
FAB-MS: 596 (M+1)

### Example 177: Benzyloxycarbonyl-Asn-(2S,3S)-AHPBA-Dtc-NH₂

### [Process 1] Boc-Dtc-NH₂

A THF solution containing 0.2 g of Boc-Dtc-OH was chilled to -15°C and 108 µl of triethylamine and 101 µl of isobutyl chloroformate were added. Further 15 min later, 2 ml of ammonia water (28%) was added and the mixture was stirred overnight. The reaction mixture was evaporated under reduced pressure and the residue was redissolved in ethyl acetate, washed with 5% aqueous sodium hydrogen-carabonate solution, 10% aqueous citric acid solution and saturated aqueous sodium chloride solution, successively, and dried over anhydrous sodium sulfate. The dried solution was evaporated under reduced pressure to give 0.12 g of the title compound as oil.
TLC: Rf 0.48 (chloroform : methanol = 9:1)

### [Process 2] Boc-(2S,3S)-AHPBA-Dtc-NH₂

Deprotection of 120 mg of the compound obtained by the process 1 was performed similarly to that in Example 125 (Process 2), and the obtained product was dissolved in dichloromethane. To the solution, 64.4 µl of triethylamine, 219 mg of Boc-(2S,3S)-AHPBA-OH and 224 mg of Bop reagent were added under ice cooling and the mixture was stirred overnight at room temperature. The reaction mixture was treated similarly to that in Example 144 (Process 2) to give 170 mg of the title compound.
TLC: Rf 0.61 (choroform:methanol = 9:1)

### [Process 3] Benzyloxycarbonyl-Asn-(2S,3S)-AHPBA-Dtc-NH₂

Deprotection of 44 mg of the compound obtained by the process 2 was performed similarly to that in Example 125 (Process 2), and the obtained product was dissolved in DMF. To the solution, 14 µl of triethylamine and 78 mg of benzyloxycarbonyl-Asn-ONp were added under ice cooling and the mixture was stirred overnight at room temperature. The reaction mixture was treated similarly to that in Example 122 to give 1 mg of the title compound.
Analytical HPLC: 21.90 min (For the condition, see: Example 35).
FAB-MS: 586 (M+1)

### Comparative Example A¹: Benzyloxycarbonyl-Asn-(2S,3S)-AHPBA decahydroisoquinoline

### [Process 1] Boc-(2S,3S)-AHPBA-Diq

In dichloromethane solution containing 28 mg of decahydroisoquinoline, 96 mg of Boc-(2S,3S)-AHPBA-OH, 102 mg of Bop reagent were added under ice cooling and the mixture was stirred overnight at room temperature. The reaction mixture was treated similarly to that in Example 144 (Process 2) to give 0.12 g of the title compound.
TLC: Rf 0.64 (chloroform : methanol = 20:1)

### [Process 2] Benzyloxycarbonyl-Asn-(2S,3S)-AHPBA-Diq

Deprotection of 120 mg of the compound obtained by the process 1 was performed similarly to that in Example 125 (Process 2), and the obtained product was dissolved in DMF. To the solution, 28 µl of triethylamine and 93 mg of benzyloxycarbonyl-Asn-ONp were added under ice cooling and the mixture was stirred overnight at room temperature. The reaction mixture was treated similarly to that in Example 122 to give 2 mg of the title compound.
Analytical HPLC: 31.57 min (For the condition, see: Example 35).
FAB-MS: 565 (M+1)
¹H NMR (DMSO-d₆, 500 MHz): Fig. 6

### Example 179: 1-Naphthoxyacetyl-Val-(2S,3S)-AHPBA-Thz-NH-tBu

### [Process 1] Boc-Val-(2S,3S)-AHPBA-Thz-NH-tBu

Deprotection of 45 mg of the compound obtained by Example 82 (Process 2) was performed similarly to that in Example 125 (Process 2), and the obtained product was dissolved in DMF. To the solution, 14 µl of triethylamine, 22 mg of Boc-Val-OH and 53 mg of Bop reagent were added under ice cooling and the mixture was stirred overnight at room temperature. The reaction mixture was treated similarly to that in Example 93 (Process 3) to give 40 mg of the title compound.
TLC: Rf 0.69 (choroform:methanol = 9:1)

### [Process 2] 1-Nahthoxyacetyl-Val-(2S,3S)-AHPBA-Thz-NH-tBu

Deprotection of 40 mg of the compound obtained by the process 1 was performed similarly to that in Example 125 (Process 2), and the obtained product was dissolved in DMF. The solution was neutralized with 10 µl of triethylamine under ice cooling. To the neutralized solution, 14 mg of 1-naphthoxyacetic acid and 37 mg of Bop reagent were added and the mixture was stirred overnight at room temperature. The reaction mixture was treated similarly to that in Example 122 to give 2 mg of the title compound.
Analytical HPLC: 30.54 min (For the condition, see: Example 35).
FAB-MS: 649 (M+1)

### Comparative Example B' : 1-Naphthoxyacetyl-Prg-(2S,3S)-AHPBA-Thz-NH-tBu

### [Process 1] N-(t-butoxycarbonyl)propargylglycine [Boc-Prg-OH]

In a THF solution containing 1.0 g of diethyl N-benzyloxycarbonylaminomalonate, 0.17 g of sodium hydride (60% in oil) was added under ice cooling and the mixture was stirred for 30 min. The reaction mixture was evaporated under reduced pressure and redissolved in dimethylsulfoxide (DMSO). To the solution, 0.36 ml of propargyl bromide was added and the mixture was stirred overnight. The reaction mixture was mixed with water and extracted with ether. The ether layer was washed with saturated aqueous sodium chloride and dried over sodium sulfate. The dried solution was evaporated under reduced pressure and the residue was hydrolyzed with 2N-NaOH for 5 hr, followed by t-butoxy-carbonylation with (Boc)₂O to give 0.6 g of the title compound.
TLC: Rf 0.37 (chloroform : methanol = 5:1)

### [Process 2] Boc-Prg-(2S,3S)-AHPBA-Thz-NH-tBu

Deprotection of 55 mg of the compound obtained by Example 82 (Process 2) was performed similarly to that in Example 125 (Process 2), and the obtained product was dissolved in DMF. To the solution, 20 µl of triethylamine, 55 mg of Boc-Prg-OH and 76 mg of Bop reagent were added under ice cooling and the mixture was stirred overnight at room temperature. The reaction mixture was treated similarly to that in Example 93 (Process 3] to give 80 mg of the title compound.
TLC: Rf 0.76 (choroform:methanol = 9:1)

### [Process 3] 1-Naphthoxyacetyl-Prg-(2S,3S)-AHPBA-Thz-NH-tBu

Deprotection of 80 mg of the compound obtained by the process 2 was performed similarly to that in Example 125 (Process 2), and the obtained product was dissolved in DMF. The solution was neutralized with 19.6 µl of triethylamine under ice cooling. To the neutralized solution, 28 mg of 1-naphthoxyacetic acid and 74 mg of Bop reagent were added and the mixture was stirred overnight at room temperature. The reaction mixture was treated similarly to that in Example 122 to give 1 mg of the title compound.
Analytical HPLC: 29.74 min (For the condition, see: Example 35).
FAB-MS: 645 (M+1)

### Comparative Example C':1-Naphthoxyacetyl-Aca-(2S,3S)-AHPBA-Thz-NH-tBu

### [Process 1] 2-(N-t-butoxycarbonylamino)-4-oxopentanoic acid [Boc-Aca-OH]

In a THF solution containing 1 g of diethyl N-benzyloxy-carbonylaminomalonate, 0.17 g of sodium hydride was added under ice cooling and the mixture was stirred for 30 min. The reaction mixture was evaporated under reduced pressure and redissolved in DMSO. To the solution, 0.26 ml of bromoacetone was added under ice cooling and the mixture was stirred overnight. The reaction mixture was treated similarly to that in Comparative Example B' (Process 1) to give 294 mg of the title compound.
TLC: Rf 0.24 (chloroform : methanol = 5:1)

### [Process 2] Boc-Aca-(2S,3S)-AHPBA-Thz-NH-tBu

Deprotection of 63 mg of the compound obtained by Example 82 (Process 2) was performed similarly to that in Example 125 (Process 2), and the obtained product was dissolved in DMF. To the solution, 31 µl of triethylamine, 90 mg of Boc-Aca-OH and 117 mg of Bop reagent were added under ice cooling and the mixture was stirred overnight at room temperature. The reaction mixture was treated similarly to that in Comparative Example Z (Process 4) to give 40 mg of the title compound.
TLC: Rf 0.74 (choroform:methanol = 9:1)

### [Process 3] 1-Naphthoxyacetyl-Aca-(2S,3S)-AHPBA-Thz-NH-tBu

Deprotection of 40 mg of the compound obtained by the process 2 was performed similarly to that in Example 125 (Process 2), and the obtained product was dissolved in DMF. The solution was neutralized with 9.7 µl of triethylamine under ice cooling. To the neutralized solution, 14 mg of 1-naphthoxyacetic acid and 37 mg of Bop reagent were added and the mixture was stirred overnight at room temperature. The reaction mixture was treated similarly to that in Example 122 to give 1 mg of the title compound.
Analytical HPLC: 25.79 min (For the condition, see: Example 35).
FAB-MS: 663 (M+1)

### Example 182: Benzyloxycarbonyl-Asn-(2S,3S)-AHPBA-Dmp-NH-tBu

### [Process 1] Boc-Dmp-NH-tBu

In dichloromethane solution containing 95 mg of tert-butylamine, 280 mg of 1-t-butoxycarbonyl-3, 3-dimethyl-pyrrolidine-2-carboxylic acid [Boc-Dmp-OH] and 320 mg of 2-chloro-1,3-dimethylimidazolinium hexafluorophosphate were added under ice cooling and the mixture was stirred overnight at room temperature. The reaction mixture was treated similarly to that in Comparative Example X (Process 3) to give 90 mg of the title compound.
TLC: Rf 0.86 (chloroform : methanol = 20:1)

### [Process 2] Boc-(2S,3S)-AHPBA-Dmp-NH-tBu

Deprotection of 90 mg of the compound obtained by the process 1 was performed similarly to that in Example 125 (Process 2), and the obtained product was dissolved in dichloromethane. To the solution, 89 mg of Boc-(2S,3S)-AHPBA-OH and 159 mg of Bop reagent were added under ice cooling and the mixture was stirred overnight at room temperature. The reaction mixture was treated similarly to that in Example 144 (Process 2) to give 82 mg of the title compound.
TLC: Rf 0.78 (chloroform : methanol = 9:1)

### [Process 3] Benzyloxycarbonyl-Asn-(2S,3S)-AHPBA-Dmp-NH-tBu

Deprotection of 65 mg of the compound obtained by the process 2 was performed similarly to that in Example 125 (Process 2), and the obtained product was dissolved in DMF. To the solution was added with 19 µl of triethylamine, 80 mg of benzyloxycarbonyl-Asn-ONp, 32 mg of HOBt and 23 µl of N-methylmorpholine under ice cooling, and the mixture was stirred overnight at room temperature. The reaction mixture was treated similarly to that in Comparative Example TT (Process 2) to give a crude title compound. The crude solid was dissolved in methanol and subjected to a reversed-phase HPLC (water-acetonitrile system), fractionated, purified and lyophilized to give 10.3 mg of the title compound.
Analytical HPLC: 23.06 min (For the condition, see: Example 35).
FAB-MS: 624 (M+1)

### Example 183: 1-Naphthoxyacetyl-Msa-(2S,3S)-AHPBA-Dmp-NH-tBu

### [Process 1] Boc-Msa-(2S,3S)-AHPBA-Dmp-NH-tBu

Deprotection of 82 mg of the compound obtained by Example 182 (Process 2) was performed similarly to that in Example 125 (Process 2), and the obtained product was dissolved in DMF. To the solution, 81 mg of Boc-Msa-OH and 134 mg of Bop reagent were added under ice cooling and the mixture was stirred overnight at room temperature. The reaction mixture was treated similarly to that in Example 144 (Process 2) to give 78 mg of the title compound.
TLC: Rf 0.54 (chloroform : methanol = 9:1)

### [Process 2] 1-Naphthoxyacetyl-Msa-(2S,3S)-AHPBA-Dmp-NH-tBu

Deprotection of 78 mg of the compound obtained by the process 1 was performed similarly to that in Example 125 (Process 2), and the obtained product was dissolved in DMF. The solution was neutralized with 15.4 µl of triethylamine under ice cooling. To the neutralized solution, 23 mg of 1-naphthoxyacetic acid and 54 mg of Bop reagent were added and the mixture was stirred overnight at room temperature. The reaction mixture was treated similarly to that in Example 122 to give 3 mg of the title compound.
Analytical HPLC: 29.27 min (For the condition, see: Example 35).
FAB-MS: 709 (M+1)

### Example 184: Benzyloxycarbonyl-Asn-(2S,3S)-AHPBA-Php-NH-tBu

### [Process 1] Boc-Php-NH-tBu

In dichloromethane solution containing 50 mg of tert-butylamine, 200 mg of 1-t-butoxycarbonyl 3-phenylpyrrolidine-2-carboxylic acid (Boc-Php-OH) and 140 mg of 2-chloro-1,3-dimethylimidazolinium hexafluorophosphate were added under ice cooling and the mixture was stirred overnight at room temperature. The reaction mixture was treated similarly to that in Comparative Example VV (Process 2) to give 190 mg of the title compound.
TLC: Rf 0.83 (chloroform : methanol = 20:1)

### [Process 2] Boc-(2S,3S)-AHPBA-Php-NH-tBu

Deprotection of 190 mg of the compound obtained by the process 1 was performed similarly to that in Example 125 (Process 2), and the obtained product was dissolved in dichloromethane. To the solution, 37 µl of triethylamine, 261 mg of Boc-(2S,3S)-AHPBA-OH and 292 mg of Bop reagent were added under ice cooling and the mixture was stirred overnight at room temperature. The reaction mixture was treated similarly to that in Comparative Example TT (Process 1) to give 100 mg of the title compound.
TLC: Rf 0.78 (chloroform : methanol = 9:1)

### [Process 3] Benzyloxycarbonyl-Asn-(2S,3S)-AHPBA-Php-NH-tBu

Deprotection of 100 mg of the compound obtained by the process 2 was performed similarly to that in Example 125 (Process 2), and the obtained product was dissolved in DMF. To the solution were added 26.6 µl of triethylamine and 77 mg of benzyloxycarbonyl-Asn-ONp under ice cooling and the mixture was stirred overnight at room temperature. The reaction mixture was treated similarly to that in Example 122 to give 2 mg of the title compound.
Analytical HPLC: 25.62 min (For the condition, see: Example 35).
FAB-MS: 672 (M+1)

### Example 185: Benzyloxycarbonyl-Asn-(2S,3S)-AHPBA-Cpp-NH-tBu

### [Process 1] Boc-Cpp-NH-tBu

In dichloromethane solution containing 25 mg of tert-butylamine, 100 mg of cis-1-t-butoxycarbonyl-4-phenyl-pyrrolidine-2-carboxylic acid (Boc-Cpp-OH), 50 mg of HOBt and 79 mg of EDC hydrochloride were added under ice cooling and the mixture was stirred overnight at room temperature. The reaction mixture was treated similarly to that in Example 144 (Process 2) to give 90 mg of the title compound.
TLC: Rf 0.86 (chloroform : methanol = 20:1)

### [Process 2] Boc-(2S,3S)-AHPBA-Cpp-NH-tBu

Deprotection of 35 mg of the compound obtained by the process 1 was performed similarly to that in Example 125 (Process 2), and the obtained product was dissolved in dichloromethane. To the solution, 14 µl of triethylamine, 48 mg of Boc-(2S,3S)-AHPBA-OH and 53 mg of Bop reagent were added under ice cooling and the mixture was stirred overnight at room temperature. The reaction mixture was treated similarly to that in Example 144 (Process 2) to give 60 mg of the title compound.
TLC: Rf 0.78 (chloroform : methanol = 9:1)

### [Process 3] Benzyloxycarbonyl-Asn-(2S,3S)-AHPBA-Cpp-NH-tBu

Deprotection of 30 mg of the compound obtained by the process 2 was performed similarly to that in Example 125 (Process 2), and the obtained product was dissolved in DMF. To the solution were added 8.4 µl of triethylamine and 23 mg of benzyloxycarbonyl-Asn-ONp under ice cooling and the mixture was treated similarly to that in Example 122 to give 2 mg of the title compound.
Analytical HPLC: 26.23 min (For the condition, see: Example 35).
FAB-MS: 672 (M+1)

### Example 186: Benzyloxycarbonyl-Asn-(2S,3S)-AHPBA-Tcp-NH-tBu

### [Process 1] Boc-Tcp-NH-tBu

In dichloromethane solution containing 25 mg of tert-butylamine, 100 mg of trans-1-t-butoxycarbonyl-4-cyclohexylpyrrolidine-2-car boxylic acid (Boc-Tcp-OH), 63 mg of HOBt and 84 mg of EDC hydrochloride were added under ice cooling and the mixture was stirred overnight at room temperature. The reaction mixture was treated similarly to that in Example 144 (Process 2) to give 160 mg of the title compound.
TLC: Rf 0.90 (chloroform : methanol = 9:1)

### [Process 2] Boc-(2S,3S)-AHPBA-Tcp-NH-tBu

Deprotection of 35 mg of the compound obtained by the process 1 was performed similarly to that in Example 125 (Process 2), and the obtained product was dissolved in dichloromethane. To the solution, 14 µl of triethylamine, 49 mg of Boc-(2S,3S)-AHPBA-OH and 53 mg of Bop reagent were added under ice cooling and the mixture was stirred overnight at room temperature. The reaction mixture was treated similarly to that in Example 93 (Process 3) to give 40 mg of the title compound.
TLC: Rf 0.52 (chloroform : methanol = 9:1)

### [Process 3] Benzyloxycarbonyl-Asn-(2S,3S)-AHPBA-Tcp-NH-tBu

Deprotection of 40 mg of the compound obtained by the process 2 was performed similarly to that in Example 125 (Process 2), and the obtained product was dissolved in DMF. To the solution were added 11.2 µl of triethylamine and 32 mg of benzyloxycarbonyl-Asn-ONp under ice cooling and the mixture was stirred overnight at room temperature. The reaction mixture was treated similarly to that in Example 122 to give 3 mg of the title compound.
Analytical HPLC: 30.16 min (For the condition, see: Example 35).
FAB-MS: 678 (M+1)

### Example 187: Benzyloxycarbonyl-Asn-(2S,3S)-AHPBA-Ccp-NH-tBu

### [Process 1] Boc-Ccp-NH-tBu

In a dichloromethane solution containing 25 mg of tert-butylamine, 110 mg of cis-1-t-butoxycarbonyl-4-cyclohexyl-pyrrolidine-2-carboxylic acid (Boc-Ccp-OH), 63 mg of HOBt and 84 mg of EDC hydrochloride were added under ice cooling and the mixture was stirred overnight at room temperature. The reaction mixture was treated similarly to that in Example 144 (Process 2) to give 170 mg of the title compound.
TLC: Rf 0.87 (chloroform : methanol = 9:1)

### [Process 2] Boc-(2S,3S)-AHPBA-Ccp-NH-tBu

Deprotection of 35 mg of the compound obtained by the process 1 was performed similarly to that in Example 125 (Process 2), and the obtained product was dissolved in dichloromethane. To the solution, 14 µl of triethylamine, 48 mg of Boc-(2S,3S)-AHPBA-OH and 53 mg of Bop reagent were added under ice cooling and the mixture was stirred overnight at room temperature. The reaction mixture was treated similarly to that in Example 93 (Process 3) to give 20 mg of the title compound.
TLC: Rf 0.78 (chloroform : methanol 9:1)

### [Process 3] Benzyloxycarbonyl-Asn-(2S,3S)-AHPBA-Ccp-NH-tBu

Deprotection of 20 mg of the compound obtained by the process 2 was performed similarly to that in Example 125 (Process 2), and the obtained product was dissolved in DMF. To the solution were added 5.6 µl of triethylamine and 16 mg of benzyloxycarbonyl-Asn-ONp under ice cooling and the mixture was stirred overnight at room temperature. The reaction mixture was treated similarly to that in Example 122 to give 2 mg of the title compound.
Analytical HPLC: 29.94 min (For the condition, see: Example 35).
FAB-MS: 678 (M+1)

### Example 188: Benzyloxycarbonyl-Asn-(2S,3S)-AHPBA-Dmp-NH₂

The title compound was obtained by a similar method to that of Example 177.
Analytical HPLC: 22.00 min (For the condition, see: Example 35).
FAB-MS: 568 (M+1)

### Example 189: Inhibitory assay using chemically synthesized HIV protease

The HIV protease was chemically synthesized by replacement of two cysteine residues in the natural sequence [Science, 230, 949 (1985)] with alanine residues. The reaction mixture contained 100 mM MES buffer (pH 5.5), 40 mM of substrate (Ac-Arg-Ala-Ser-Gln-Asn-Tyr-Pro-Val-Val-NH₂ trifluoroacetate), inhibitors at varying concentrations dissolved in DMSO and 9.2 µg of the HIV protease in a total volume of 15 µl. Incubation was carried out at 37 °C for 60 min. The reaction was started by the addition of the enzyme and terminated by 15 µl of acetonitrile. The amount of a fragment peptide produced was measured by reversed-phase HPLC analysis using an internal standerd. The HPLC condition was as followes.
Column: VYDAC 218 TP 54, C18
Solvent A: 0.1% trifluoroacetic acid aqueous solution
Solvent B: acetonitrile
Gradient: B was increased in 1.0%/min from 100% of A,

The residual enzymic activity in the presence of 5 µM (final concentration) of the inhibitor obtained in Example 32 was 4.0%. In addition, the inhibitor showed 60 nM of IC₅₀ and 10 nM of Ki.

The residual activities in the presence of various inhibitors were determined by similar methods and the results are shown in Tables 1-6.

### Example 190: Inhibitory activity assay using recombinant HIV Protease

The inhibitory assay using recombinant HIV protease with the natural amino acid sequence expressed by Escherichia coli [Biochemistry, 29, 264 (1990)] was performed by a similar condition to that in Example 189, except for the amount of the enzyme used (2.0 µg). The residual enzymic activity in the presence of 5 µM (final concentration) of the inhibitor obtained in Example 32 was 11.0%.

### Example 191: Pharmaceutical preparation

(1) The peptide derivative obtained in Example 32 (10 mg), 200 mg of lactose and 10 mg of magnesium stearate were thoroughly mixed and filled in a hard capsule for oral preparation.
(2) The peptide derivative obtained in Example 32 (5 mg), vegetable oil and saline solution for injection were mixed to make an ampule preparation of 2 ml volume.

### Example 192: 5Isoquinoline-O-CH₂CO-Asn-(2S,3S)-AHPBA-Thz-NH-tBu • AcOH

The title compound, which may be prepared from the protected peptide obtained in Example 101 (Process 1) and the carboxylic acid obtained in Example 115 (Process 1) by a similar method to that in Example 123 (Process 2), is expected to show high inhibitory activity against the HIV protease [cf. Example 101 in Table 4 and Example 115 in Table 4].

### Example 193: 5Isoquinoline-O-CH₂CO-Mta-(2S,3S)-AHPBA-Thz-tBu • AcOH

The title compound, which may be prepared from the protected peptide obtained in Example 162 (Process 1) and the carboxylic acid obtained in Example 115 (Process 1) by a similar method to that in Example 123 (Process 2), is expected to show high inhibitory activity against the HIV protease [cf. Example 130 in Table 5 and Example 162 in Table 6].

## Claims

1. A compound of formula (3) or a salt thereof: 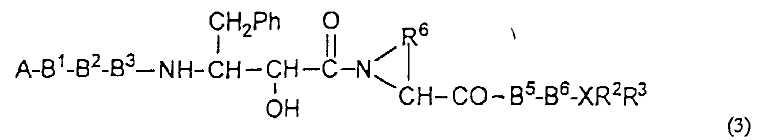 where A is a hydrogen atom or a peptide N-terminal protective group;
B³ is the residue of an amino acid selected from asparagine, glutamine, serine, O-methylserine, O-methylthreonine, methylthioalanine, methanesulfonylalanine, valine, norvaline, leucine, isoleucine and tert-leucine;
B¹, B², B⁵ and B⁶ are selected independently from amino acid residues with amino optionally substituted with hydrocarbon of 12 or less carbon atoms but any one or more of B¹, B², B⁵, and B⁶ may be absent;
R⁶ is a bivalent hydrocarbon group forming a 5-7 membered ring optionally substituted or fused with other 5-7 membered ring, carbon atoms in said rings optionally being partially replaced by hetero atom or atoms;
X is a nitrogen atom or oxygen atom; and
R² and R³ are selected independently from a hydrogen atom and optionally substituted hydrocarbon groups of 12 or less carbon atoms which may form cycles of which carbon atoms may be replaced with oxygen, nitrogen or sulfur with the proviso that no R³ is present when X is oxygen.

2. An HIV protease inhibitor of formula (3) or a salt thereof : 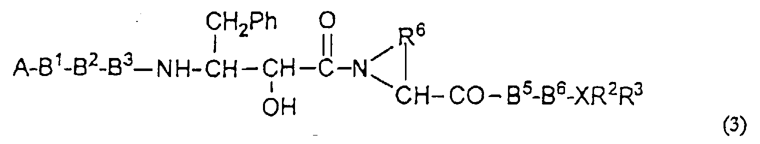 where A is a hydrogen atom or a peptide N-terminal protective group;
B³ is the residue of an amino acid selected from asparagine, glutamine, O-methylserine, O-methylthreonine, methylthioalanine, methanesulfonylalanine, valine, norvaline, leucine, and tert-leucine;
B¹, B², B⁵ and B⁶ are selected independently from amino acid residues with amino optionally substituted with hydrocarbon of 12 or less carbon atoms but any one or more of B¹, B², B⁵, and B⁶ may be absent;
R⁶ is a bivalent hydrocarbon group forming a 5-7 membered ring optionally substituted or fused with other 5-7 membered ring, carbon atoms in said rings optionally being partially replaced by hetero atom or atoms;
X is a nitrogen atom or oxygen atom; and
R² and R³ are selected independently from a hydrogen atom and optionally substituted hydrocarbon groups of 12 or less carbon atoms which may form cycles of which carbon atoms may be replaced with oxygen, nitrogen or sulfur with the proviso that no R³ is present when X is oxygen.

3. A compound according to claim 1 or 2 wherein A is a peptide N-terminal protective group, and B¹, B², B⁵ and B⁶ are all absent.

4. A compound according to any preceding claim wherein A is an aryloxyacetyl or heteroaryloxyacetyl group.

5. A compound according to any preceding claim wherein (a) the residue of a cyclic amino acid including R⁶ is a proline, 3,3-dimethylpyrrolidine-2-carboxylic acid, 1,3-thiazolidine-4-carboxylic acid or 5,5-dimethyl-1,3-thiazolidine-4-carboxylic acid residue; and/or (b) -XR²R³ is an N-tert-butylamino group.

6. A compound according to any preceding claim wherein X is a nitrogen atom.

7. A pharmaceutical composition for inhibiting viral replication which comprises an effective protease inhibiting amount of protease inhibitor according to any preceding claim as an active ingredient for inhibiting viral replication; and a pharmaceutically acceptable carrier therefor.

8. The use of a compound or salt as defined in any one of claims 1 to 6 for the preparation of a medicament for inhibiting viral replication.

9. A process for the production of novel compounds or HIV protease inhibitors, the process comprising converting an amino acid derivative of general formula (6) 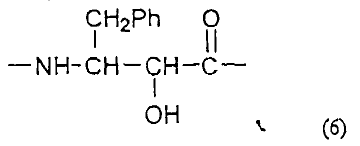 into a peptide derivative of general formula (3) 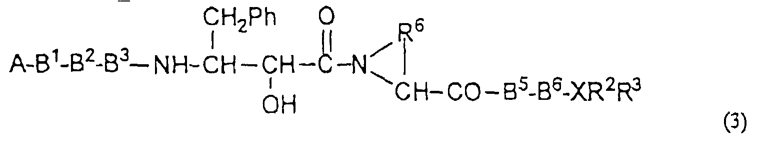 where A is a hydrogen atom or a peptide N-terminal protective group;
B³ is the residue of an amino acid selected from asparagine, glutamine, serine, O-methylserine, O-methylthreonine, methylthioalanine, methanesulfonylalanine, valine, norvaline, leucine, isoleucine and tert-leucine;
B¹, B², B⁵ and B⁶ are selected independently from amino acid residues with amino optionally substituted with hydrocarbon of 12 or less carbon atoms but any one or more of B¹, B², B⁵ and B⁶ may be absent;
R⁶ is a bivalent hydrocarbon group forming a 5-7 membered ring optionally substituted or fused with other 5-7 membered ring, carbon atoms in said rings optionally being partially replaced by hetero atom or atoms;
X is a nitrogen atom or oxygen atom; and
R² and R³ are selected independently from a hydrogen atom and optionally substituted hydrocarbon groups of 12 or less carbon atoms which may form cycles of which carbon atoms may be replaced with oxygen, nitrogen or sulfur with the proviso that no R³ is present when X is oxygen.

## Patentansprüche

1. Verbindung der Formel (3) oder ein Salz davon: 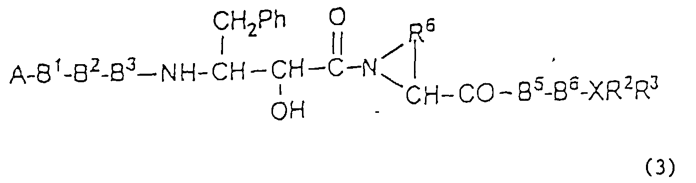 wobei A ein Wasserstoffatom oder eine N-terminale Peptid-Schutzgruppe ist;
B³ der Rückstand einer Aminosäure ist, die ausgewählt wird aus Asparagin, Glutamin, Serin, O-Methylserin, O-Methylthreonin, Methylthioalanin, Methansulfonylalanin, Valin, Norvalin, Leucin, Isoleucin und Tert-Leucin;
B¹, B², B⁵ und B⁶ unabhängig ausgewählt werden aus Aminosäurerückständen, wobei Amino bedarfsabhängig durch Kohlenwasserstoff mit 12 oder weniger Kohlenstoffatomen substituiert wird, wobei aber einer oder mehrere von B¹, B², B⁵ und B⁶ abwesend sein kann/können;
R⁶ eine bivalente Kohlenwasserstoffgruppe ist, die einen 5- bis 7-gliedrigen Ring bildet, der bedarfsabhängig mit einem anderen 5- bis 7-gliedrigen Ring substituiert oder anelliert wird, wobei Kohlenstoffatome in den genannten Ringen bedarfsabhängig teilweise durch ein Heteroatom oder -atome ersetzt werden;
X ein Stickstoffatom oder ein Sauerstoffatom ist; und
R² und R³ unabhängig ausgewählt werden aus einem Wasserstoffatom und bedarfsabhängig substituierten Kohlenwasserstoffgruppen mit 12 oder weniger Kohlenstoffatomen, die Zyklen bilden können, deren Kohlenstoffatome durch Sauerstoff, Stickstoff oder Schwefel unter der Voraussetzung ersetzt werden können, daß kein R³ anwesend ist, wenn X Sauerstoff ist.

2. HIV-Proteaseinhibitor der Formel (3) oder ein Salz davon: 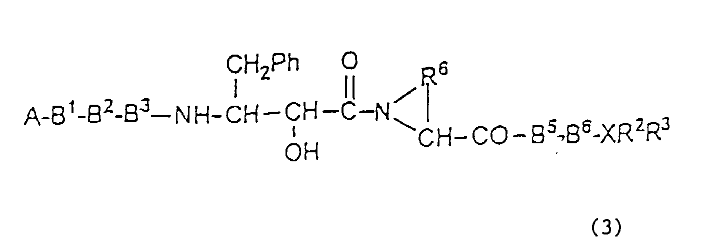 wobei A ein Wasserstoffatom oder eine N-terminale Peptid-Schutzgruppe ist;
B³ der Rückstand einer Aminosäure ist, die ausgewählt wird aus Asparagin, Glutamin, O-Methylserin, O-Methylthreonin, Methylthioalanin, Methansulfonylalanin, Valin, Norvalin, Leucin, und Tert-Leucin;
B¹, B², B⁵ und B⁶ unabhängig ausgewählt werden aus Aminosäurerückständen, wobei Amino bedarfsabhängig durch Kohlenwasserstoff mit 12 oder weniger Kohlenstoffatomen substituiert wird, wobei aber einer oder mehrere von B¹, B², B⁵ und B⁶ abwesend sein kann/können;
R⁶ eine bivalente Kohlenwasserstoffgruppe ist, die einen 5- bis 7-gliedrigen Ring bildet, der bedarfsabhängig mit einem anderen 5- bis 7-gliedrigen Ring substituiert oder anelliert wird, wobei Kohlenstoffatome in den genannten Ringen bedarfsabhängig teilweise durch ein Heteroatom oder -atome ersetzt werden;
X ein Stickstoffatom oder ein Sauerstoffatom ist; und
R² und R³ unabhängig ausgewählt werden aus einem Wasserstoffatom und bedarfsabhängig substituierten Kohlenwasserstoffgruppen mit 12 oder weniger Kohlenstoffatomen, die Zyklen bilden können, deren Kohlenstoffatome durch Sauerstoff, Stickstoff oder Schwefel unter der Voraussetzung ersetzt werden können, daß kein R³ anwesend ist, wenn X Sauerstoff ist.

3. Verbindung nach Anspruch 1 oder 2, bei der A eine N-terminale Peptid-Schutzgruppe ist und B¹, B², B⁵ und B⁶ ganz abwesend sind.

4. Verbindung nach einem der vorherigen Ansprüche, bei der A eine Aryloxyacetyl- oder Heteroaryloxyacetylgruppe ist.

5. Verbindung nach einem der vorherigen Ansprüche, bei der (a) der Rückstand einer cyclischen Aminosäure einschließlich R⁶ ein Prolin-, 3,3-Dimethylpyrrolidin-2-Carbonsäure-, 1,3-Thiazolidin-4-Carbonsäure- oder 5,5-Dimethyl-1,3-Thiazolidin-4-Carbonsäurerückstand und/oder (b) -XR²R³ eine N-Tert-Butylaminogruppe ist.

6. Verbindung nach einem der vorherigen Ansprüche, bei der X ein Stickstoffatom ist.

7. Pharmazeutische Zusammensetzung zur Inhibierung von Virusreplikation, die eine wirksame proteaseinhibierende Menge eines Proteaseinhibitors nach einem der vorherigen Ansprüche als aktiven Bestandteil zur Inhibierung von Virusreplikation und einen pharmazeutisch akzeptablen Träger dafür umfaßt.

8. Verwendung einer Verbindung oder eines Salzes gemäß Definition in einem der Ansprüche 1 bis 6 für die Herstellung eines Medikamentes zur Inhibierung von Virusreplikation.

9. Verfahren zur Produktion von neuartigen Verbindungen oder HIV-Proteaseinhibitoren, wobei das Verfahren die Umwandlung eines Aminosäurederivates der allgemeinen Formel (6) 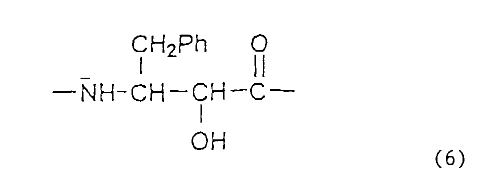 in ein Peptidderivat der allgemeinen Formel (3) umfaßt, 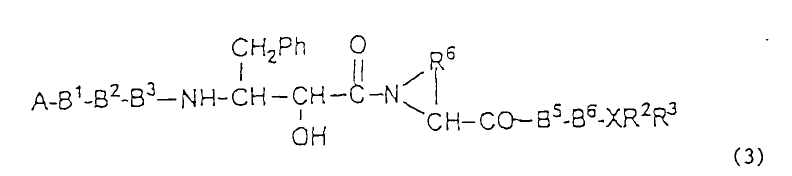 wobei A ein Wasserstoffatom oder eine N-terminale Peptid-Schutzgruppe ist;
B³ der Rückstand einer Aminosäure ist, die ausgewählt wird aus Asparagin, Glutamin, Serin, O-Methylserin, O-Methylthreonin, Methylthioalanin, Methansulfonylalanin, Valin, Norvalin, Leucin, Isoleucin und Tert-Leucin;
B¹, B², B⁵ und B⁶ unabhängig ausgewählt werden aus Aminosäurerückständen, wobei Amino bedarfsabhängig durch Kohlenwasserstoff mit 12 oder weniger Kohlenstoffatomen substituiert wird, wobei aber einer oder mehrere von B¹, B², B⁵ und B⁶ abwesend sein kann/können;
R⁶ eine bivalente Kohlenwasserstoffgruppe ist, die einen 5- bis 7-gliedrigen Ring bildet, der bedarfsabhängig mit einem anderen 5- bis 7-gliedrigen Ring substituiert oder anelliert wird, wobei Kohlenstoffatome in den genannten Ringen bedarfsabhängig teilweise durch ein Heteroatom oder -atome ersetzt werden;
X ein Stickstoffatom oder ein Sauerstoffatom ist; und
R² und R³ unabhängig ausgewählt werden aus einem Wasserstoffatom und bedarfsabhängig substituierten Kohlenwasserstoffgruppen mit 12 oder weniger Kohlenstoffatomen, die Zyklen bilden können, deren Kohlenstoffatome durch Sauerstoff, Stickstoff oder Schwefel unter der Voraussetzung ersetzt werden können, daß kein R³ anwesend ist, wenn X Sauerstoff ist.

## Revendications

1. Composé de la formula (3) ou un sel du celui-ci: 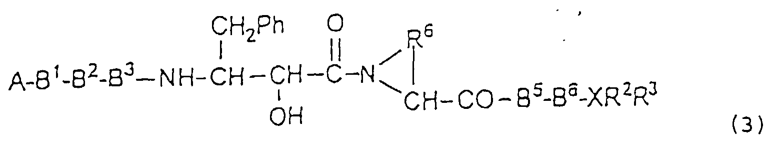 où A est Un atome d'hydrogène ou un groupe protecteur peptide N-terminal;
B³ est le résidu d'un acide aminé sélectionné à partir de l'asparagine, de la glutamine, de la sérine, de la O-méthylsérine, de la O-méthylthréonine, de la méthylthioalanine, de la méthanesulfonylalanine, de la valine, de la norvaline, de la leucine, de l'isoleucine et de la tert-leucine;
B¹, B², B⁵ et B⁶ sont sélectionnés indépendamment à partir de résidus d'acides aminés avec un groupe aminé facultativement substitué par un hydrocarbure de 12 atomes de carbone ou moins mais l'un quelconque ou plusieurs de B¹, B², B⁵ et B⁶ peuvent être absents;
R⁶ est un groupe d'hydrocarbure bivalent formant un noyau pentagonal-heptagonal facultativement substitué par ou fusionné avec un autre noyau pentagonal-heptagonal, les atomes de carbone dans lesdits noyaux étant facultativement partiellement remplacés par un hétéroatome ou des hétéroatomes;
X est un atome d'azote ou un atome d'oxygène; et
R² et R³ sont sélectionnés indépendamment à partir d'un atome d'hydrogène et des groupes d'hydrocarbure facultativement substitués de 12 atomes de carbone ou moins qui peuvent former des cycles dont les atomes de carbone peuvent être remplacés par de l'oxygène, de l'azote ou du soufre à condition que aucun R³ ne soit présent lorsque X est de l'oxygène.

2. Inhibiteur de la VIH protéase de la formule (3) ou un sel de celui-ci: 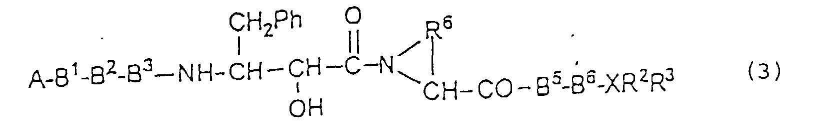 où A est un atome d'hydrogène ou un groupe protecteur peptide N-terminal;
B³ est le résidu d'un acide aminé sélectionné à partir de l'asparagine, de la glutamine, de la O-méthylsérine, de la O-méthylthréonine, de la méthylthioalanine, de la méthanesulfonylalanine, de la valine, de la norvaline, de la leucine, et de la tert-leucine;
B¹, B², B⁵ et B⁶ sont sélectionnés indépendamment à partir de résidus d'acides aminés avec un groupe aminé facultativement substitué par un hydrocarbure de 12 atomes de carbone ou moins mais l'un quelconque ou plusieurs de B¹, B², B⁵ et B⁶ peuvent être absents;
R⁶ est un groupe d'hydrocarbure bivalent formant un noyau pentagonal-heptagonal facultativement substitué par ou fusionné avec un autre noyau pentagonal-heptagonal, les atomes de carbone dans lesdits noyaux étant facultativement partiellement remplacés par un hétéroatome ou des hétéroatomes;
X est un atome d'azote ou un atome d'oxygène; et
R² et R³ sont sélectionnés indépendamment à partir d'un atome d'hydrogène et des groupes d'hydrocarbure facultativement substitués de 12 atomes de carbone ou moins qui peuvent former des cycles dont les atomes de carbone peuvent être remplacés par de l'oxygène, de l'azote ou du soufre à condition que aucun R³ ne soit présent lorsque X est de l'oxygène.

3. Composé selon la revendication 1 ou 2, dans lequel A est un groupe protecteur peptide N-terminal, et B¹, B², B⁵ et B⁶ sont tous absents.

4. Composé selon l'une quelconque des revendications précédentes, dans lequel A est un groupe aryloxyacétyle ou hétéroaryloxyacétyle.

5. Composé selon l'une quelconque des revendications précédentes, dans lequel (a) le résidu d'un acide aminé cyclique incluant R⁶, est une proline, de l'acide 3,3-diméthylpyrrolidine-2-carboxylique, de l'acide 1,3-thiazolidine-4-carboxylique ou un résidu d'acide 5,5-diméthyl-1,3-thiazolidine-4-carboxylique; et/ou (b) -XR²R³ est un groupe N-tert-butylaminé.

6. Composé selon l'une quelconque des revendications précédentes, dans lequel X est un atome d'azote.

7. Composition pharmaceutique pour inhibiter la réplication virale qui comprend une quantité protéase inhibitrice effective d'inhibiteur de protéase selon l'une quelconque des revendications précédentes, comme un principe actif pour inhiber la réplication virale; et un support de celui-ci pharmaceutiquement acceptable.

8. Emploi d'un composé ou sel tel que défini dans l'une quelconque des revendications 1 à 6, pour la préparation d'un médicament pour inhiber la réplication virale.

9. Procédé pour la production de composés ou d'inhibiteurs de VIH protéase nouveaux, le procédé comprenant convertir un dérivé d'acide aminé de la formule qénérale (6) 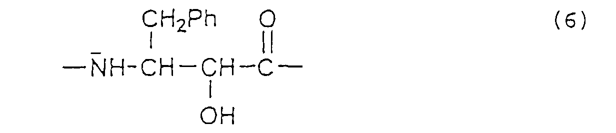 en un dérivé peptide de la formule générale (3) 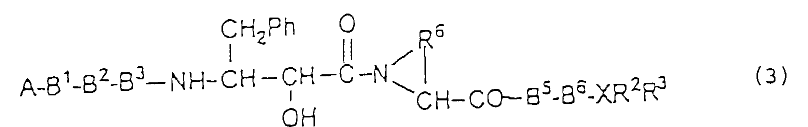 où A est un atome d'hydrogène ou un groupe protecteur peptide N-terminal;
B³ est le résidu d'un acide aminé sélectionné à partir de l'asparagine, de la glutamine, de la sérine, de la O-méthylsérine, de la O-méthylthréonine, de la méthylthioalanine, de la méthanesulfonylalanine, de la valine, de la norvaline, de la leucine, de l'isoleucine et de la tert-leucine;
B¹, B², B⁵ et B⁶ sont sélectionnés indépendamment à partir de résidus d'acides aminés avec un groupe aminé facultativement substitué par un hydrocarbure de 12 atomes de carbone ou moins mais l'un quelconque ou plusieurs de B¹, B², B⁶ et B⁶ peuvent être absents;
R⁶ est un groupe d'hydrocarbure bivalent formant un noyau pentagonal-heptagonal facultativement substitué par ou fusionné avec un autre noyau pentagonal-heptagonal, les atomes de carbone dans lesdits noyaux étant facultativement partiellement remplacés par un hétéroatome ou des hétéroatomes;
X est un atome d'azote ou un atome d'oxygène; et
R² et R³ sont sélectionnés indépendamment à partir d'un atome d'hydrogène et des groupes d'hydrocarbure facultativement substitués de 12 atomes de carbone ou moins qui peuvent former des cycles dont les atomes de carbone peuvent être remplacés par de l'oxygène, de l'azote ou du soufre à condition que aucun R³ ne soit présent lorsque X est de l'oxygène.
